# EUROPEAN PATENT SPECIFICATION

(11) **EP 1 127 150 B1**
(45) Date of publication and mention of the grant of the patent: **16.05.2007**
(21) Application number: 99961559.4
(22) Date of filing: 02.11.1999
(51) Int. Cl.: C12N 15/86, C12N 5/10, A61K 48/00

(54) **ADENO-ASSOCIATED VIRUS SEROTYPE 1 NUCLEIC ACID SEQUENCES, VECTORS AND HOST CELLS CONTAINING SAME**
NUKLEINSÄURESEQUENZEN DES ADENO-ASSOZIIERTEN VIRUS DES SEROTYPS I, UND VEKTOREN UND WIRTSZELLEN, DIE DIESE ENTHALTEN
SEQUENCES D'ACIDE NUCLEIQUE DU SEROTYPE I DU VIRUS ASSOCIE AUX ADENOVIRUS, VECTEURS ET CELLULES HOTES CONTENANT CES DERNIERS

(30) Priority: 05.11.1998 US 107114 P
(43) Date of publication of application: 29.08.2001
(73) Proprietor: THE TRUSTEES OF THE UNIVERSITY OF PENNSYLVANIA, Philadelphia, Pennsylvania 19104-3147 (US)
(72) Inventor: WILSON, James, M., Gladwyne, PA 19035 (US); XIAO, Weidong, Jenkintown, PA 19046 (US)
(74) Representative: Manaton, Ross Timothy
(86) International application number: PCT/US1999/025694
(87) International publication number: WO 2000/028061

(56) References cited:
- WO-A-98/11244
- RUTLEDGE E. A. ET AL.: "Infectious clones and vectors derived from adeno-associated virus (AAV) serotypes other than AAV type 2." JOURNAL OF VIROLOGY, vol. 72, no. 1, January 1998 (1998-01), pages 309-319, XP002137089 ISSN: 0022-538X cited in the application
- XIAO W. ET AL.: "Gene therapy vectors based on adeno-associated virus type 1." JOURNAL OF VIROLOGY, vol. 73, no. 5, May 1999 (1999-05), pages 3994-4003, XP002137090 ISSN: 0022-538X

## Description

This work was supported by the National Institutes of Health, grant no. P30 DK47757-06 and PO1 HD32649-04. The US government may have certain rights in this invention.

### Field of the Invention

This invention relates generally to recombinant viruses useful for gene therapy. It also relates to pharmaceutical compositions, recombinant host cells, vectors and the use of recombinant viruses in gene therapy.

### Background of the Invention

Adeno-associated viruses are small, single-stranded DNA viruses which require helper virus to facilitate efficient replication [K.I. Berns, *Parvoviridae: the viruses and their replication.* p. 1007-1041, in F.N. Fields et al., Fundamental virology, 3rd ed., vol. 2, (Lippencott-Raven Publishers, Philadelphia, PA) (1995)]. The 4.7 kb genome of AAV is characterized by two inverted terminal repeats (ITR) and two open reading frames which encode the Rep proteins and Cap proteins, respectively. The Rep reading frame encodes four proteins of molecular weight 78 kD, 68 kD, 52 kD and 40 kD. These proteins function mainly in regulating AAV replication and integration of the AAV into a host cell's chromosomes. The Cap reading frame encodes three structural proteins in molecular weight 85 kD (VP 1), 72 kD (VP2) and 61 kD (VP3) [Berns, cited above]. More than 80% of total proteins in AAV virion comprise VP3. The two ITRs are the only cis elements essential for AAV replication, packaging and integration. There are two conformations of AAV ITRs called "flip" and "flop". These differences in conformation originated from the replication model of adeno-associated virus which use the ITR to initiate and reinitiate the replication [R.O. Snyder et al., J. Virol., 67:6096-6104 (1993); K.I. Berns, Microbiological Reviews. 54:316-329 (1990)].

AAVs have been found in many animal species, including primates, canine, fowl and human [F.A. Murphy et al., "The Classification and Nomenclature of Viruses: Sixth Report of the International Committee on Taxonomy of Viruses", Archives of Virology, (Springer-Verlag, Vienna) (1995)]. In addition to five known primate AAVs (AAV-1 to AAV-5), AAV-6, another serotype closely related to AAV-2 and AAV-1 has also been isolated [E. A. Rutledge et al., J. Virol., 72:309-319 (1998)]. Among all known AAV serotypes, AAV-2 is perhaps the most well-characterized serotype, because its infectious clone was the first made [R.J. Samulski et al., Proc. Natl. Acad. Sci. USA, 79:2077-2081 (1982)]. Subsequently, the full sequences for AAV-3A, AAV-3B, AAV-4 and AAV-6 have also been determined [Rutledge, cited above; J.A.Chiorini et al., J. Virol., 71:6823-6833 (1997); S. Muramatsu et al., Virol., 221:208-217 (1996)]. Generally, all AAVs share more than 80% homology in nucleotide sequence.

A number of unique properties make AAV a promising vector for human gene therapy [Muzyczka, Current Topics in Microbiology and Immunology. 158:97-129 (1992)]. Unlike other viral vectors, AAVs have not been shown to be associated with any known human disease and are generally not considered pathogenic. Wild type AAV is capable of integrating into host chromosomes in a site specific manner [R. M. Kotin et al., Proc, Natl. Acad. Sci, USA, 87:2211-2215 (1990)- R.J. Samulski, EMBO J., 10(12):3941-3950 (1991)]. Recombinant AAV vectors can integrate into tissue cultured cells in chromosome 19 if the rep proteins are supplied in *trans* [C. Balague et al., J. Virol., 71:3299-3306 (1997); R. T. Surosky et al., J. Virol., 71:7951-7959 (1997)]. The integrated genomes of AAV have been shown to allow long term gene expression in a number of tissues, including, muscle, liver, and brain [K. J. Fisher, Nature Med., 3(3):306-312 (1997); R. 0. Snyder et al., Nature Genetics, 16:270-276 (1997); X. Xiao et al., Experimental Neurology, 144:113-124 (1997); Xiao, J. Virol., 70(11):8098-8108 (1996)].

AAV-2 has been shown to be present in about 80-90% of the human population. Earlier studies showed that neutralizing antibodies for AAV-2 are prevalent [W. P. Parks et al., J. Virol., 2:716-722 (1970)]. The presence of such antibodies may significantly decrease the usefulness of AAV vectors based on AAV-2 despite its other merits. What are needed in the art are vectors characterized by the advantages of AAV-2, including those described above, without the disadvantages, including the presence of neutralizing antibodies.

### Summary of the Invention

In one aspect, the invention provides a recombinant virus having an AAV-1 capsid comprising an AAV-1 protein selected from among AAV-1 vp1 having the amino acid sequence of SEQ ID NO: 13, AAV-1 vp2 having the amino acid sequence of SEQ ID NO: 15, AAV-1 vp3 having the amino acid sequence of SEQ ID NO: 17, and a heterologous molecule comprising an AAV 5' inverted terminal repeat sequence (ITR), a transgene and an AAV 3' ITR.

In another aspect, the invention provides a pharmaceutical composition comprising a recombinant virus and a pharmaceutically acceptable carrier.

In yet another aspect, the invention provides for the use of a recombinant virus for preparing a medicament useful for transducing a muscle cell.

In still another aspect, the invention provides a recombinant host cell transduced with a recombinant virus of the invention.

In yet another aspect, the invention provides a vector encoding AAV-1 helper functions, said molecule comprising an AAV rep coding region and an AAV cap coding region, wherein said cap coding region comprises at least one member selected from the group consisting of a) vp 1 having the nucleic acid sequence of nt 2223 to 4431 of SEQ ID NO: 1; b) vp2 having the nucleic acid sequence of nt 2634 to 4432 of SEQ ID NO: 1; and c) vp3 having the nucleic acid sequence of nt 2829 to 4432 of SEQ ID NO: 1.

In another aspect, the invention provides for the use of an AAV virion which comprises: a) a capsid comprising at least one capsid protein encoded by an AAV-1 cap gene having at least 99% identity to nt 2223 to 4431 of SEQ ID NO: 1; and b) a DNA molecule comprising a transgene under the control of regulatory sequences directing its expression, for preparing a medicament for delivery of a transgene to a host cell.

### Brief Description of the Drawings

Figs. 1A-1C illustrate the alignment of nucleotides of AAV-1 [SEQ ID NO: 1], AAV-2 [SEQ ID NO: 18] and AAV-6 [SEQ ID NO: 19]. The alignment was done with MacVector 6.0. The full sequences of AAV-1 are shown in the top line. Nucleotides in AAV-2 and AAV-6 identical to AAV-1 are symbolized by "." and gaps by "-". Some of the conserved features among AAVs are marked in this figure. Note the 3' ITRs of AAV-1 and AAV-6 are shown in different orientations.
Fig. 2 illustrates the predicted secondary structure of AAV-1 ITR. The nucleotides in AAV-2 and AAV-6 are shown in italic and bold respectively.
Fig. 3 A illustrates a hypothesis of how AAV-6 arose from the homologous recombination between AAV-1 and AAV-2. The major elements of AAV-1 are indicated in the graph. A region that is shared between AAV-1, AAV-2 and AAV-6 is shown in box with waved lines.
Fig. 3B is a detailed illustration of a 71 bp homologous region among AAV-1, AAV-2 and AAV-6. Nucleotides that differ among these serotypes are indicated by arrows.
Fig. 4A is a bar chart illustrating expression levels of human alpha 1 antitrypsin (α1AT) in serum following delivery of hAAT via recombinant AAV-1 and recombinant AAV-2 viruses.
Fig. 4B is a bar chart illustrating expression levels of erythropoietin (epo) in serum following delivery of the epo gene via recombinant AAV-1 and recombinant AAV-2 viruses.
Fig. 5A is a bar chart illustrating expression levels of α1AT in liver following delivery of alAT as described in Example 7.
Fig. 5B is a bar chart demonstrating expression levels of epo in liver following delivery of epo as described in Example 7.
Fig. 5C is a bar chart demonstrating neutralizing antibodies (NAB) directed to AAV-1 following delivery of α1AT or epo to liver as described in Example 7.
Fig. 5D is a bar chart demonstrating neutralizing antibodies (NAB) directed to AAV-2 following delivery of α1AT or epo to liver as described in Example 7.
Fig. 6A is a bar chart illustrating expression levels of α1AT in muscle following delivery of α1AT as described in Example 7.
Fig. 6B is a bar chart demonstrating expression levels of epo in muscle following delivery of epo as described in Example 7.
Fig. 6C is a bar chart demonstrating neutralizing antibodies (NAB) directed to AAV-1 following delivery of α1AT or epo to muscle as described in Example 7.
Fig. 6D is a bar chart demonstrating neutralizing antibodies (NAB) directed to AAV-2 following delivery of α1AT or epo to muscle as described in Example 7.

### Detailed Description of the Invention

As used throughout this specification and the claims, the term "comprising" is inclusive of other components, elements, integers, steps and the like.

### 1. AAV-1 NUCLEIC ACID AND PROTEIN SEQUENCES

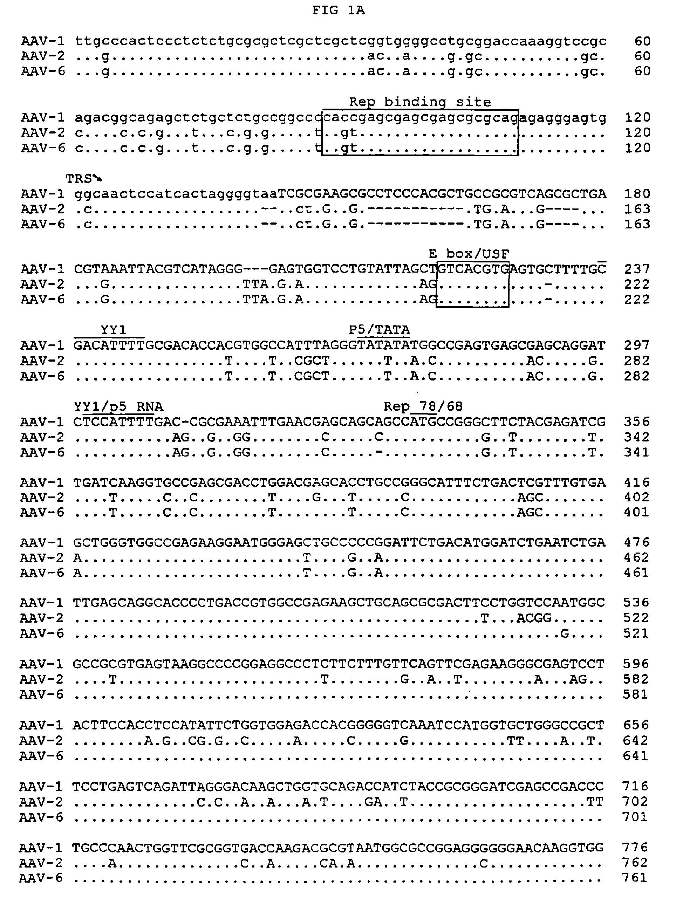

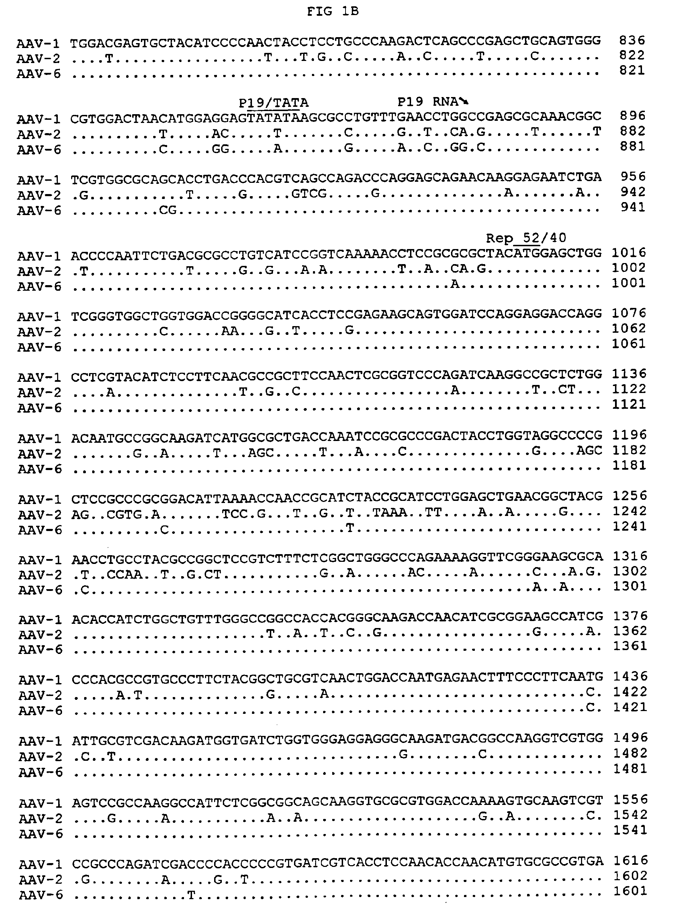

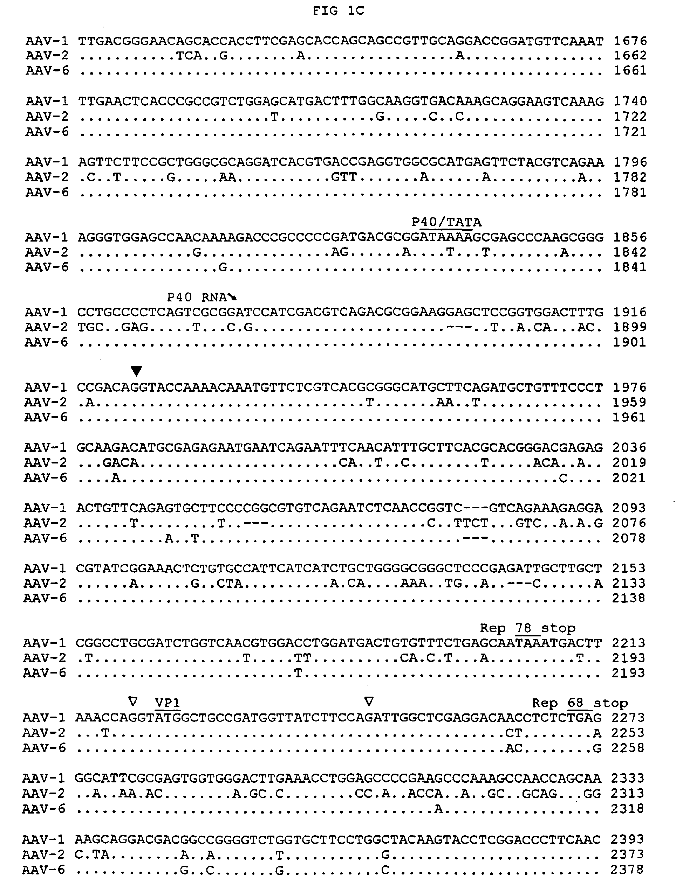

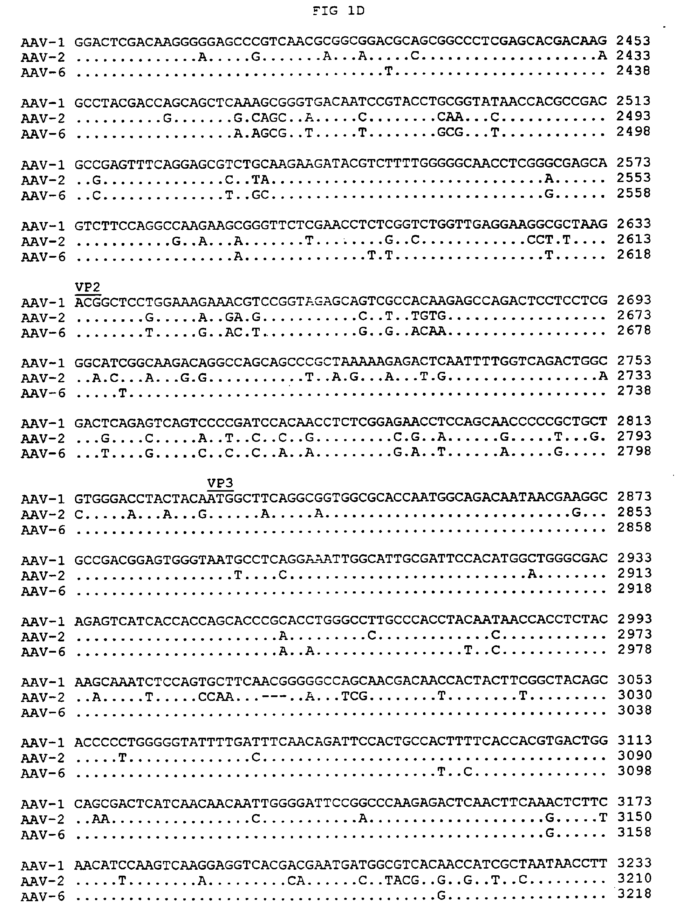

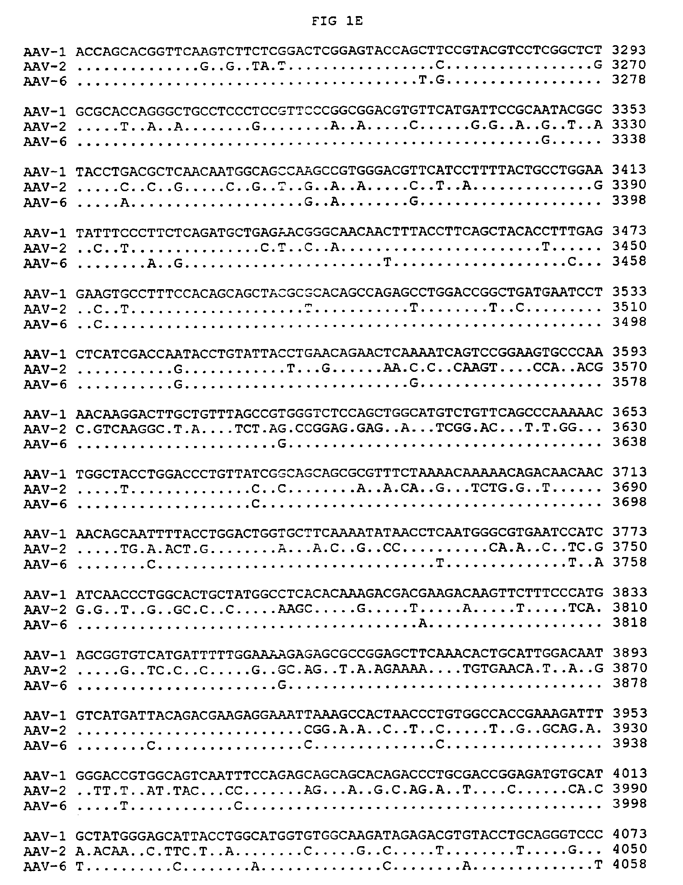

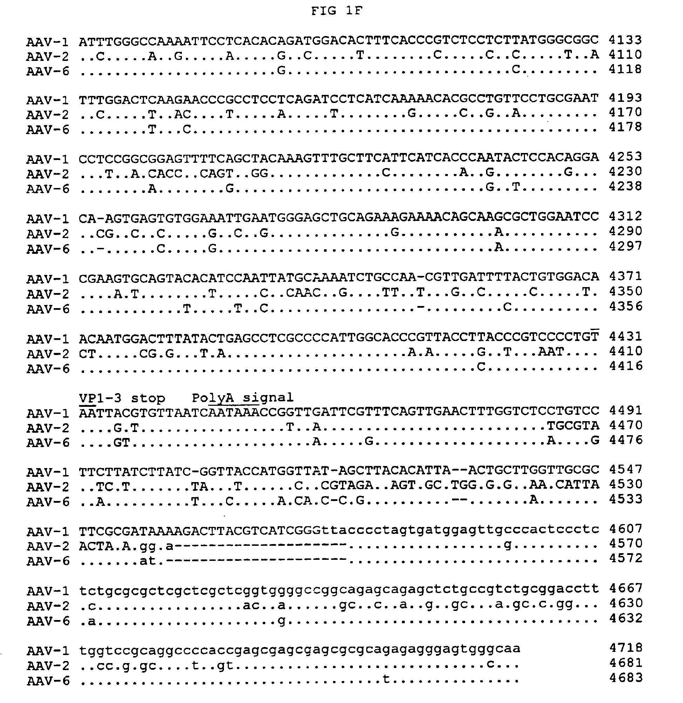

The AAV-1 nucleic acid sequences of the invention include the DNA sequences of SEQ ID NO: I (Figs. 1A-1C), which consists of 4718 nucleotides. The AAV-1 nucleic acid sequences of the invention further encompass the strand which is complementary to SEQ ID NO: 1, as well as the RNA and cDNA sequences corresponding to SEQ ID NO: 1 and its complementary strand. Also included in the nucleic acid sequences of the invention are natural variants and engineered modifications of SEQ ID NO: 1 and its complementary strand. Such modifications include, for example, labels which are known in the art, methylation, and substitution of one or more of the naturally occurring nucleotides with an analog.

Further included in this invention are nucleic acid sequences which are at least 99% identical or homologous to SEQ ID NO: 1. The term "percent sequence identity" or "identical" in the context of nucleic acid sequences refers to the residues in the two sequences which are the same when aligned for maximum correspondence. The length of sequence identity comparison may be over the full-length sequence, or a fragment at least about nine nucleotides, usually at least about 20 - 24 nucleotides, at least about 28-32 nucleotides, and preferably at least about 36 or more nucleotides. There are a number of different algorithms known in the art which can be used to measure nucleotide sequence identity. For instance, polynucleotide sequences can be compared using Fasta, a program in GCG Version 6.1. Fasta provides alignments and percent sequence identity of the regions of the best overlap between the query and search sequences (Pearson, 1990, herein incorporated by reference). For instance, percent sequence identity between nucleic acid sequences can be determined using Fasta with its default parameters (a word size of 6 and the NOPAM factor for the scoring matrix) as provided in GCG Version 6.1, herein incorporated by reference.

The term "substantial homology" or "substantial similarity," when referring to a nucleic acid or fragment thereof, indicates that, when optimally aligned with appropriate nucleotide insertions or deletions with another nucleic acid (or its complementary strand), there is nucleotide sequence identity in at least 99% of the sequence.

Also included within the invention are fragments of SEQ ID NO: 1, its complementary strand, cDNA and RNA complementary thereto. Suitable fragments are at least 15 nucleotides in length, and encompass functional fragments which are of biological interest. Certain of these fragments may be identified by reference to Figs. 1A-1C. Examples of particularly desirable functional fragments include the AAV-1 inverted terminal repeat (ITR) sequences of the invention. In contrast to the 145 nt ITRs of AAV-2, AAV-3, and AAV-4, the AAV-1 ITRs have been found to consist of only 143 nucleotides, yet advantageously are characterized by the T-shaped hairpin structure which is believed to be responsible for the ability of the AAV-2 ITRs to direct site-specific integration. In addition, AAV-1 is unique among other AAV serotypes, in that the 5' and 3' ITRs are identical. The full-length 5' ITR sequences of AAV-1 are provided at nucleotides 1-143 of SEQ ID NO: I (Fig. 1A) and the full-length 3' ITR sequences of AAV-1 are provided at nt 4576-4718 of SEQ ID NO: (Fig. 1C). One of skill in the art can readily utilize less than the full-length 5' and/or 3' ITR sequences for various purposes and may construct modified ITRs using conventional techniques, e.g., as described for AAV-2 ITRs in Samulski et al, Cell, 33:135-143 (1983).

Another desirable functional fragment of the AAV-1 genome is the P5 promoter of AAV-1 which has sequences unique among AAV P5 promoters, while maintaining critical regulatory elements and functions. This promoter is located within nt 236 - 299 of SEQ ID NO: 1 (Fig. 1A). Other examples of functional fragments of interest include the sequences at the junction of the rep/cap, e.g., the sequences spanning nt 2306-2223, as well as larger fragments which encompass this junction which may comprise 50 nucleotides on either side of this junction. Still other examples of functional fragments include the sequences encoding the rep proteins. Rep 78 is located in the region of nt 334 - 2306 of SEQ ID NO: 1; Rep 68 is located in the region of nt 334-2272, and contains an intron spanning nt 1924-2220 of SEQ ID NO: 1. Rep 52 is located in the region of nt 1007 - 2304 of SEQ ID NO: 1; rep 40 is located in the region of nt 1007 - 2272, and contains an intron spanning nt 1924-2246 of SEQ ID NO: 1. Also of interest are the sequences encoding the capsid proteins, VP 1 [nt 2223-4431 of SEQ ID NO: 1], VP2 [nt 2634-4432 of SEQ ID NO: 1] and VP3 [nt 2829-4432 of SEQ ID NO: 1]. Other fragments of interest may include the AAV-1 P19 sequences, AAV-1 P40 sequences, the rep binding site, and the terminal resolute site (TRS).

The invention further provides the proteins and fragments thereof which are encoded by the AAV-1 nucleic acids of the invention. Particularly desirable proteins include the rep and cap proteins, which are encoded by the nucleotide sequences identified above. These proteins include rep 78 [SEQ ID NO:5], rep 68 [SEQ ID NO:7], rep 52 [SEQ ID NO:9], rep 40 [SEQ ID NO: 11], vp1 [SEQ ID NO: 13], vp2 [SEQ ID NO: 15], and vp3 [SEQ IID NO: 17] and functional fragments thereof while the sequences of the rep and cap proteins have been found to be closely related to those of AAV-6, there are differences in the amino acid sequences (see Table 1 below), as well as differences in the recognition of these proteins by the immune system. However, one of skill in the art may readily select other suitable proteins or protein fragments of biological interest. Suitably, such fragments are at least 8 amino acids in length. However, fragments of other desired lengths may be readily utilized. Such fragments may be produced recombinantly or by other suitable means, e.g., chemical synthesis.

The sequences, proteins, and fragments of the invention may be produced by any suitable means, including recombinant production, chemical synthesis, or other synthetic means. Such production methods are within the knowledge of those of skill in the art and are not a limitation of the present invention.

### II. VIRAL VECTORS

In another aspect, the present invention provides vectors which utilize the AAV-1 sequences of the invention, including fragments thereof, for delivery of a heterologous gene or other nucleic acid sequences to a target cell. Suitably, these heterologous sequences (i.e., a transgene) encode a protein or gene product which is capable of being expressed in the target cell. Such a transgene may be constructed in the form of a "minigene". Such a "minigene" includes selected heterologous gene sequences and the other regulatory elements necessary to transcribe the gene and express the gene product in a host cell. Thus, the gene sequences are operatively linked to regulatory components in a manner which permit their transcription. Such components include conventional regulatory elements necessary to drive expression of the transgene in a cell containing the viral vector. The minigene may also contain a selected promoter which is linked to the transgene and located, with other regulatory elements, within the selected viral sequences of the recombinant vector.

Selection of the promoter is a routine matter and is not a limitation of this invention. Useful promoters may be constitutive promoters or regulated (inducible) promoters, which will enable control of the timing and amount of the transgene to be expressed. For example, desirable promoters include the cytomegalovirus (CMV) immediate early promoter/enhancer [see, e.g., Boshart et al, Cell, 41:521-530 (1985)], the Rous sarcoma virus LTR promoter/enhancer, and the chicken cytoplasmic β-actin promoter [T. A. Kost et al, Nucl. Acids Res., 11 (23):8287 (1983)]. Still other desirable promoters are the albumin promoter and an AAV P5 promoter. Optionally, the selected promoter is used in conjunction with a heterologous enhancer, e.g., the β-actin promoter may be used in conjunction with the CMV enhancer. Yet other suitable or desirable promoters and enhancers may be selected by one of skill in the art.

The minigene may also desirably contain nucleic acid sequences heterologous to the viral vector sequences including sequences providing signals required for efficient polyadenylation of the transcript (poly-A or pA) and introns with functional splice donor and acceptor sites. A common poly-A sequence which is employed in the exemplary vectors of this invention is that derived from the papovavirus SV-40. The poly-A sequence generally is inserted in the minigene downstream of the transgene sequences and upstream of the viral vector sequences. A common intron sequence is also derived from SV-40, and is referred to as the SV40 T intron sequence. A minigene of the present invention may also contain such an intron, desirably located between the promoter/enhancer sequence and the transgene. Selection of these and other common vector elements are conventional [see, e.g., Sambrook et al, "Molecular Cloning. A Laboratory Manual", 2d edit., Cold Spring Harbor Laboratory, New York (1989) and references cited therein] and many such sequences are available from commercial and industrial sources as well as from Genebank.

The selection of the transgene is not a limitation of the present invention. Suitable transgenes may be readily selected from among desirable reporter genes, therapeutic genes, and optionally, genes encoding immunogenic polypeptides. Examples of suitable reporter genes include β-galactosidase (β-gal), an alkaline phosphatase gene, and green fluorescent protein (GFP). Examples of therapeutic genes include, cytokines, growth factors, hormones, and differentiation factors, among others. The transgene may be readily selected by one of skill in the art. See, e.g., WO 98/09657, which identifies other suitable transgenes.

Suitably, the vectors of the invention contain, at a minimum, cassettes which consist of fragments of the AAV-1 sequences and proteins. In one embodiment, a vector of the invention comprises a selected transgene, which is flanked by a 5' ITR and a 3' ITR, at least one of which is an AAV-1 ITR of the invention. Suitably, vectors of the invention may contain a AAV-1 P5 promoter of the invention. In yet another embodiment, a plasmid or vector of the invention contains AAV-1 rep sequences. In still another embodiment, a plasmid or vector of the invention contains at least one of the AAV-1 cap proteins of the invention. Most suitably, these AAV-1-derived vectors are assembled into viral vectors, as described herein.

### A. AAV Viral Vectors

In one aspect, the present invention provides a recombinant AAV-1 viral vector produced using the AAV-1 capsid proteins of the invention. The packaged rAAV-1 virions of the invention may contain, in addition to a selected minigene, other AAV-1 sequences, or may contain sequences from other AAV serotypes.

Methods of generating rAAV virions are well known and the selection of a suitable method is not a limitation on the present invention. See, e.g., K. Fisher et al, J. Virol., 70:520-532 (1993) and US Patent 5,478,745. In one suitable method, a selected host cell is provided with the AAV sequence encoding a rep protein, the gene encoding the AAV cap protein and with the sequences for packaging and subsequent delivery. Desirably, the method utilizes the sequences encoding the AAV-1 rep and/or cap proteins of the invention.

In one embodiment, the rep/cap genes and the sequences for delivery are supplied by co-transfection of vectors carrying these genes and sequences. In one currently preferred embodiment, a cis (vector) plasmid, a trans plasmid containing the rep and cap genes, and a plasmid containing the adenovirus helper genes are cotransfected into a suitable cell line, e.g., 293. Alternatively, one or more of these functions may be provided in trans via separate vectors, or may be found in a suitably engineered packaging cell line.

An exemplary cis plasmid will contain, in 5' to 3' order, AAV 5' ITR, the selected transgene, and AAV 3' ITR. In one desirable embodiment, at least one of the AAV ITRs is a 143 nt AAV-1 ITR. However, other AAV serotype ITRs may be readily selected. Suitably, the full-length ITRs are utilized. However, one of skill in the art can readily prepare modified AAV ITRs using conventional techniques. Similarly, methods for construction of such plasmids is well known to those of skill in the art.

A trans plasmid for use in the production of the rAAV-1 virion particle may be prepared according to known techniques. In one desired embodiment, this plasmid contains the rep and cap proteins of AAV-1, or functional fragments thereof Alternatively, the rep sequences may be from another selected AAV serotype.

The cis and trans plasmid may then be co-transfected with a wild-type helper virus (e.g., Ad2, Ad5, or a herpesvirus), or more desirably, a replication - defective adenovirus, into a selected host cell. Alternatively, the cis and trans plasmid may be co-transfected into a selected host cell together with a transfected plasmid which provides the necessary helper functions. Selection of a suitable host cell is well within the skill of those in the art and include such mammalian cells as 293 cells, HeLa cells, among others.

Alternatively, the cis plasmid and, optionally the trans plasmid, may be transfected into a packaging cell line which provides the remaining helper functions necessary for production of a rAAV containing the desired AAV-1 sequences of the invention. An example of a suitable packaging cell line, where an AAV-2 capsid is desired, is B-50, which stably expresses AAV-2 rep and cap genes under the control of a homologous P5 promoter. This cell line is characterized by integration into the cellular chromosome of multiple copies (at least 5 copies) of P5-rep-cap gene cassettes in a concatomer form. This B-50 cell line was deposited with the American Type Culture Collection, 10801 University Boulevard, Manassas, Virginia 20110-2209, on September 18, 1997 under Accession No. CRL-12401 pursuant to the provisions of the Budapest Treaty. However, the present invention is not limited as to the selection of the packaging cell line.

Exemplary transducing vectors based on AAV-1 capsid proteins have been tested both *in vivo and in vitro,* as described in more detail in Example 4. In these studies, it was demonstrated that recombinant AAV vector with an AAV-1 virion can transduce both mouse liver and muscle. These, and other AAV-1 based gene therapy vectors which may be generated by one of skill in the art are beneficial for gene delivery to selected host cells and gene therapy patients since the neutralization antibodies of AAV-1 present in much of the human population exhibit different patterns from other AAV serotypes and therefore do not neutralize the AAV-1 virions. One of skill in the art may readily prepare other rAAV viral vectors containing the AAV-1 capsid proteins provided herein using a variety of techniques known to those of skill in the art. One may similarly prepare still other rAAV viral vectors containing AAV-1 sequence and AAV capsids of another serotype.

### B. Other Viral Vectors

One of skill in the art will readily understand that the AAV-1 sequences of the invention can be readily adapted for use in these and other viral vector systems for *in vitro, ex vivo or in vivo* gene delivery. Particularly well suited for use in such viral vector systems are the AAV-1 ITR sequences, the AAV-1 rep, the AAV-1 cap, and the AAV-1 P5 promoter sequences.

For example, in one desirable embodiment, the AAV-1 ITR sequences of the invention may be used in an expression cassette which includes AAV-1 5' ITR, a non-AAV DNA sequences of interest (e.g., a minigene), and 3' ITR and which lacks functional rep/cap. Such a cassette containing an AAV-1 ITR may be located on a plasmid for subsequent transfection into a desired host cell, such as the cis plasmid described above. This expression cassette may further be provided with an AAV capsid of a selected serotype to permit infection of a cell or stably transfected into a desired host cell for packaging of rAAV virions. Such an expression cassette may be readily adapted for use in other viral systems, including adenovirus systems and lentivirus systems. Methods of producing Ad/AAV vectors are well known to those of skill in the art. One desirable method is described in PCT/US95/14018. However, the present invention is not limited to any particular method.

Another aspect of the present invention is the novel AAV-1 P5 promoter sequences which are located in the region spanning nt 236 - 299 of SEQ ID NO: 1. This promoter is useful in a variety of viral vectors for driving expression of a desired transgene.

Similarly, one of skill in the art can readily select other fragments of the AAV-1 genome of the invention for use in a variety of vector systems. Such vectors systems may include, e.g., lentiviruses, retroviruses, poxviruses, vaccinia viruses, and adenoviral systems, among others. Selection of these vector systems is not a limitation of the present invention.

### C. Host Cells And Packaging Cell Lines

In yet another aspect, the present invention provides host cells which may be transiently transfected with AAV-1 nucleic acid sequences of the invention to permit expression of a desired transgene or production of a rAAV particle. For example, a selected host cell may be transfected with the AAV-1 P5 promoter sequences and/or the AAV-1 5' ITR sequences using conventional techniques. Providing AAV helper functions to the transfected cell lines of the invention results in packaging of the rAAV as infectious rAAV particles. Such cell lines may be produced in accordance with known techniques [see, e.g, US Patent No. 5,658,785], making use of the AAV-1 sequences of the invention.

Alternatively, host cells of the invention may be stably transfected with a rAAV expression cassette of the invention, and with copies of AAV-1 rep and cap genes. Suitable parental cell lines include mammalian cell lines and it may be desirable to select host cells from among non-simian mammalian cells. Examples of suitable parental cell lines include, without limitation, HeLa [ATCC CCL 2], A549 [ATCC Accession No. CCL 185], KB [CCL 17], Detroit [e.g., Detroit 510, CCL 72] and WI-38 [CCL 75] cells. These cell lines are all available from the American Type Culture Collection, 10801 University Boulevard, Manassas, Virginia 20110-2209 USA. Other suitable parent cell lines may be obtained from other sources and may be used to construct stable cell lines containing the P5 and/or AAV rep and cap sequences of the invention.

Recombinant vectors generated as described above are useful for delivery of the DNA of interest to cells.

### III. METHODS OF DELIVERING GENES VIA AAV-1 DERIVED VECTORS

In another aspect, the-present invention provides a method for delivery of a transgene to a host which involves transfecting or infecting a selected host cell with a recombinant viral vector generated with the AAV-1 sequences (or functional fragments thereof) of the invention. Methods for delivery are well known to those of skill in the art and are not a limitation of the present invention.

In one desirable embodiment, the invention provides a method for AAVmediated delivery of a transgene to a host. This method involves transfecting or infecting a selected host cell with a recombinant viral vector containing a selected transgene under the control of sequences which direct expression thereof and AAV-1 capsid proteins.

Optionally, a sample from the host may be first assayed for the presence of antibodies to a selected AAV serotype. A variety of assay formats for detecting neutralizing antibodies are well known to those of skill in the art. The selection of such an assay is not a limitation of the present invention. See, e.g., Fisher et al, Nature Med., 3(3):306-312 (March 1997) and W. C. Manning et al, Human Gene Therapy, 9:477-485 (March 1, 1998). The results of this assay may be used to determine which AAV vector containing capsid proteins of a particular serotype are preferred for delivery, e.g., by the absence of neutralizing antibodies specific for that capsid serotype.

In one aspect of this method, the delivery of vector with AAV-1 capsid proteins may precede or follow delivery of a gene via a vector with a different serotype AAV capsid protein. Thus, gene delivery via rAAV vectors may be used for repeat gene delivery to a selected host cell. Desirably, subsequently administered rAAV vectors carry the same transgene as the first rAAV vector, but the subsequently administered vectors contain capsid proteins of serotypes which differ from the first vector. For example, if a first vector has AAV-2 capsid proteins, subsequently administered vectors may have capsid proteins selected from among the other serotypes, including AAV-1, AAV-3A, AAV-3B, AAV-4 and AAV-6.

Thus, a rAAV-1-derived recombinant viral vector of the invention provides an efficient gene transfer vehicle which can deliver a selected transgene to a selected host cell *in vivo or ex vivo* even where the organism has neutralizing antibodies to one or more AAV serotypes. These compositions are particularly well suited to gene delivery for therapeutic purposes. However, the compositions of the invention may also be useful in immunization. Further, the compositions of the invention may also be used for production of a desired gene product *in vitro.*

The above-described recombinant vectors may be delivered to host cells according to published methods. An AAV viral vector bearing the selected transgene may be administered to a patient, preferably suspended in a biologically compatible solution or pharmaceutically acceptable delivery vehicle. A suitable vehicle includes sterile saline. Other aqueous and non-aqueous isotonic sterile injection solutions and aqueous and non-aqueous sterile suspensions known to be pharmaceutically acceptable carriers and well known to those of skill in the art may be employed for this purpose.

The viral vectors are administered in sufficient amounts to transfect the cells and to provide sufficient levels of gene transfer and expression to provide a therapeutic benefit without undue adverse effects, or with medically acceptable physiological effects, which can be determined by those skilled in the medical arts. Conventional and pharmaceutically acceptable routes of administration include, but are not limited to, direct delivery to the liver, oral, intranasal, intravenous, intramuscular, subcutaneous, intradermal, and other parental routes of administration. Routes of administration may be combined, if desired.

Dosages of the viral vector will depend primarily on factors such as the condition being treated, the age, weight and health of the patient, and may thus vary among patients. For example, a therapeutically effective human dosage of the viral vector is generally in the range of from about 1 ml to about 100 ml of solution containing concentrations of from about 1 x 10⁹ to 1 x 10¹⁶ genomes virus vector. A preferred human dosage may be about 1 x 10¹³ to 1 x 10¹⁶ AAV genomes. The dosage will be adjusted to balance the therapeutic benefit against any side effects and such dosages may vary depending upon the therapeutic application for which the recombinant vector is employed. The levels of expression of the transgene can be monitored to determine the frequency of dosage resulting in viral vectors, preferably AAV vectors containing the minigene. Optionally, dosage regimens similar to those described for therapeutic purposes may be utilized for immunization using the compositions of the invention. For *in vitro* production, a desired protein may be obtained from a desired culture following transfection of host cells with a rAAV containing the gene encoding the desired protein and culturing the cell culture under conditions which permits expression. The expressed protein may then be purified and isolated, as desired. Suitable techniques for transfection, cell culturing, purification, and isolation are known to those of skill in the art.

The following examples illustrate several aspects and embodiments of the invention.

### Example 1 - Generation of Infectious Clone of AAV-1

The replicated form DNA of AAV-1 was extracted from 293 cells that were infected by AAV-1 and wild type adenovirus type 5.

### A. Cell Culture and Virus

AAV-free 293 cells and 84-31 cells were provided by the human application laboratory of the University of Pennsylvania. These cells were cultured in Dulbecco's Modified Eagle Medium with 10% fetal bovine serum (Hyclone), penicillin (100 U/ml) and streptomycin at 37°C in a moisturized environment supplied with 5% C0₂. The 84-31 cell line constitutively expresses adenovirus genes E1a, E1b, E4/ORF6, and has been described previously [K. J. Fisher, J. Virol., 70:520-532 (1996)]. AAV-1 (ATCC VR-645) seed stock was purchased from American Type Culture Collection (ATCC, Manassas, VA). AAV viruses were propagated in 293 cells with wild type Ad5 as a helper virus.

### B. Recombinant AAV Generation

The recombinant AAV viruses were generated by transfection using an adenovirus free method. Briefly, the cis plasmid (with AAV ITR), trans plasmid (with AAV rep gene and cap gene) and helper plasmid (pFΔ13, with essential regions from the adenovirus genome) were simultaneously co-transfected into 293 cells in a ratio of 1:1:2 by calcium phosphate precipitation. The pFΔ 13 helper plasmid has an 8 kb deletion in the adenovirus E2B region and has deletions in most of the late genes. This helper plasmid was generated by deleting the RsrII fragment from pFG140 (Microbix, Canada). Typically, 50 µg of DNA (cis:trans:PFΔ13 at ratios of 1:1:2, respectively) was transfected onto a 15 cm tissue culture dish. The cells were harvested 96 hours post-transfection, sonicated and treated with 0.5% sodium deoxycholate (37°C for 10 min). Cell lysates were then subjected to two rounds of a CsCl gradient. Peak fractions containing AAV vector were collected, pooled, and dialyzed against PBS before injecting into animals. To make rAAV virus with AAV-1 I virion, the pAV1H or p5E18 (2/1) was used as the *trans* plasmid to provide rep and cap function.

For the generation of rAAV based on AAV-2, p5E 18 was used as the *trans* plasmid since it greatly improved the rAAV yield. This plasmid, p5E18(2/2), expresses AAV-2 Rep and Cap and contains a P5 promoter relocated to a position 3' to the Cap gene, thereby minimizing expression of Rep78 and Rep68. The strategy was initially described by Li et al, J. Virol., 71:5236-5243 (1997). P5E 18(2/2) was constructed in the following way. The previously described pMMTV-trans vector (i.e., the mouse mammary tumor virus promoter substituted for the P5 promoter in an AAV-2-based vector) was digested with *Sma*I and *Cla*I*,* filled in with the Klenow enzyme, and then recircularized with DNA ligase. The resulting construct was digested with *Xba*I, filled in, and ligated to the blunt-ended BamHI-*Xba*I fragment from pCR-p5, constructed in the following way. The P5 promoter of AAV was amplified by PCR and the amplified fragment was subsequently cloned into pCR2.1 (Invitrogen) to yield pCR-P5. The helper plasmid pAV1H was constructed by cloning the *Bfa*I fragment of pAAV-2 into pBluescript II-SK(+) at the *Bcor*V and *Sma*I sites. The 3.0-kb *Xba*I-*Kpn*I fragment from p5E18(2/2), the 2.3-kb *Xba*I-*Kpn*I fragment from pAV1H, and the 1.7-kb KpnI fragment from p5E18(2/2) were incorporated into a separate plasmid P5E18(2/1), which contains AAV-2 Rep, AAV-1 Cap, and the AAV-2 P5 promoter located 3' to the Cap gene. Plasmid p5E18(2/1) produced 10- to 20-fold higher quantities of the vector than pAV1H (i.e., 10¹² genomes/50 15-cm² plates).

### C. DNA Techniques

Hirt DNA extraction was performed as described in the art with minor modification [R.J. Samulski et al., Cell, 33:135-143 (1983)]. More particularly, Hirst solution without SDS was used instead of using original Hirt solution containing SDS. The amount of SDS present in the original Hirst solution was added after the cells had been fully suspended. To construct AAV-1 infectious clone, the Hirt DNA from AAV-1 infected 293 cells was repaired with Klenow enzyme (New England Biolabs) to ensure the ends were blunt. The treated AAV-1 Hirt DNA was then digested with *BamH*I and cloned into three vectors, respectively. The internal *BamH*I was cloned into pBlueScript II-SK+ cut with *BamH*I to get pAV1-BM. The left and right fragments were cloned into pBlueScript II-SK+ cut with *BamH*I + EcoRV to obtain pAV1-BL and pAVI-BR, respectively. The AAV sequence in these three plasmids were subsequently assembled into the same vector to get AAV-1 infectious clone pAAV-1. The helper plasmid for recombinant AAV-1 virus generation was constructed by cloning the Bfa I fragment of pAAV-1 into pBlueScript II-SK+ at the EcoRV site.

Analysis of the Hirt DNA revealed three bands, a dimer at 9.4 kb, a monomer at 4.7 kb and single-stranded DNA at 1.7 kb, which correlated to different replication forms of AAV-1. The monomer band was excised from the gel and then digested with *BamH*I*.* This resulted in three fragments of 1.1 kb, 0.8 kb and 2.8 kb. This pattern is in accordance with the description by Bantel-schaal and zur Hausen, Virol., 134(1): 52-63 (1984). The 1.1 kb and 2.8 kb *Bam*HI fragments were cloned into pBlueScript-KS(+) at *Bam*HI and EcoRV site. The internal 0.8 kb fragment was cloned into *BamH*I site of pBlueScript-KS(+).

These three fragments were then subcloned into the same construct to obtain a plasmid (pAAV-1) that contained the full sequence of AAV-1. The pAAV-1 was then tested for its ability to rescue from the plasmid backbone and package infectious virus. The pAAV-1 was then transfected to 293 cells and supplied with adenovirus type as helper at MOI 10. The virus supernatant was used to reinfect 293 cells.

For Southern blot analysis, Hirt DNA was digested with *Dpn*I to remove bacteria-borne plasmid and probed with internal *BamH*I fragment of AAV-1. The membrane was then washed at high stringency conditions, which included: twice 30 minutes with 2X SSC, 0.1% SDS at 65°C and twice 30 minutes with 0.1X SSC, 0.1% SDS at 65°C. The membrane was then analyzed by both phosphor image and X-ray autoradiography. The results confirmed that pAAV-1 is indeed an infectious clone of AAV serotype 1.

### Example 2 - Sequencing Analysis of AAV-1

The entire AAV-1 genome was then determined by automatic sequencing and was found to be 4718 nucleotides in length (Figs. 1A-1C). For sequencing, an ABI 373 automatic sequencer as used to determine the sequences for all plasmids and PCR fragments related to this study using the FS dye chemistry. All sequences were confirmed by sequencing both plus and minus strands. These sequences were also confirmed by sequencing two independent clones of pAV-BM, pAV-BL and pAV-BR. Since the replicated form of AAV-1 DNA served as the template for sequence determination, these sequences were also confirmed by sequencing a series of PCR products using original AAV-1 seed stock as a template.

The length of AAV-1 was found to be within the range of the other serotypes: AAV-3 (4726 nucleotides), AAV-4 (4774 nucleotides), AAV-2 (4681 nucleotides), and AAV-6 (4683 nucleotides).

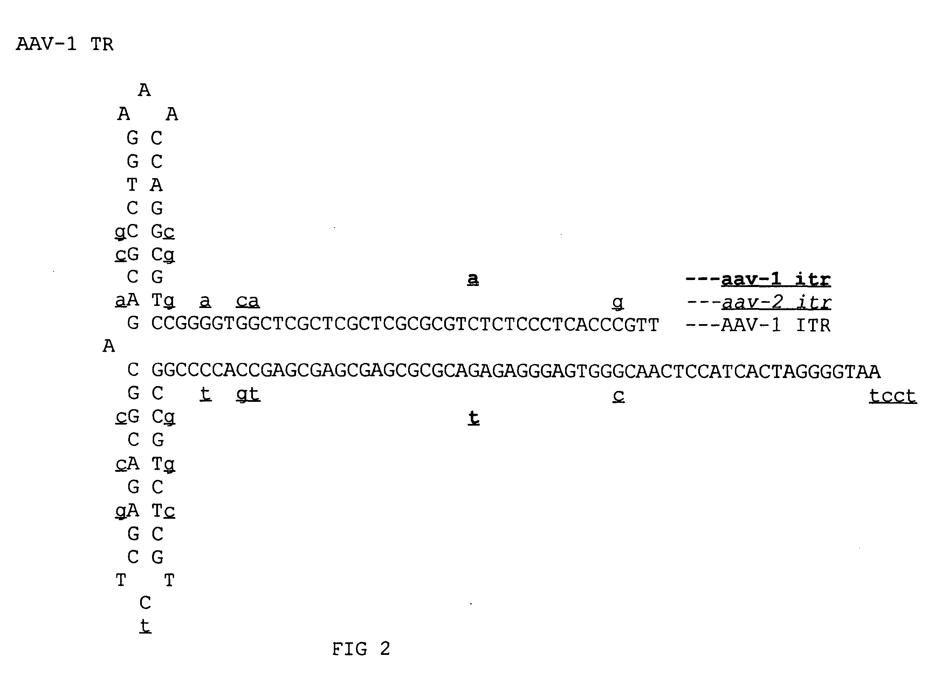

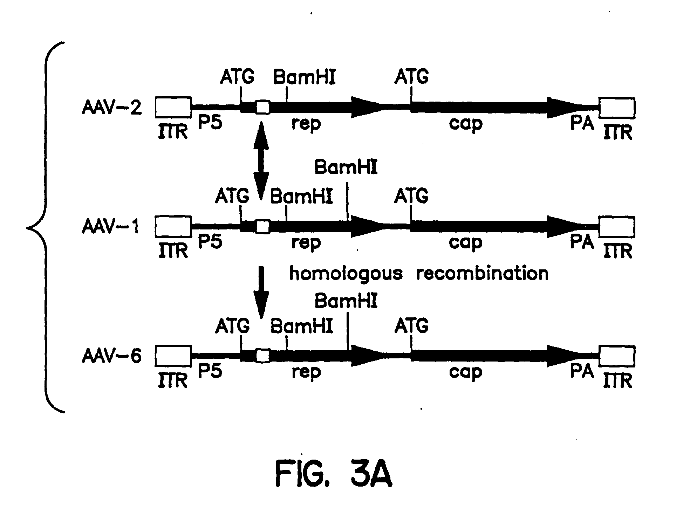

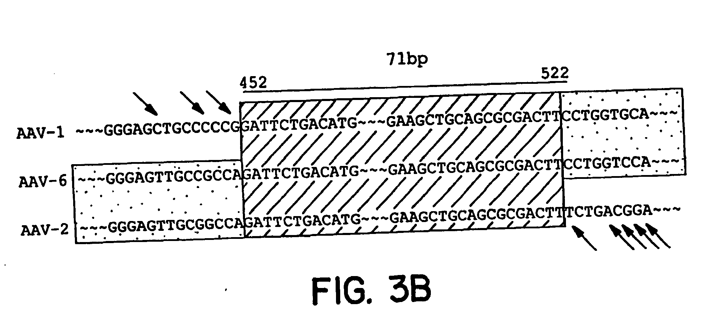

The AAV-1 genome exhibited similarities to other serotypes of adeno-associated viruses. Overall, it shares more than 80% identity with other known AAV viruses as determined by the computer program Megalign using default settings [DNASTAR, Madison, WI]. The key features in AAV-2 can also be found in AAV-1. First, AAV-1 has the same type of inverted terminal repeat which is capable of forming T-shaped hairpin structures, despite the differences at the nucleotide level (Figs. 2 and 3). The sequences of right ITRs and left ITRs of AAV-1 are identical. The AAV TR sequence is subdivided into A, A', B, B', C, C', D and D' [Bern, cited above].

These AAV ITR sequences are also virtually the same as those found in AAV-6 right ITR, there being one nucleotide difference in each of A and A' sequence, and the last nucleotide of the D sequence. Second, the AAV-2 rep binding motif [GCTCGCTCGCTCGCTG (SEQ ID NO: 20)] is well conserved. Such motif can also be found in the human chromosome 19 AAV-2 pre-integration region. Finally, non-structural and structural coding regions, and regulatory elements similar to those of other AAV serotypes also exist in AAV-1 genome.

Although the overall features of AAV terminal repeats are very much conserved, the total length of the AAV terminal repeat exhibits divergence. The terminal repeat of AAV-1 consists of 143 nucleotides while those of AAV-2, AAV-3, and AAV-4 are about 145 or 146 nucleotides. The loop region of AAV-1 ITR most closely resembles that of AAV-4 in that it also uses TCT instead of the TTT found in AAV-2 and AAV-3. The possibility of sequencing error was eliminated using restriction enzyme digestion, since these three nucleotides are part of the SacI site (gagctc; nt 69-74 of SEQ ID NO: 1). The p5 promoter region of AAV-1 shows more variations in nucleotide sequences with other AAV serotypes. However, it still maintains the critical regulatory elements. The two copies of YY 1 [See, Fig. 1A-1C] sites seemed to be preserved in all known AAV serotypes, which have been shown to be involved in regulating AAV gene expression. In AAV-4, there are 56 additional nucleotides inserted between YY1 and E-box/USF site, while in AAV-1, there are 26 additional nucleotides inserted before the E-box/USF site. The p 19 promoter, p40 promoter and polyA can also be identified from the AAV-1 genome by analogy to known AAV serotypes, which are also highly conserved.

Thus, the analysis of AAV terminal repeats of various serotypes showed that the A and A' sequence is very much conserved. One of the reasons may be the Rep binding motif (GCTC)₃GCTG [SEQ ID NO: 20]. These sequences appear to be essential for AAV DNA replication and site-specific integration. The same sequence has also been shown to be preserved in a monkey genome [Samulski, personal communication]. The first 8 nucleotides of the D sequence are also identical in all known AAV serotypes. This is in accordance with the observation of the Srivastava group that only the first 10 nucleotides are essential for AAV packaging [X.S. Wang et al, J. Virol., 71:3077-3082 (1997); X.S. Wang et al, J. Virol., 71:1140-1146 (1997)]. The function of the rest of the D sequences still remain unclear. They may be somehow related to their tissue specificities. The variation of nucleotide in B and C sequence may also suggest that the secondary structure of the ITRs is more critical for its biological function, which has been demonstrated in many previous publications.

### Example 3 - Comparison of AAV-1 Sequences

The nucleotide sequences of AAV-1, obtained as described above, were compared with known AAV sequences, including AAV-2, AAV-4 and AAV-6 using DNA Star Megalign. This comparison revealed a stretch of 71 identical nucleotides shared by AAV-1, AAV-2 and AAV-6. See, Figs. 1A-1C.

This comparison further suggested that AAV-6 is a hybrid formed by homologous recombination of AAV-1 and AAV-2. See, Figs. 3A and 3B. These nucleotides divide the AAV-6 genome into two regions. The 5' half of AAV-6 of 522 nucleotides is identical to that of AAV-2 except in 2 positions. The 3' half of AAV-6 including the majority of the rep gene, complete cap gene and 3' ITR is 98% identical to AAV-1.

Biologically, such recombination may enable AAV-1 to acquire the ability to transmit through the human population. It is also interesting to note that the ITRs of AAV-6 comprise one AAV-1 ITR and one AAV-2 ITR. The replication model of defective parvovirus can maintain this special arrangement. Studies on AAV integration have shown that a majority of AAV integrants carries deletions in at least one of the terminal repeats. These deletions have been shown to be able to be repaired through gene conversion using the other intact terminal repeat as a template. Therefore, it would be very difficult to maintain AAV-6 as a homogenous population when an integrated copy of AAV-6 is rescued from host cells with helper virus infection. The AAV-6 with two identical AAV-2 ITRs or two identical AAV-1 ITRs should be the dominant variants. The AAV-6 with two AAV-1 ITRs has been observed by Russell's group [Rutledge, cited above (1998)]. So far there is no report on AAV-6 with two AAV-2 ITRs. Acquirement of AAV-2 P5 promoter by AAV-6 may have explained that AAV-6 have been isolated from human origin while AAV-1 with the same virion has not. The regulation of P5 promoter between different species of AAV may be different *in vivo.* This observation suggests the capsid proteins of AAV were not the only determinants for tissue specificity.

Although it is clear that AAV-6 is a hybrid of AAV-1 and AAV-2, AAV-6 has already exhibited divergence from either AAV-1 or AAV-2. There are two nucleotide differences between AAV-6 and AAV-2 in their first 450 nucleotides. There are about 1% differences between AAV-6 and AAV-1 in nucleotide levels from nucleotides 522 to the 3' end. There also exists a quite divergent region (nucleotide 4486-4593) between AAV-6 and AAV-1 (Figs. 1A-1C). This region does not encode any known proteins for AAVs. These differences in nucleotide sequences may suggest that AAV-6 and AAV-1 have gone through some evolution since the recombination took place. Another possible explanation is that there exists another variant of AAV-1 which has yet to be identified. So far, there is no evidence to rule out either possibility. It is still unknown if other hybrids (AAV-2 to AAV-4, etc.) existed in nature.

The coding region of AAV-1 was deduced by comparison with other known AAV serotypes. Table 1 illustrates the coding region differences between AAV-1 and AAV-6. The amino acid residues are deduced according to AAV-2.

With reference to the amino acid position of AAV-1, Table 1 lists the amino acids of AAV-1 which have been changed to the corresponding ones of AAV-6. The amino acids of AAV-1 are shown to the left of the arrow. Reference may be made to SEQ ID NO: 5 of the amino acid sequence of AAV-1 Rep 78 and to SEQ ID NO: 13 for the amino acid sequence of AAV-1 VP1.

**Table 1**

| Coding region variations between AAV-1 and AAV-6 | | | | |
|---|---|---|---|---|
| Rep protein (Rep78) | | | Cap protein (VP1) | |
| Position(s) | Amino acids | | Position(s) | Amino acids |
| 28 | S-N | | 129 | L-F |
| 191 | Q-H | | 418 | E-D |
| 192 | H-D | | 531 | E-K |
| 308 | E-D | | 584 | F-L |
| | | | 598 | A-V |
| | | | 642 | N-H |

It was surprising to see that the sequence of the AAV-1 coding region is almost identical to that of AAV-6 from position 452 to the end of coding region (99%). The first 508 nucleotides of AAV-6 have been shown to be identical to those of AAV-2 [Rutledge, cited above (1998)]. Since the components of AAV-6 genome seemed to be AAV-2 left ITR - AAV-2 p5 promoter - AAV-1 coding region - AAV-1 right ITR, it was concluded that AAV-6 is a naturally occurred hybrid between AAV-1 and AAV-2.

### Example 4 - Gene Therapy Vector Based on AAV-1

Recombinant gene transfer vectors based on AAV-1 viruses were constructed by the methods described in Example 1. To produce a hybrid recombinant virus with AAV-1 virion and AAV-2 ITR, the AAV-1 trans plasmid (pAVIH) and the AAV-2 cis-lacZ plasmid (with AAV-2 ITR) were used. The AAV-2 ITR was used in this vector in view of its known ability to direct site-specific integration. Also constructed for use in this experiment was an AAV-1 vector carrying the green fluorescent protein (GFP) marker gene under the control of the immediate early promoter of CMV using pAV1H as the trans plasmid.

### A. rAAV-1 Viruses Transfect Host Cells in Vitro

84-31 cells, which are subclones of 293 cells (which express adenovirus E1a, E1b) which stably express E4/ORFS, were infected with rAAV-1 GFP or rAAV-lacZ. High levels of expression of GFP and lacZ was detected in the cultured 84-31 1 cells. This suggested that rAAV-1 based vector was very similar to AAV-2 based vectors in ability to infect and expression levels.

### B. rAAV-1 Viruses Transfect Cells in Vivo

The performance of AAV-1 based vectors was also tested *in vivo.* The rAAV-1 CMV-α1AT virus was constructed as follows. The EcoRI fragment of pAT85 (ATCC) containing human α1-antitrypsin (α1AT) cDNA fragment was blunted and cloned into PCR (Promega) at a SmaI site to obtain PCR-α1AT. The CMV promoter was cloned into PCR-α1AT at the XbaI site. The Alb-α1AT expression cassette was removed by XhoI and ClaI and cloned into pAV1H at the XbaI site. This vector plasmid was used to generate AAV-1-CMV-α1AT virus used in the experiment described below.

For screening human antibodies against AAV, purified AAV virus is lysed with Ripa buffer (10 mM Tris pH 8.2, 1% Triton X-100, 1% SDS, 0.15 M NaCl) and separated in 10% SDS-PAGE gel. The heat inactivated human serum was used at a 1 to 1000 dilution in this assay. The rAAV-1 CMV-α1AT viruses were injected into Rag-1 mice through tail vein injection at different dosages. The concentration of human α1-antitrypsin in mouse serum was measured using ELISA. The coating antibody is rabbit anti-human human α1-antitrypsin (Sigma). The goat-antihuman α1-antitrypsin (Sigma) was used as the primary detection antibodies. The sensitivity of this assay is around 0.3 ng/ml to 30 ng/ml. The expression of human α-antitrypsin in mouse blood can be detected in a very encouraging level. This result is shown in Table 2.

**Table 2**

| Human Antitrypsin Expressed in Mouse Liver | | |
|---|---|---|
| Amount of virus injected | Week 2 (ng/ml) | Week 4 (ng/ml) |
| 2x10¹⁰ genomes | 214.2 | 171.4 |
| 1x10¹⁰ genomes | 117.8 | 109.8 |
| 5x10¹⁰ genomes | 64.5 | 67.8 |
| 2.5x10¹⁰ genomes | 30.9 | 58.4 |

rAAV-1 CMV-1acZ viruses were also injected into the muscle of C57BL6 mice and similar results were obtained. Collectively, these results suggested that AAV-1 based vector would be appropriate for both liver and muscle gene delivery.

### Example 5 - Neutralizing Antibodies Against AAV-1

Simple and quantitative assays for neutralizing antibodies (NAB) to AAV-1 and AAV-2 were developed with recombinant vectors. A total of 33 rhesus monkeys and 77 normal human subjects were screened.

### A. Nonhuman Primates

Wild-caught juvenile rhesus monkeys were purchased from Covance (Alice, Tex.) and LABS of Virginia (Yemassee, SC) and kept in full quarantine. The monkeys weighed approximately 3 to 4 kg. The nonhuman primates used in the Institute for Human Gene Therapy research program are purposefully bred in the United States from specific-pathogen-free closed colonies. All vendors are US Department of Agriculture class A dealers. The rhesus macaques are therefore not infected with important simian pathogens, including the tuberculosis agent, major simian lentiviruses (simian immunodeficiency virus and simian retroviruses), and cercopithecine herpesvirus. The animals are also free of internal and external parasites. The excellent health status of these premium animals minimized the potential for extraneous variables. For this study, serum was obtained from monkeys prior to initiation of any protocol.

NAB titers were analyzed by assessing the ability of serum antibody to inhibit the transduction of reporter virus expressing green fluorescent protein (GFP) (AAV1-GFP or AAV2-GFP) into 84-31 cells. Various dilutions of antibodies preincubated with reporter virus for 1 hour at 37°C were added to 90% confluent cell cultures. Cells were incubated for 48 hours and the expression of green fluorescent protein was measured by FluoroImaging (Molecular Dynamics). NAB titers were calculated as the highest dilution at which 50% of the cells stained green.

Analysis of NAB in rhesus monkeys showed that 61 % of animals tested positive for AAV-1; a minority (24%) has NAB to AAV-2. Over one-third of animals had antibodies to AAV-1 but not AAV-2 (i.e., were monospecific for AAV-1), whereas no animals were positive for AAV-2 without reacting to AAV-1. These data support the hypothesis that AAV-1 is endemic in rhesus monkeys. The presence of true AAV-2 infections in this group of nonhuman primates is less clear, since cross-neutralizing activity of an AAV-1 response to AAV-2 can not be ruled out. It is interesting that there is a linear relationship between AAV-2 NAB and AAV-1 NAB in animals that had both.

### B. Humans

For these neutralization antibody assays, human serum samples were incubated at 56°C for 30 min to inactivate complement and then diluted in DMEM. The virus (rAAV or rAd with either lacZ or GFP) was then mixed with each serum dilution (20X, 400X, 2000X, 4000X, etc.) and incubated for 1 hour at 37°C before applied to 90% confluent cultures of 84-31 cells (for AAV) or Hela cells (for adenovirus) in 96-well plates. After 60 minutes of incubation at culture condition, 100 µl additional media containing 20% FCS was added to make final culture media containing 10% FCS.

The result is summarized in Table 3.

**Table 3**

| Adenovirus | AAV-1 | AAV-2 | # of samples | Percentage |
|---|---|---|---|---|
| - | - | - | 41 | 53.2% |
| + | - | - | 16 | 20.8% |
| - | + | - | 0 | 0.0% |
| - | - | + | 2 | 2.6% |
| - | + | + | 2 | 2.6% |
| + | - | + | 3 | 3.9% |
| + | + | - | 0 | 0.0% |
| + | + | + | 13 | 16.9% |
| | | Total | 77 | 100% |

The human neutralizing antibodies against these three viruses seemed to be unrelated since the existence of neutralizing antibodies against AAV are not indications for antibodies against adenovirus. However, AAV requires adenovirus as helper virus, in most of the cases, the neutralizing antibodies against AAV correlated with the existence of neutralizing antibodies to adenovirus. Among the 77 human serum samples screened, 41% of the samples can neutralize the infectivity of recombinant adenovirus based on Ad5. 15/77 (19%) of serum samples can neutralize the transduction of rAAV-1 while 20/77 (20%) of the samples inhibit rAAV-2 transduction at 1 to 80 dilutions or higher. All serum samples positive in neutralizing antibodies for AAV-1 in are also positive for AAV-2. However, there are five (6%) rAAV-2 positive samples that failed to neutralize rAAV-1. In samples that are positive for neutralizing antibodies, the titer of antibodies also varied in the positive ones. The results from screening human sera for antibodies against AAVs supported the conclusion that AAV-1 presents the same epitome as that of AAV-2 to interact with cellular receptors since AAV-1 neutralizing human serums can also decrease the infectivity of AAV-2. - However, the profile of neutralizing antibodies for these AAVs is not identical, there are additional specific receptors for each AAV serotype.

### Example 6 - Recombinant AAV Viruses Exhibit Tissue Tropism

The recombinant AAV-1 vectors of the invention and the recombinant AAV-2 vectors [containing the gene encoding human α1-antitrypsin (α1AT) or murine erythropoietin (Epo) from a cytomegalovirus-enhanced β-actin promoter (CB)] were evaluated in a direct comparison to equivalent copies of AAV-2 vectors containing the same vector genes.

Recombinant viruses with AAV-1 capsids were constructed using the techniques in Example 1. To make rAAV with AAV-1 virions, pAV1H or p5E18 (2/1) was used as the *trans* plasmid to provide Rep and Cap functions. For the generation of the rAAV based on AAV-2, p5E 18(2/2) was used as the *trans* plasmid, since it greatly improved the rAAV yield. [Early experiments indicated similar *in vivo* performances of AAV-1 vectors produced with pAV1H and p5E 19 (2/1). All subsequent studies used AAV-1 vectors derived from p5E18(2/1) because of the increased yield.]

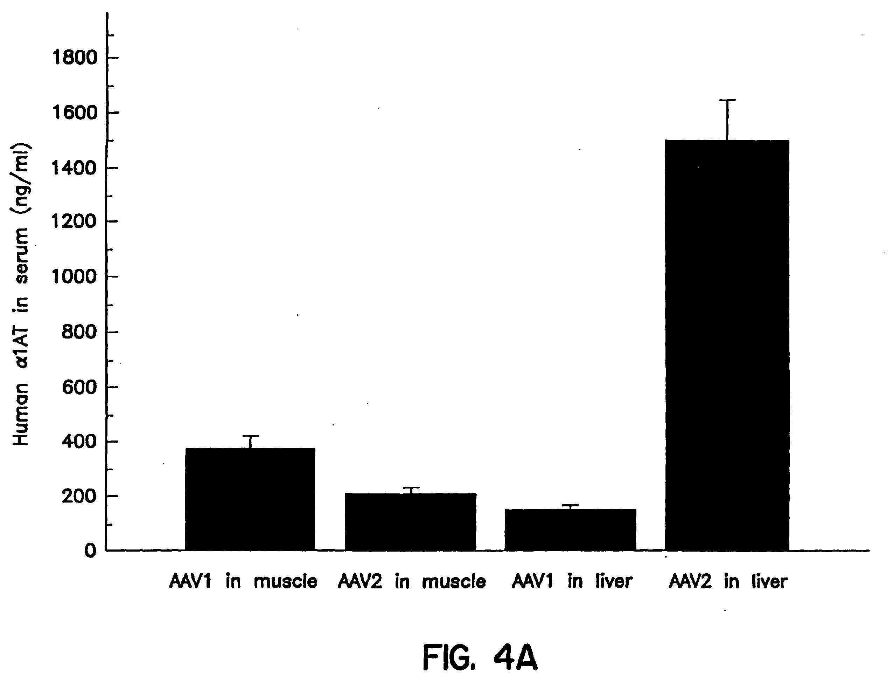

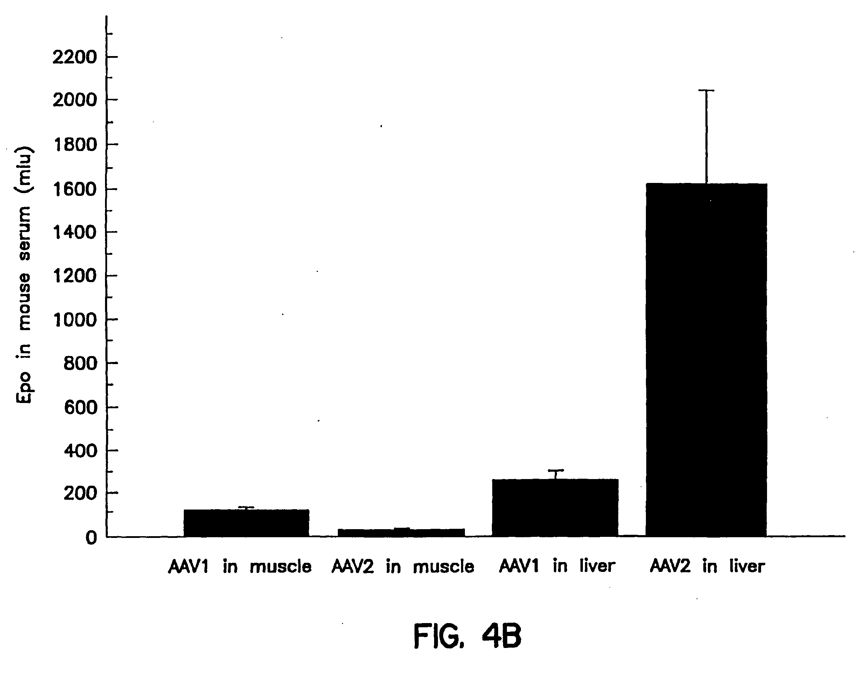

Equivalent stocks of the AAV-1 and AAV-2 vectors were injected intramuscularly (5 x 10¹⁰ genomes) or liver via the portal circulation (1 x 10¹¹ genomes) into immunodeficient mice, and the animals (four groups) were analyzed on day 30 for expression of transgene. See, Figs. 4A and 4B.

AAV-2 vectors consistently produced 10- to 50-fold more serum erythropoietin or α1-antitrypsin when injected into liver compared to muscle. (However, the AAV-1-delivered genes did achieve acceptable expression levels in the liver.) This result was very different from that for AAV-1 vectors, with which muscle expression was equivalent to or greater than liver expression. In fact, AAV-1 outperformed AAV-2 in muscle when equivalent titers based on genomes were administered.

### Example 7 - Gene Delivery via rAAV-1

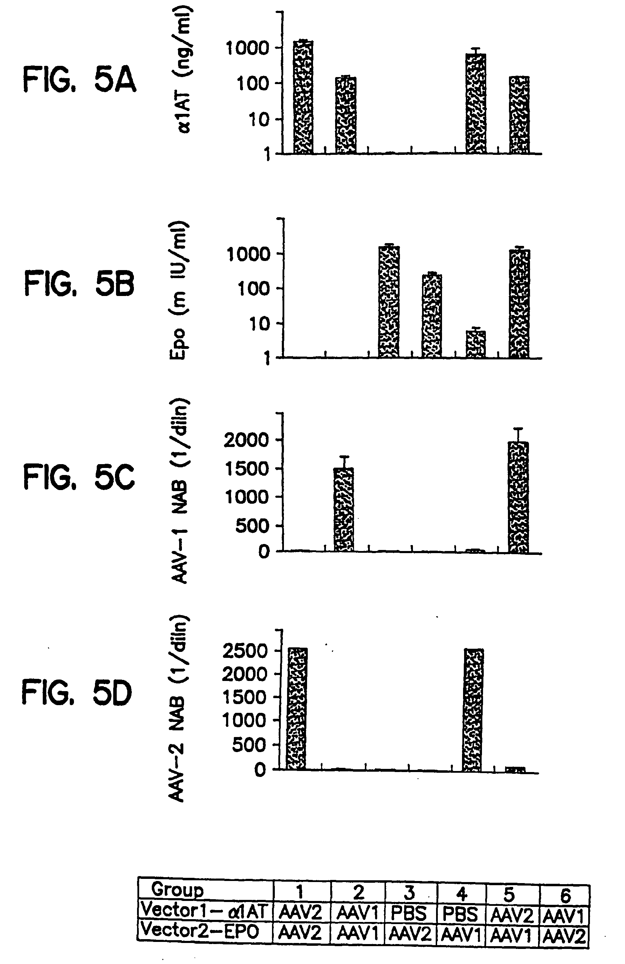

C57BL/6 mice (6- to 8-week old males, Jackson Laboratories) were analyzed for AAV mediated gene transfer to liver following intrasplenic injection of vector (i.e., targeted to liver). A total of 10¹¹ genome equivalents of rAAV-1 or rAAV-2 vector were injected into the circulation in 100 µl buffered saline. The first vector contained either an AAV-1 capsid or an AAV-2 capsid and expressed α1AT under the control of the chicken β-actin (CB) promoter. Day 28 sera were analyzed for antibodies against AAV-1 or AAV-2 and serum α1AT levels were checked. Animals were then injected with an AAV-1 or AAV-2 construct expressing erythropoietin (Epo, also under the control of the CB promoter). One month later sera was analyzed for serum levels of Epo. The following groups were analyzed (Figs. 5A-5D).

In Group 1, vector 1 was AAV-2 expressing α1AT and vector 2 was AAV-2 expressing Epo. Animals generated antibodies against AAV-2 following the first vector administration which prevented the readministration of the AAV-2 based vector. There was no evidence for cross-neutralizing the antibody to AAV-1.

In Group 2, vector 1 was AAV-1 expressing α1AT while vector 2 was AAV-1 expressing Epo. The first vector administration did result in significant α1AT expression at one month associated with antibodies to neutralizing antibodies to AAV-1. The animals were not successfully readministered with the AAV-1 Epo expressing construct.

In Group 3, the effectiveness of an AAV-2 vector expressing Epo injected into a naive animal was measured. The animals were injected with PBS and injected with AAV-2 Epo vector at day 28 and analyzed for Epo expression one month later. The neutralizing antibodies were evaluated at day 28 so we did not expect to see anything since they received PBS with the first vector injection. This shows that in naive animals AAV-2 is very efficient at transferring the Epo gene as demonstrated by high level of serum Epo one month later.

Group 4 was an experiment similar to Group 3 in which the animals originally received PBS for vector 1 and then the AAV-1 expressing Epo construct 28 days later. At the time of vector injection, there obviously were no antibodies to either AAV-1 or AAV-2. The AAV-1 based vector was capable of generating significant expression of Epo when measured one month later.

Group 5 is a cross-over experiment where the initial vector is AAV-2 expressing α1AT followed by the AAV-1 construct expressing Epo. The animals, as expected, were efficiently infected with the AAV-2 vector expressing α1AT as shown by high levels of the protein in blood at 28 days. This was associated with significant neutralizing antibodies to AAV-2. Importantly, the animals were successfully administered AAV-1 following the AAV-2 vector as shown by the presence of Epo in serum 28 days following the second vector administration. At the time of this vector administration, there was high level AAV-2 neutralizing antibodies and very low cross-reaction to AAV-1. The level ofEpo was slightly diminished possibly due to a small amount of cross-reactivity. Group 6 was the opposite cross-over experiment in which the initial vector was AAV-1 based, whereas the second experiment was AAV-2 based. The AAV-1 vector did lead to significant gene expression of α1AT, which also resulted in high level AAV-1 neutralizing antibody. The animals were very efficiently administered AAV-2 following the initial AAV-1 vector as evidenced by high level Epo.

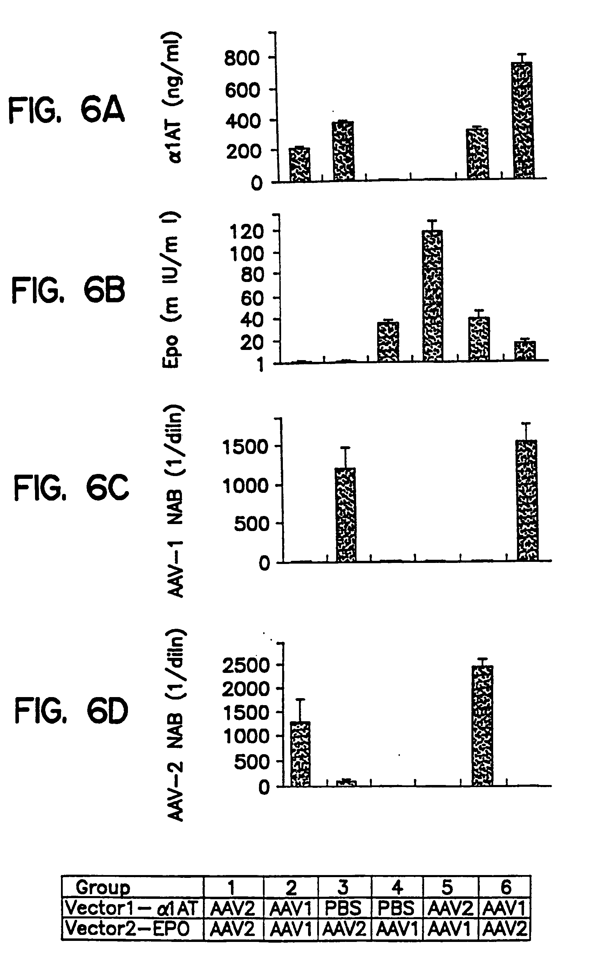

A substantially identical experiment was performed in muscle in which 5 x 10¹⁰ genomes were injected into the tibialis anterior of C57BL/6 mice as a model for muscle directed gene therapy. The results are illustrated in Figs. 6A-6D and are essentially the same as for liver.

In summary, this experiment demonstrates the utility of using an AAV-1 vector in patients who have pre-existing antibodies to AAV-2 or who had initially received an AAV-2 vector and need readministration.

### Example 8 - Construction of Recombinant Viruses Containing AAV-1 ITRs

This example illustrates the construction of recombinant AAV vectors which contain AAV-1 ITRs of the invention.

An AAV-1 cis plasmid is constructed as follows. A 160 bp Xho-NruI AAV-1 fragment containing the AAV-1 5' ITR is obtained from pAV1-BL. pAV1-BL was generated as described in Example 1. The Xho-NruI fragment is then cloned into a second pAV1-BL plasmid at an XbaI site to provide the plasmid with two AAV-1 ITRs. The desired transgene is then cloned into the modified pAV-1BL at the NruI and BamHI site, which is located between the AAV-1 ITR sequences. The resulting AAV-1 cis plasmid contains AAV-1 ITRs flanking the transgene and lacks functional AAV-1 rep and cap.

Recombinant AAV is produced by simultaneously transfecting three plasmids into 293 cells. These include the AAV-1 cis plasmid described above; a trans plasmid which provides AAV rep/cap functions and lacks AAV ITRs; and a plasmid providing adenovirus helper functions. The rep and/or cap functions may be provided in trans by AAV-1 or another AAV serotype, depending on the immunity profile of the intended recipient. Alternatively, the rep or cap functions may be provided in cis by AAV-1 or another serotype, again depending on the patient's immunity profile.

In a typical cotransfection, 50 µg of DNA (cis:trans:helper at ratios of 1:1:2, respectively) is transfected onto a 15 cm tissue culture dish. Cells are harvested 96 hours post transfection, sonicated and treated with 0.5% sodium deoxycholate (37° for 10 min). Cell lysates are then subjected to 2-3 rounds of ultracentrifugation in a cesium gradient. Peak fractions containing rAAV are collected, pooled and dialyzed against PBS. A typical yield is 1 x 10³ genomes/10⁹ cells.

Using this method, one recombinant virus construct is prepared which contains the AAV-1 ITRs flanking the transgene, with an AAV-1 capsid. Another recombinant virus construct is prepared with contains the AAV-1 ITRs flanking the transgene, with an AAV-2 capsid.

## Claims

1. A recombinant virus having an AAV-1 capsid comprising an AAV-1 protein selected from among AAV-1 vp1 having the amino acid sequence of SEQ ID NO:13; AAV-1 vp2 having the amino acid sequence of SEQ ID NO:15, AAV-1 vp3 having the amino acid sequence of SEQ ID NO: 17, and a heterologous molecule comprising an AAV 5' inverted terminal repeat sequence (ITR), a transgene, and an AAV3'ITR.

2. The recombinant virus according to claim 1, wherein the AAV-1 protein vp1 is encoded by a nucleic acid having at least 99% identity to nucleotides 2223 to 4431 of SEQ ID NO:1.

3. The recombinant virus according to claim 1 or claim 2, wherein the AAV-1 protein vp2 is encoded by a nucleic acid having at least 99% identity to nucleotides 2634 to 4431 of SEQ ID NO:1.

4. The recombinant virus according to any of claims 1 to 3, wherein the AAV-1 protein vp3 is encoded by a nucleic acid having at least 99% identity to nucleotides 2829 to 4431 of SEQ ID NO:1.

5. The recombinant virus according to any of claims 1 to 4 wherein the AAV 5' ITR and 3' ITR are of AAV serotype 2.

6. The recombinant virus according to any of claims 1 to 4 further **characterized by** a regulatable promoter which directs expression of the transgene.

7. The recombinant virus according to any of claims 1 to 4, wherein said transgene encoded is alpha-1-antitrypsin or erythropoietin.

8. A pharmaceutical composition comprising a recombinant virus according to any of claims 1 to 7 and a pharmaceutically acceptable carrier.

9. Use of a recombinant virus according to any of claims 1 to 7 for preparing a medicament useful for transducing a muscle cell.

10. Use of a recombinant virus according to claim 9, wherein said medicament is formulated for intramuscular administration.

11. Use of a recombinant virus according to any of claims I to 7 for preparing a medicament useful for transducing a liver cell.

12. Use of a recombinant virus according to claim 11, wherein said medicament is formulated for intravenous administration.

13. A recombinant host cell comprising the recombinant virus of any of claims 1 to 7.

14. A recombinant host cell transformed with a nucleic acid sequence expressing one or more AAV-1 rep proteins selected from among rep78 having the amino acid sequence of SEQ ID NO:5, rep 68 having the amino acid sequence of SEQ ID NO:7; rep 52 having the amino acid sequence of SEQ ID NO:9, and rep 40 having the amino acid sequence of SEQ ID NO: 11.

15. A recombinant host cell transformed with a nucleic acid sequence expressing one or more AAV-1 cap proteins selected from among vp1 having the amino acid sequence af SEQ ID NO:13, vp2 having the amino acid sequence of SEQ ID NO: 15 and vp3 having the amino acid sequence of SEQ ID NO: 17.

16. A recombinant host cell according to any of claims 13 to 15 comprising an AAV-1 inverted terminal repeat (ITR) sequence selected from the group consisting of:
(a) a 5' ITR having the nucleic acid sequence of nt 1 to 143 of SEQ ID NO: I :
(b) a 3' ITR having the nucleic acid sequence of nt 4576 to 4718 of SEQ ID NO: 1; and
(c) a nucleic acid sequence complementary to (a) or (b).

17. The recombinant host cell according to claim 16, wherein said vector further comprises a 3' AAV-1 ITR and a 5' AAV-1 ITR.

18. The recombinant host cell according to claim 16 or 17, wherein said vector further comprises adenovirus sequences.

19. A vector encoding AAV-1 helper functions, said molecule comprising an AA V rep coding region and an AAV cap coding region, wherein said cap coding region comprises at least one member selected from the group consisting of:
(a) vp I having the nucleic acid sequence of nt 2223 to 4431 of SEQ ID NO:
(b) vp2 having the nucleic acid sequence of nt 2634 to 4432 of SEQ ID NO: 1; and
(c) vp3 having the nucleic acid sequence of nt 2829 to 4432 of SEQ ID NO: 1.

20. A vector encoding AAV-1 helper functions, said molecule comprising an AAV-1 rep coding region and an AAV cap coding region, wherein said rep coding region comprises an AAV- rep coding region comprising at least one member selected from the group consisting of:
(a) rep78 having the nucleic acid sequence of nt 335 to 2304 of SEQ ID NO: 1 ;
(b) rep68 having the nucleic acid sequence of nt 335 to 2272 of SEQ ID NO: 1 or the cDNA corresponding thereto;
(c) rep52 having the nucleic acid sequence of nt 1007 to 2304 of SEQ ID NO: 1; and
(d) rep40 having the nucleic acid sequence of nt 1007 to 2272 of SEQ ID NO: 1 or the cDNA corresponding thereto.

21. A host cell transduced with a recombinant vector according to any of claims 19 to 20.

22. A host cell according to any of claims 13 to 17 or 21 stably transduced with an AAV-1 P5 promoter having the sequence of nt 236 to 299 of SEQ ID NO: 1.

23. Use of an AAV virion which comprises:
(a) a capsid comprising at least one capsid protein encoded by an AAV-1 cap gene having at least 99% identity to nucleotides 2223 to 4431 of SEQ ID NO:1; and
(b) a DNA molecule comprising a transgene under the control of regulatory sequences directing its expression for preparing a medicament for delivery of a transgene to a host cell.

## Patentansprüche

1. Rekombinantes Virus mit einem AAV-1-Capsid umfassend ein AAV-1-Protein, ausgewählt aus AAV-1 vp1 mit der Aminosäuresequenz von SEQ ID NO: 13; AAV-1 vp2 mit der Aminosäuresequenz von SEQ ID NO: 15, AAV-1 vp3 mit der Aminosäuresequenz von SEQ ID NO: 17 und ein heterologes Molekül umfassend eine AAV-5'-invertierte terminale Wiederholungssequenz (ITR), ein Transgen und eine AAV-3'-ITR.

2. Rekombinantes Virus nach Anspruch 1, wobei das AAV-1-Protein vp1 von einer Nucleinsäure mit mindestens 99%iger Identität zu den Nucleotiden 2223 bis 4431 von SEQ ID NO: 1 codiert wird.

3. Rekombinantes Virus nach Anspruch 1 oder Anspruch 2, wobei das AAV-1-Protein vp2 von einer Nucleinsäure mit mindestens 99%iger Identität zu den Nucleotiden 2634 bis 4431 von SEQ ID NO: 1 codiert wird.

4. Rekombinantes Virus nach einem der Ansprüche 1 bis 3, wobei das AAV-1-Protein vp3 von einer Nucleinsäure mit mindestens 99%iger Identität zu den Nucleotiden 2829 bis 4431 von SEQ ID NO: 1 codiert wird.

5. Rekombinantes Virus nach einem der Ansprüche 1 bis 4, wobei die AAV-5'-ITR und -3'-ITR vom AAV-Serotyp 2 sind.

6. Rekombinantes Virus nach einem der Ansprüche 1 bis 4, das weiterhin durch einen regulierbaren Promotor **gekennzeichnet** ist, der die Expression des Transgens steuert.

7. Rekombinantes Virus nach einem der Ansprüche 1 bis 4, wobei das codierte Transgen alpha-1-Antitrypsin oder Erythropoietin ist.

8. Pharmazeutische Zusammensetzung, die ein rekombinantes Virus nach einem der Ansprüche 1 bis 4 und einen pharmazeutisch verträglichen Träger umfasst.

9. Verwendung eines rekombinanten Virus nach einem der Ansprüche 1 bis 4 zur Herstellung eines Medikaments, das zur Transduktion einer Muskelzelle geeignet ist.

10. Verwendung eines rekombinanten Virus nach Anspruch 9, wobei das Medikament zur intramuskulären Verabreichung formuliert ist.

11. Verwendung eines rekombinanten Virus nach einem der Ansprüche 1 bis 7 zur Herstellung eines Medikaments, das zur Transduktion einer Leberzelle geeignet ist.

12. Verwendung eines rekombinanten Virus nach Anspruch 11, wobei das Medikament zur intravenösen Verabreichung formuliert ist.

13. Rekombinante Wirtszelle, die das rekombinante Virus nach einem der Ansprüche 1 bis 7 umfasst.

14. Rekombinante Wirtszelle, die mit einer Nucleinsäuresequenz transformiert ist, die ein oder mehrere AAV-1-rep-Proteine exprimiert, ausgewählt aus rep78 mit der Aminosäuresequenz von SEQ ID NO:5, rep68 mit der Aminosäuresequenz von SEQ ID NO:7, rep52 mit der Aminosäuresequenz von SEQ ID NO:9 und rep40 mit der Aminosäuresequenz von SEQ ID NO: 11.

15. Rekombinante Wirtszelle, die mit einer Nucleinsäuresequenz transformiert ist, die ein oder mehrere AAV-1-cap-Proteine exprimiert, ausgewählt aus vp1 mit der Aminosäuresequenz von SEQ ID NO:13, vp2 mit der Aminosäuresequenz von SEQ ID NO:15 und vp3 mit der Aminosäuresequenz von SEQ ID NO:17.

16. Rekombinante Wirtszelle nach einem der Ansprüche 13 bis 15, umfassend eine AAV-1-invertierte terminale Wiederholungssequenz (ITR), ausgewählt aus der Gruppe, bestehend aus:
(a) einer 5'-ITR mit der Nucleinsäuresequenz von nt 1 bis 143 von SEQ ID NO:1;
(b) einer 3'-ITR mit der Nucleinsäuresequenz von nt 4576 bis 4718 von SEQ ID NO:1; und
(c) einer zu (a) oder (b) komplementären Nucleinsäuresequenz.

17. Rekombinante Wirtszelle nach Anspruch 16, wobei der Vektor weiterhin eine 3'-AAV-1-ITR und eine 5'-AAV-1-ITR umfasst.

18. Rekombinante Wirtszelle nach Anspruch 16 oder 17, wobei der Vektor außerdem Adenovirussequenzen umfasst.

19. Vektor, der AAV-1-Helferfunktionen codiert, wobei das Molekül eine AAV-repcodierende Region und eine AAV-cap-codierende Region einschließt, wobei die cap-codierende Region mindestens ein Element umfasst, ausgewählt aus der Gruppe, bestehend aus:
(a) vp 1 mit der Nucleinsäuresequenz von nt 2223 bis 4431 von SEQ .ID NO:1;
(b) vp2 mit der Nucleinsäuresequenz von nt 2634 bis 4432 von SEQ ID NO:1; und
(c) vp3 mit der Nucleinsäuresequenz von nt 2829 bis 4432 von SEQ ID NO:1.

20. Vektor, der AAV-1-Helferfunktionen codiert, wobei das Molekül eine AAV-1-rep-codierende Region und eine AAV-cap-codierende Region umfasst, wobei die repcodierende Region eine AAV-1-rep-codierende Region umfasst, die mindestens ein Element umfasst, ausgewählt aus der Gruppe bestehend aus:
(a) rep78 mit der Nucleinsäuresequenz von nt 335 bis 2304 von SEQ ID NO: 1;
(b) rep68 mit der Nucleinsäuresequenz von nt335 bis 2272 von SEQ ID NO:1 oder der dazu entsprechenden cDNA;
(c) rep52 mit der Nucleinsäuresequenz von nt1007 bis 2304 von SEQ ID NO:1; und
(d) rep40 mit der Nucleinsäursequenz von nt1007 bis 2272 von SEQ ID NO:1 oder der dazu entsprechenden cDNA.

21. Wirtszelle, die mit einem rekombinanten Vektor nach einem der Ansprüche 19 bis 20 transduziert wurde.

22. Wirtszelle nach einem der Ansprüche 13 bis 17 oder 21, die stabil mit einem AAV-1-P5-Promotor mit der Sequenz von nt 236 bis 299 von SEQ ID NO:1 transduziert wurde.

23. Verwendung eines AAV-Virions, welches umfasst:
(a) ein Capsid, das mindestens ein Capsidprotein umfasst, das von einem AAV-1-cap-Gen mit mindestens 99%iger Identität zu den Nucleotiden 2223 bis 4431 von SEQ ID NO: 1 codiert wird; und
(b) ein DNA-Molekül, das ein Transgen unter der Kontrolle von regulatorischen Sequenzen, die seine Expression steuern, umfasst,
zur Herstellung eines Medikaments zur Abgabe eines Transgens an eine Wirtszelle.

## Revendications

1. Virus recombinant ayant une capside de l'AAV-1 comprenant une protéine de l'AAV-1 choisie parmi vp1 de l'AAV-1 ayant la séquence d'acides aminés de la SEQ ID NO :13 ; vp2 de l'AAV-1 ayant la séquence d'acides aminés de la SEQ ID NO:15, vp3 de l'AAV-1 ayant la séquence d'acides aminés de la SEQ ID NO:17, et une molécule hétérologue comprenant une séquence de répétition terminale inversée (ITR) 5' de l'AAV, un transgène et une ITR 3' de l'AAV.

2. Virus recombinant selon la revendication 1, où la protéine vp1 de l'AAV-1 est codée par un acide nucléique ayant une identité d'au moins 99 % aux nucléotides 2223 à 4431 de la SEQ ID NO:1.

3. Virus recombinant selon la revendication 1 ou la revendication 2, où la protéine vp2 de l'AAV-1 est codée par un acide nucléique ayant une identité d'au moins 99 % aux nucléotides 2634 à 4431 de la SEQ ID NO:1.

4. Virus recombinant selon l'une quelconque des revendications 1 à 3, où la protéine vp3 de l'AAV-1 est codée par un acide nucléique ayant une identité d'au moins 99 % aux nucléotides 2829 à 4431 de la SEQ ID NO:1.

5. Virus recombinant selon l'une quelconque des revendications 1 à 4, où l'ITR 5' de l'AAV et l'ITR 3' sont de l'AAV de sérotype 2.

6. Virus recombinant selon l'une quelconque des revendications 1 à 4 **caractérisé de plus par** un promoteur régulable qui dirige l'expression du transgène.

7. Virus recombinant selon l'une quelconque des revendications 1 à 4, où le transgène codé est l'alpha-1-antitrypsine ou l'érythropoiétine.

8. Composition pharmaceutique comprenant un virus recombinant selon l'une quelconque des revendications 1 à 7 et un véhicule pharmaceutiquement acceptable.

9. Utilisation d'un virus recombinant selon l'une quelconque des revendications 1 à 7, pour la préparation d'un médicament utile pour la transduction d'une cellule de muscle.

10. Utilisation d'un virus recombinant selon la revendication 9, où ledit médicament est formulé pour l'administration intramusculaire.

11. Utilisation d'un virus recombinant selon l'une quelconque des revendications 1 à 7, pour la préparation d'un médicament utile pour la transduction d'une cellule de foie.

12. Utilisation d'un virus recombinant selon la revendication 11, où ledit médicament est formulé pour l'administration intraveineuse.

13. Cellule hôte recombinante comprenant un virus recombinant de l'une quelconque des revendications 1 à 7.

14. Cellule hôte recombinante transformée avec une séquence d'acide nucléique exprimant une ou plusieurs protéines rep de l'AAV-1 choisies parmi rep 78 ayant la séquence d'acides aminés de la SEQ ID NO:5, rep 68 ayant la séquence d'acides aminés de la SEQ ID NO:7, rep 52 ayant la séquence d'acides aminés de la SEQ ID NO:9 et rep 40 ayant la séquence d'acides aminés de la SEQ ID NO:11.

15. Cellule hôte recombinante transformée avec une séquence d'acide nucléique exprimant une ou plusieurs protéines cap de l'AAV-1 choisies parmi vp1 ayant la séquence d'acides aminés de la SEQ ID NO:13, vp2 ayant la séquence d'acides aminés de la SEQ ID NO:15 et vp3 ayant la séquence d'acides aminés de la SEQ ID NO:17.

16. Cellule hôte recombinante selon l'une quelconque des revendications 13 à 15 comprenant une séquence de répétition terminale inversée (ITR) de l'AAV-1 choisie dans le groupe constitué par:
(a) une ITR 5' ayant la séquence d'acide nucléique de nt 1 à 143 de la SEQ ID NO:1 ;
(b) une ITR 3' ayant la séquence d'acide nucléique de nt 4576 à 4718 de la SEQ ID NO:1 ; et
(c) une séquence d'acide nucléique complémentaire de (a) ou (b).

17. Cellule hôte recombinante selon la revendication 16, où ledit vecteur comprend de plus une ITR 3' de l'AAV-1 et une ITR 5' de l'AAV-1.

18. Cellule hôte recombinante selon la revendication 16 ou 17, où ledit vecteur comprend de plus des séquences adénovirales.

19. Vecteur codant les fonctions auxiliaires d'AAV-1, ladite molécule comprenant une région codante rep de l'AAV et une région codante cap de l'AAV, où ladite région cap comprend au moins un membre choisi dans le groupe constitué par:
(a) vp1 ayant la séquence d'acide nucléique de nt 2223 à 4431 de la SEQ ID NO:1;
(b) vp2 ayant la séquence d'acide nucléique de nt 2634 à 4432 de la SEQ ID NO:1; et
(c) vp3 ayant la séquence d'acide nucléique de nt 2829 à 4432 de la SEQ ID NO: 1.

20. Vecteur codant les fonctions auxiliaires de l'AAV-1, ladite molécule comprenant une région codante rep de l'AAV-1 et une région codante cap de l'AAV, où ladite région codante rep comprend une région codante de l'AAV-1 comprenant au moins un membre choisi dans le groupe constitué par:
(a) rep78 ayant la séquence d'acide nucléique de nt 335 à 2304 de la SEQ ID NO:1;
(b) rep68 ayant la séquence d'acide nucléique de nt 335 à 2272 de la SEQ ID NO:1 ou l'ADNc correspondant à celle-ci ;
(c) rep52 ayant la séquence d'acide nucléique de nt 1007 à 2304 de la SEQ ID NO:1; et
(d) rep40 ayant la séquence d'acide nucléique de nt 1007 à 2272 de la SEQ ID NO:1 ou l'ADNc correspondant à celle-ci.

21. Cellule hôte transduite avec un vecteur recombinant selon l'une quelconque des revendications 19 à 20.

22. Cellule hôte selon l'une quelconque des revendications 13 à 17 ou 21 transduite de manière stable avec un promoteur P5 de l'AAV-1 ayant la séquence de nt 236 à 299 de la SEQ ID NO:1.

23. Utilisation d'un virion d'AAV qui comprend :
(a) une capside comprenant au moins une protéine de capside codée par un gène cap de l'AAV-1 ayant une identité d'au moins 99 % aux nucléotides 2223 à 4431 de la SEQ ID NO:1, et
(b) une molécule d'ADN comprenant un transgène sous le contrôle des séquences régulatrices dirigeant son expression,
pour la préparation d'un médicament pour la délivrance d'un transgène à une cellule hôte.


### SEQUENCE LISTING

<110> Wilson, James M.
   Xiao, Weidong
   The Trustees of the University of Pennsylvania
<120> Adeno-Associated Virus Serotype I Nucleic Acid
   Sequences, Vectors and Host Cells Containing Same
<130> GNVPN.031PCT
<140>
   <141>
<150> 60/107,114
   <151> 1998-11-05
<160> 20
<170> PatentIn Ver. 2.0
<210> 1
   <211> 4718
   <212> DNA
   <213> AAV-1
<220>
   <221> CDS
   <222> (335)..(2206)
<220>
   <221> CDS
   <222> (2223)..(4430)
<400> 1
   ttgcccactc cctctctgcg cgctcgctcg ctcggtgggg cctgcggacc aaaggtccgc 60
   agacggcaga gctctgctct gccggcccca ccgagcgagc gagcgcgcag agagggagtg 120
   ggcaactcca tcactagggg taatcgcgaa gcgcctccca cgctgccgcg tcagcgctga 180
   cgtaaattac gtcatagggg agtggtcctg tattagctgt cacgtgagtg cttttgcgac 240
   attttgcgac accacgtggc catttagggt atatatggcc gagtgagcga gcaggatctc 300
   cattttgacc gcgaaatttg aacgagcagc agcc atg ccg ggc ttc tac gag atc 355
                                         Met Pro Gly Phe Tyr Glu Ile
                                          1               5
   gtg atc aag gtg ccg agc gac ctg gac gag cac ctg ccg ggc att tct      403
   Val Ile Lys Val Pro Ser Asp Leu Asp Glu His Leu Pro Gly Ile Ser
           10                  15                  20
   gac tcg ttt gtg agc tgg gtg gcc gag aag gaa tgg gag ctg ccc ccg      451
   Asp Ser Phe Val Ser Trp Val Ala Glu Lys Glu Trp Glu Leu Pro Pro
       25                  30                  35
   gat tct gac atg gat ctg aat ctg att gag cag gca ccc ctg acc gtg      499
   Asp Ser Asp Met Asp Leu Asn Leu Ile Glu Gln Ala Pro Leu Thr Val
   40                  45                  50                  55
   gcc gag aag ctg cag cgc gac ttc ctg gtc caa tgg cgc cgc gtg agt      547
   Ala Glu Lys Leu Gln Arg Asp Phe Leu Val Gln Trp Arg Arg Val Ser
                   60                  65                  70
   aag gcc ccg gag gcc ctc ttc ttt gtt cag ttc gag aag ggc gag tcc      595
   Lys Ala Pro Glu Ala Leu Phe Phe Val Gln Phe Glu Lys Gly Glu Ser
               75                  80                  85
   tac ttc cac ctc cat att ctg gtg gag acc acg ggg gtc aaa tcc atg      643
   Tyr Phe His Leu His Ile Leu Val Glu Thr Thr Gly Val Lys Ser Met
           90                  95                  100
   gtg ctg ggc cgc ttc ctg agt cag att agg gac aag ctg gtg cag acc      691
   Val Leu Gly Arg Phe Leu Ser Gln Ile Arg Asp Lys Leu Val Gln Thr
       105                 110                 115
   atc tac cgc ggg atc gag ccg acc ctg ccc aac tgg ttc gcg gtg acc      739
   Ile Tyr Arg Gly Ile Glu Pro Thr Leu Pro Asn Trp Phe Ala Val Thr
   120                 125                 130                 135
   aag acg cgt aat ggc gcc gga ggg ggg aac aag gtg gtg gac gag tgc      787
   Lys Thr Arg Asn Gly Ala Gly Gly Gly Asn Lys Val Val Asp Glu Cys
                   140                 145                 150
   tac atc ccc aac tac ctc ctg ccc aag act cag ccc gag ctg cag tgg      835
   Tyr Ile Pro Asn Tyr Leu Leu Pro Lys Thr Gln Pro Glu Leu Gln Trp
               155                 160                 165
   gcg tgg act aac atg gag gag tat ata agc gcc tgt ttg aac ctg gcc      883
   Ala Trp Thr Asn Met Glu Glu Tyr Ile Ser Ala Cys Leu Asn Leu Ala
           170                 175                 180
   gag cgc aaa cgg ctc gtg gcg cag cac ctg acc cac gtc agc cag acc      931
   Glu Arg Lys Arg Leu Val Ala Gln His Leu Thr His Val Ser Gln Thr
       185                 190                 195
   cag gag cag aac aag gag aat ctg aac ccc aat tct gac gcg cct gtc    979
   Gln Glu Gln Asn Lys Glu Asn Leu Asn Pro Asn Ser Asp Ala Pro Val
   200                 205                 210                 215
   atc cgg tca aaa acc tcc gcg cgc tac atg gag ctg gtc ggg tgg ctg   1027
   Ile Arg Ser Lys Thr Ser Ala Arg Tyr Met Glu Leu Val Gly Trp Leu
                   220                 225                 230
   gtg gac cgg ggc atc acc tcc gag aag cag tgg atc cag gag gac cag   1075
   Val Asp Arg Gly Ile Thr Ser Glu Lys Gln Trp Ile Gln Glu Asp Gln
               235                 240                 245
   gcc tcg tac atc tcc ttc aac gcc gct tcc aac tcg cgg tcc cag atc   1123
   Ala Ser Tyr Ile Ser Phe Asn Ala Ala Ser Asn Ser Arg Ser Gln Ile
           250                 255                 260
   aag gcc gct ctg gac aat gcc ggc aag atc atg gcg ctg acc aaa tcc   1171
   Lys Ala Ala Leu Asp Asn Ala Gly Lys Ile Met Ala Leu Thr Lys Ser
       265                 270                 275
   gcg ccc gac tac ctg gta ggc ccc gct ccg ccc gcg gac att aaa acc   1219
   Ala Pro Asp Tyr Leu Val Gly Pro Ala Pro Pro Ala Asp Ile Lys Thr
   280                 285                 290                 295
   aac cgc atc tac cgc atc ctg gag ctg aac ggc tac gaa cct gcc tac   1267
   Asn Arg Ile Tyr Arg Ile Leu Glu Leu Asn Gly Tyr Glu Pro Ala Tyr
                   300                 305                 310
   gcc ggc tcc gtc ttt ctc ggc tgg gcc cag aaa agg ttc ggg aag cgc   1315
   Ala Gly Ser Val Phe Leu Gly Trp Ala Gln Lys Arg Phe Gly Lys Arg
               315                 320                 325
   aac acc atc tgg ctg ttt ggg ccg gcc acc acg ggc aag acc aac atc   1363
   Asn Thr Ile Trp Leu Phe Gly Pro Ala Thr Thr Gly Lys Thr Asn Ile
           330                 335                 340
   gcg gaa gcc atc gcc cac gcc gtg ccc ttc tac ggc tgc gtc aac tgg   1411
   Ala Glu Ala Ile Ala His Ala Val Pro Phe Tyr Gly Cys Val Asn Trp
       345                 350                 355
   acc aat gag aac ttt ccc ttc aat gat tgc gtc gac aag atg gtg atc   1459
   Thr Asn Glu Asn Phe Pro Phe Asn Asp Cys Val Asp Lys Met Val Ile
   360                 365                 370                 375
   tgg tgg gag gag ggc aag atg acg gcc aag gtc gtg gag tcc gcc aag   1507
   Trp Trp Glu Glu Gly Lys Met Thr Ala Lys Val Val Glu Ser Ala Lys
                   380                 385                 390
   gcc att ctc ggc ggc agc aag gtg cgc gtg gac caa aag tgc aag tcg    1555
   Ala Ile Leu Gly Gly Ser Lys Val Arg Val Asp Gln Lys Cys Lys Ser
               395                 400                 405
   tcc gcc cag atc gac ccc acc ccc gtg atc gtc acc tcc aac acc aac    1603
   Ser Ala Gln Ile Asp Pro Thr Pro Val Ile Val Thr Ser Asn Thr Asn
           410                 415                 420
   atg tgc gcc gtg att gac ggg aac agc acc acc ttc gag cac cag cag    1651
   Met Cys Ala Val Ile Asp Gly Asn Ser Thr Thr Phe Glu His Gln Gln
       425                 430                 435
   ccg ttg cag gac cgg atg ttc aaa ttt gaa ctc acc cgc cgt ctg gag    1699
   Pro Leu Gln Asp Arg Met Phe Lys Phe Glu Leu Thr Arg Arg Leu Glu
   440                 445                 450                 455
   cat gac ttt ggc aag gtg aca aag cag gaa gtc aaa gag ttc ttc cgc    1747
   His Asp Phe Gly Lys Val Thr Lys Gln Glu Val Lys Glu Phe Phe Arg
                   460                 465                 470
   tgg gcg cag gat cac gtg acc gag gtg gcg cat gag ttc tac gtc aga    1795
   Trp Ala Gln Asp His Val Thr Glu Val Ala His Glu Phe Tyr Val Arg
               475                 480                 485
   aag ggt gga gcc aac aaa aga ccc gcc ccc gat gac gcg gat aaa agc    1843
   Lys Gly Gly Ala Asn Lys Arg Pro Ala Pro Asp Asp Ala Asp Lys Ser
           490                 495                 500
   gag ccc aag cgg gcc tgc ccc tca gtc gcg gat cca tcg acg tca gac    1891
   Glu Pro Lys Arg Ala Cys Pro Ser Val Ala Asp Pro Ser Thr Ser Asp
       505                 510                 515
   gcg gaa gga gct ccg gtg gac ttt gcc gac agg tac caa aac aaa tgt    1939
   Ala Glu Gly Ala Pro Val Asp Phe Ala Asp Arg Tyr Gln Asn Lys Cys
   520                 525                 530                 535
   tct cgt cac gcg ggc atg ctt cag atg ctg ttt ccc tgc aag aca tgc    1987
   Ser Arg His Ala Gly Met Leu Gln Met Leu Phe Pro Cys Lys Thr Cys
                   540                 545                 550
   gag aga atg aat cag aat ttc aac att tgc ttc acg cac ggg acg aga    2035
   Glu Arg Met Asn Gln Asn Phe Asn Ile Cys Phe Thr His Gly Thr Arg
               555                 560                 565
   gac tgt tca gag tgc ttc ccc ggc gtg tca gaa tct caa ccg gtc gtc    2083
   Asp Cys Ser Glu Cys Phe Pro Gly Val Ser Glu Ser Gln Pro Val Val
           570                 575                 580
   aga aag agg acg tat cgg aaa ctc tgt gcc att cat cat ctg ctg ggg    2131
   Arg Lys Arg Thr Tyr Arg Lys Leu Cys Ala Ile His His Leu Leu Gly
       585                 590                 595
   cgg gct ccc gag att gct tgc tcg gcc tgc gat ctg gtc aac gtg gac    2179
   Arg Ala Pro Glu Ile Ala Cys Ser Ala Cys Asp Leu Val Asn Val Asp
   600                 605                 610                 615
   ctg gat gac tgt gtt tct gag caa taa atgacttaaa ccaggt atg gct gcc 2231
   Leu Asp Asp Cys Val Ser Glu Gln                       Met Ala Ala
                   620                                   625
   gat ggt tat ctt cca gat tgg ctc gag gac aac ctc tct gag ggc att    2279
   Asp Gly Tyr Leu Pro Asp Trp Leu Glu Asp Asn Leu Ser Glu Gly Ile
           630                 635                 640
   cgc gag tgg tgg gac ttg aaa cct gga gcc ccg aag ccc aaa gcc aac    2327
   Arg Glu Trp Trp Asp Leu Lys Pro Gly Ala Pro Lys Pro Lys Ala Asn
       645                 650                 655
   cag caa aag cag gac gac ggc cgg ggt ctg gtg ctt cct ggc tac aag    2375
   Gln Gln Lys Gln Asp Asp Gly Arg Gly Leu Val Leu Pro Gly Tyr Lys
   660                 665                 670                 675
   tac ctc gga ccc ttc aac gga ctc gac aag ggg gag ccc gtc aac gcg    2423
   Tyr Leu Gly Pro Phe Asn Gly Leu Asp Lys Gly Glu Pro Val Asn Ala
                   680                 685                 690
   gcg gac gca gcg gcc ctc gag cac gac aag gcc tac gac cag cag ctc    2471
   Ala Asp Ala Ala Ala Leu Glu His Asp Lys Ala Tyr Asp Gln Gln Leu
               695                 700                 705
   aaa gcg ggt gac aat ccg tac ctg cgg tat aac cac gcc gac gcc gag    2519
   Lys Ala Gly Asp Asn Pro Tyr Leu Arg Tyr Asn His Ala Asp Ala Glu
           710                 715                 720
   ttt cag gag cgt ctg caa gaa gat acg tct ttt ggg ggc aac ctc ggg    2567
   Phe Gln Glu Arg Leu Gln Glu Asp Thr Ser Phe Gly Gly Asn Leu Gly
       725                 730                 735
   cga gca gtc ttc cag gcc aag aag cgg gtt ctc gaa cct ctc ggt ctg    2615
   Arg Ala Val Phe Gln Ala Lys Lys Arg Val Leu Glu Pro Leu Gly Leu
   740                 745                 750                 755
   gtt gag gaa ggc gct aag acg gct cct gga aag aaa cgt ccg gta gag    2663
   Val Glu Glu Gly Ala Lys Thr Ala Pro Gly Lys Lys Arg Pro Val Glu
                   760                 765                 770
   cag tcg cca caa gag cca gac tcc tcc tcg ggc atc ggc aag aca ggc    2711
   Gln Ser Pro Gln Glu Pro Asp Ser Ser Ser Gly Ile Gly Lys Thr Gly
               775                 780                 785
   cag cag ccc gct aaa aag aga ctc aat ttt ggt cag act ggc gac tca    2759
   Gln Gln Pro Ala Lys Lys Arg Leu Asn Phe Gly Gln Thr Gly Asp Ser
           790                 795                 800
   gag tca gtc ccc gat cca caa cct ctc gga gaa cct cca gca acc ccc    2807
   Glu Ser Val Pro Asp Pro Gln Pro Leu Gly Glu Pro Pro Ala Thr Pro
       805                 810                 815
   gct gct gtg gga cct act aca atg gct tca ggc ggt ggc gca cca atg    2855
   Ala Ala Val Gly Pro Thr Thr Met Ala Ser Gly Gly Gly Ala Pro Met
   820                 825                 830                 835
   gca gac aat aac gaa ggc gcc gac gga gtg ggt aat gcc tca gga aat    2903
   Ala Asp Asn Asn Glu Gly Ala Asp Gly Val Gly Asn Ala Ser Gly Asn
                   840                 845                 850
   tgg cat tgc gat tcc aca tgg ctg ggc gac aga gtc atc acc acc agc    2951
   Trp His Cys Asp Ser Thr Trp Leu Gly Asp Arg Val Ile Thr Thr Ser
               855                 860                 865
   acc cgc acc tgg gcc ttg ccc acc tac aat aac cac ctc tac aag caa    2999
   Thr Arg Thr Trp Ala Leu Pro Thr Tyr Asn Asn His Leu Tyr Lys Gln
           870                 875                 880
   atc tcc agt gct tca acg ggg gcc agc aac gac aac cac tac ttc ggc    3047
   Ile Ser Ser Ala Ser Thr Gly Ala Ser Asn Asp Asn His Tyr Phe Gly
       885                 890                 895
   tac agc acc ccc tgg ggg tat ttt gat ttc aac aga ttc cac tgc cac    3095
   Tyr Ser Thr Pro Trp Gly Tyr Phe Asp Phe Asn Arg Phe His Cys His
   900                 905                 910                 915
   ttt tca cca cgt gac tgg cag cga ctc atc aac aac aat tgg gga ttc    3143
   Phe Ser Pro Arg Asp Trp Gln Arg Leu Ile Asn Asn Asn Trp Gly Phe
                   920                 925                 930
   cgg ccc aag aga ctc aac ttc aaa ctc ttc aac atc caa gtc aag gag    3191
   Arg Pro Lys Arg Leu Asn Phe Lys Leu Phe Asn Ile Gln Val Lys Glu
               935                 940                 945
   gtc acg acg aat gat ggc gtc aca acc atc gct aat aac ctt acc agc    3239
   Val Thr Thr Asn Asp Gly Val Thr Thr Ile Ala Asn Asn Leu Thr Ser
           950                 955                 960
   acg gtt caa gtc ttc tcg gac tcg gag tac cag ctt ccg tac gtc ctc     3287
   Thr Val Gln Val Phe Ser Asp Ser Glu Tyr Gln Leu Pro Tyr Val Leu
       965                 970                 975
   ggc tct gcg cac cag ggc tgc ctc cct ccg ttc ccg gcg gac gtg ttc     3335
   Gly Ser Ala His Gln Gly Cys Leu Pro Pro Phe Pro Ala Asp Val Phe
   980                 985                 990                 995
   atg att ccg caa tac ggc tac ctg acg ctc aac aat ggc agc caa gcc     3383
   Met Ile Pro Gln Tyr Gly Tyr Leu Thr Leu Asn Asn Gly Ser Gln Ala
                   1000                1005                1010
   gtg gga cgt tca tcc ttt tac tgc ctg gaa tat ttc cct tct cag atg     3431
   Val Gly Arg Ser Ser Phe Tyr Cys Leu Glu Tyr Phe Pro Ser Gln Met
               1015                1020                1025
   ctg aga acg ggc aac aac ttt acc ttc agc tac acc ttt gag gaa gtg     3479
   Leu Arg Thr Gly Asn Asn Phe Thr Phe Ser Tyr Thr Phe Glu Glu Val
           1030                1035                1040
   cct ttc cac agc agc tac gcg cac agc cag agc ctg gac cgg ctg atg     3527
   Pro Phe His Ser Ser Tyr Ala His Ser Gln Ser Leu Asp Arg Leu Met
       1045                1050                1055
   aat cct ctc atc gac caa tac ctg tat tac ctg aac aga act caa aat     3575
   Asn Pro Leu Ile Asp Gln Tyr Leu Tyr Tyr Leu Asn Arg Thr Gln Asn
   1060                1065                1070                1075
   cag tcc gga agt gcc caa aac aag gac ttg ctg ttt agc cgt ggg tct     3623
   Gln Ser Gly Ser Ala Gln Asn Lys Asp Leu Leu Phe Ser Arg Gly Ser
                   1080                1085                1090
   cca gct ggc atg tct gtt cag ccc aaa aac tgg cta cct gga ccc tgt     3671
   Pro Ala Gly Met Ser Val Gln Pro Lys Asn Trp Leu Pro Gly Pro Cys
               1095                1100                1105
   tat cgg cag cag cgc gtt tct aaa aca aaa aca gac aac aac aac agc     3719
   Tyr Arg Gln Gln Arg Val Ser Lys Thr Lys Thr Asp Asn Asn Asn Ser
           1110                1115                1120
   aat ttt acc tgg act ggt gct tca aaa tat aac ctc aat ggg cgt gaa     3767
   Asn Phe Thr Trp Thr Gly Ala Ser Lys Tyr Asn Leu Asn Gly Arg Glu
       1125                1130                1135
   tcc atc atc aac cct ggc act gct atg gcc tca cac aaa gac gac gaa     3815
   Ser Ile Ile Asn Pro Gly Thr Ala Met Ala Ser His Lys Asp Asp Glu
   1140                1145                1150                1155
   gac aag ttc ttt ccc atg agc ggt gtc atg att ttt gga aaa gag agc     3863
   Asp Lys Phe Phe Pro Met Ser Gly Val Met Ile Phe Gly Lys Glu Ser
                   1160                1165                1170
   gcc gga gct tca aac act gca ttg gac aat gtc atg att aca gac gaa     3911
   Ala Gly Ala Ser Asn Thr Ala Leu Asp Asn Val Met Ile Thr Asp Glu
               1175                1180                1185
   gag gaa att aaa gcc act aac cct gtg gcc acc gaa aga ttt ggg acc     3959
   Glu Glu Ile Lys Ala Thr Asn Pro Val Ala Thr Glu Arg Phe Gly Thr
           1190                1195                1200
   gtg gca gtc aat ttc cag agc agc agc aca gac cct gcg acc gga gat     4007
   Val Ala Val Asn Phe Gln Ser Ser Ser Thr Asp Pro Ala Thr Gly Asp
       1205                1210                1215
   gtg cat gct atg gga gca tta cct ggc atg gtg tgg caa gat aga gac     4055
   Val His Ala Met Gly Ala Leu Pro Gly Met Val Trp Gln Asp Arg Asp
   1220                1225                1230                1235
   gtg tac ctg cag ggt ccc att tgg gcc aaa att cct cac aca gat gga     4103
   Val Tyr Leu Gln Gly Pro Ile Trp Ala Lys Ile Pro His Thr Asp Gly
                   1240                1245                1250
   cac ttt cac ccg tct cct ctt atg ggc ggc ttt gga ctc aag aac ccg     4151
   His Phe His Pro Ser Pro Leu Met Gly Gly Phe Gly Leu Lys Asn Pro
               1255                1260                1265
   cct cct cag atc ctc atc aaa aac acg cct gtt cct gcg aat cct ccg     4199
   Pro Pro Gln Ile Leu Ile Lys Asn Thr Pro Val Pro Ala Asn Pro Pro
           1270                1275                1280
   gcg gag ttt tca gct aca aag ttt gct tca ttc atc acc caa tac tcc     4247
   Ala Glu Phe Ser Ala Thr Lys Phe Ala Ser Phe Ile Thr Gln Tyr Ser
       1285                1290                1295
   aca gga caa gtg agt gtg gaa att gaa tgg gag ctg cag aaa gaa aac     4295
   Thr Gly Gln Val Ser Val Glu Ile Glu Trp Glu Leu Gln Lys Glu Asn
   1300                1305                1310                1315
   agc aag cgc tgg aat ccc gaa gtg cag tac aca tcc aat tat gca aaa     4343
   Ser Lys Arg Trp Asn Pro Glu Val Gln Tyr Thr Ser Asn Tyr Ala Lys
                   1320                1325                1330
   tct gcc aac gtt gat ttt act gtg gac aac aat gga ctt tat act gag     4391
   Ser Ala Asn Val Asp Phe Thr Val Asp Asn Asn Gly Leu Tyr Thr Glu
               1335                1340                1345
   cct cgc ccc att ggc acc cgt tac ctt acc cgt ccc ctg taattacgtg    4440
   Pro Arg Pro Ile Gly Thr Arg Tyr Leu Thr Arg Pro Leu
         1350                1355                1360
   ttaatcaata aaccggttga ttcgtttcag ttgaactttg gtctcctgtc cttcttatct 4500
   tatcggttac catggttata gcttacacat taactgcttg gttgcgcttc gcgataaaag 4560
   acttacgtca tcgggttacc cctagtgatg gagttgccca ctccctctct gcgcgctcgc 4620
   tcgctcggtg gggcctgcgg accaaaggtc cgcagacggc agagctctgc tctgccggcc 4680
   ccaccgagcg agcgagcgcg cagagaggga gtgggcaa                         4718
<210> 2
   <211> 623
   <212> PRT
   <213> AAV-1
<400> 2
   Met Pro Gly Phe Tyr Glu Ile Val Ile Lys Val Pro Ser Asp Leu Asp
    1               5                  10                  15
   Glu His Leu Pro Gly Ile Ser Asp Ser Phe Val Ser Trp Val Ala Glu
               20                  25                  30
   Lys Glu Trp Glu Leu Pro Pro Asp Ser Asp Met Asp Leu Asn Leu Ile
           35                  40                  45
   Glu Gln Ala Pro Leu Thr Val Ala Glu Lys Leu Gln Arg Asp Phe Leu
       50                  55                  60
   Val Gln Trp Arg Arg Val Ser Lys Ala Pro Glu Ala Leu Phe Phe Val
   65                  70                  75                  80
   Gln Phe Glu Lys Gly Glu Ser Tyr Phe His Leu His Ile Leu Val Glu
                   85                  90                  95
   Thr Thr Gly Val Lys Ser Met Val Leu Gly Arg Phe Leu Ser Gln Ile
               100                 105                 110
   Arg Asp Lys Leu Val Gln Thr Ile Tyr Arg Gly Ile Glu Pro Thr Leu
           115                 120                 125
   Pro Asn Trp Phe Ala Val Thr Lys Thr Arg Asn Gly Ala Gly Gly Gly
       130                 135                 140
   Asn Lys Val Val Asp Glu Cys Tyr Ile Pro Asn Tyr Leu Leu Pro Lys
   145                 150                 155                 160
   Thr Gln Pro Glu Leu Gln Trp Ala Trp Thr Asn Met Glu Glu Tyr Ile
                   165                 170                 175
   Ser Ala Cys Leu Asn Leu Ala Glu Arg Lys Arg Leu Val Ala Gln His
               180                 185                 190
   Leu Thr His Val Ser Gln Thr Gln Glu Gln Asn Lys Glu Asn Leu Asn
           195                 200                 205
   Pro Asn Ser Asp Ala Pro Val Ile Arg Ser Lys Thr Ser Ala Arg Tyr
       210                 215                 220
   Met Glu Leu Val Gly Trp Leu Val Asp Arg Gly Ile Thr Ser Glu Lys
   225                 230                 235                 240
   Gln Trp Ile Gln Glu Asp Gln Ala Ser Tyr Ile Ser Phe Asn Ala Ala
                   245                 250                 255
   Ser Asn Ser Arg Ser Gln Ile Lys Ala Ala Leu Asp Asn Ala Gly Lys
               260                 265                 270
   Ile Met Ala Leu Thr Lys Ser Ala Pro Asp Tyr Leu Val Gly Pro Ala
           275                 280                 285
   Pro Pro Ala Asp Ile Lys Thr Asn Arg Ile Tyr Arg Ile Leu Glu Leu
       290                 295                 300
   Asn Gly Tyr Glu Pro Ala Tyr Ala Gly Ser Val Phe Leu Gly Trp Ala
   305                 310                 315                 320
   Gln Lys Arg Phe Gly Lys Arg Asn Thr Ile Trp Leu Phe Gly Pro Ala
                   325                 330                 335
   Thr Thr Gly Lys Thr Asn Ile Ala Glu Ala Ile Ala His Ala Val Pro
               340                 345                 350
   Phe Tyr Gly Cys Val Asn Trp Thr Asn Glu Asn Phe Pro Phe Asn Asp
           355                 360                 365
   Cys Val Asp Lys Met Val Ile Trp Trp Glu Glu Gly Lys Met Thr Ala
       370                 375                 380
   Lys Val Val Glu Ser Ala Lys Ala Ile Leu Gly Gly Ser Lys Val Arg
   385                 390                 395                 400
   Val Asp Gln Lys Cys Lys Ser Ser Ala Gln Ile Asp Pro Thr Pro Val
   405 410 415
   Ile Val Thr Ser Asn Thr Asn Met Cys Ala Val Ile Asp Gly Asn Ser
   420 425 430
   Thr Thr Phe Glu His Gln Gln Pro Leu Gln Asp Arg Met Phe Lys Phe
   435 440 445
   Glu Leu Thr Arg Arg Leu Glu His Asp Phe Gly Lys Val Thr Lys Gln
   450 455 460
   Glu Val Lys Glu Phe Phe Arg Trp Ala Gln Asp His Val Thr Glu Val
   465 470 475 480
   Ala His Glu Phe Tyr Val Arg Lys Gly Gly Ala Asn Lys Arg Pro Ala
   485 490 495
   Pro Asp Asp Ala Asp Lys Ser Glu Pro Lys Arg Ala Cys Pro Ser Val
   500 505 510
   Ala Asp Pro Ser Thr Ser Asp Ala Glu Gly Ala Pro Val Asp Phe Ala
   515 520 525
   Asp Arg Tyr Gln Asn Lys Cys Ser Arg His Ala Gly Met Leu Gln Met
   530 535 540
   Leu Phe Pro Cys Lys Thr Cys Glu Arg Met Asn Gln Asn Phe Asn Ile
   545 550 555 560
   Cys Phe Thr His Gly Thr Arg Asp Cys Ser Glu Cys Phe Pro Gly Val
   565 570 575
   Ser Glu Ser Gln Pro Val Val Arg Lys Arg Thr Tyr Arg Lys Leu Cys
   580 585 590
   Ala Ile His His Leu Leu Gly Arg Ala Pro Glu Ile Ala Cys Ser Ala
   595 600 605
   Cys Asp Leu Val Asn Val Asp Leu Asp Asp Cys Val Ser Glu Gln
   610 615 620
<210> 3
   <211> 736
   <212> PRT
   <213> AAV-1
<400> 3
   Met Ala Ala Asp Gly Tyr Leu Pro Asp Trp Leu Glu Asp Asn Leu Ser
   1 5 10 15
   Glu Gly Ile Arg Glu Trp Trp Asp Leu Lys Pro Gly Ala Pro Lys Pro
   20 25 30
   Lys Ala Asn Gln Gln Lys Gln Asp Asp Gly Arg Gly Leu Val Leu Pro
   35 40 45
   Gly Tyr Lys Tyr Leu Gly Pro Phe Asn Gly Leu Asp Lys Gly Glu Pro
   50 55 60
   Val Asn Ala Ala Asp Ala Ala Ala Leu Glu His Asp Lys Ala Tyr Asp
   65 70 75 80
   Gln Gln Leu Lys Ala Gly Asp Asn Pro Tyr Leu Arg Tyr Asn His Ala
   85 90 95
   Asp Ala Glu Phe Gln Glu Arg Leu Gln Glu Asp Thr Ser Phe Gly Gly
   100 105 110
   Asn Leu Gly Arg Ala Val Phe Gln Ala Lys Lys Arg Val Leu Glu Pro
   115 120 125
   Leu Gly Leu Val Glu Glu Gly Ala Lys Thr Ala Pro Gly Lys Lys Arg
   130 135 140
   Pro Val Glu Gln Ser Pro Gln Glu Pro Asp Ser Ser Ser Gly Ile Gly
   145 150 155 160
   Lys Thr Gly Gln Gln Pro Ala Lys Lys Arg Leu Asn Phe Gly Gln Thr
   165 170 175
   Gly Asp Ser Glu Ser Val Pro Asp Pro Gln Pro Leu Gly Glu Pro Pro
   180 185 190
   Ala Thr Pro Ala Ala Val Gly Pro Thr Thr Met Ala Ser Gly Gly Gly
   195 200 205
   Ala Pro Met Ala Asp Asn Asn Glu Gly Ala Asp Gly Val Gly Asn Ala
   210 215 220
   Ser Gly Asn Trp His Cys Asp Ser Thr Trp Leu Gly Asp Arg Val Ile
   225 230 235 240
   Thr Thr Ser Thr Arg Thr Trp Ala Leu Pro Thr Tyr Asn Asn His Leu
   245 250 255
   Tyr Lys Gln Ile Ser Ser Ala Ser Thr Gly Ala Ser Asn Asp Asn His
   260 265 270
   Tyr Phe Gly Tyr Ser Thr Pro Trp Gly Tyr Phe Asp Phe Asn Arg Phe
   275 280 285
   His Cys His Phe Ser Pro Arg Asp Trp Gln Arg Leu Ile Asn Asn Asn
   290 295 300
   Trp Gly Phe Arg Pro Lys Arg Leu Asn Phe Lys Leu Phe Asn Ile Gln
   305 310 315 320
   Val Lys Glu Val Thr Thr Asn Asp Gly Val Thr Thr Ile Ala Asn Asn
   325 330 335
   Leu Thr Ser Thr Val Gln Val Phe Ser Asp Ser Glu Tyr Gln Leu Pro
   340 345 350
   Tyr Val Leu Gly Ser Ala His Gln Gly Cys Leu Pro Pro Phe Pro Ala
   355 360 365
   Asp Val Phe Met Ile Pro Gln Tyr Gly Tyr Leu Thr Leu Asn Asn Gly
   370 375 380
   Ser Gln Ala Val Gly Arg Ser Ser Phe Tyr Cys Leu Glu Tyr Phe Pro
   385 390 395 400
   Ser Gln Met Leu Arg Thr Gly Asn Asn Phe Thr Phe Ser Tyr Thr Phe
   405 410 415
   Glu Glu Val Pro Phe His Ser Ser Tyr Ala His Ser Gln Ser Leu Asp
   420 425 430
   Arg Leu Met Asn Pro Leu Ile Asp Gln Tyr Leu Tyr Tyr Leu Asn Arg
   435 440 445
   Thr Gln Asn Gln Ser Gly Ser Ala Gln Asn Lys Asp Leu Leu Phe Ser
   450 455 460
   Arg Gly Ser Pro Ala Gly Met Ser Val Gln Pro Lys Asn Trp Leu Pro
   465 470 475 480
   Gly Pro Cys Tyr Arg Gln Gln Arg Val Ser Lys Thr Lys Thr Asp Asn
   485 490 495
   Asn Asn Ser Asn Phe Thr Trp Thr Gly Ala Ser Lys Tyr Asn Leu Asn
   500 505 510
   Gly Arg Glu Ser Ile Ile Asn Pro Gly Thr Ala Met Ala Ser His Lys
   515 520 525
   Asp Asp Glu Asp Lys Phe Phe Pro Met Ser Gly Val Met Ile Phe Gly
   530 535 540
   Lys Glu Ser Ala Gly Ala Ser Asn Thr Ala Leu Asp Asn Val Met Ile
   545 550 555 560
   Thr Asp Glu Glu Glu Ile Lys Ala Thr Asn Pro Val Ala Thr Glu Arg
   565 570 575
   Phe Gly Thr Val Ala Val Asn Phe Gln Ser Ser Ser Thr Asp Pro Ala
   580 585 590
   Thr Gly Asp Val His Ala Met Gly Ala Leu Pro Gly Met Val Trp Gln
   595 600 605
   Asp Arg Asp Val Tyr Leu Gln Gly Pro Ile Trp Ala Lys Ile Pro His
   610 615 620
   Thr Asp Gly His Phe His Pro Ser Pro Leu Met Gly Gly Phe Gly Leu
   625 630 635 640
   Lys Asn Pro Pro Pro Gln Ile Leu Ile Lys Asn Thr Pro Val Pro Ala
   645 650 655
   Asn Pro Pro Ala Glu Phe Ser Ala Thr Lys Phe Ala Ser Phe Ile Thr
   660 665 670
   Gln Tyr Ser Thr Gly Gln Val Ser Val Glu Ile Glu Trp Glu Leu Gln
   675 680 685
   Lys Glu Asn Ser Lys Arg Trp Asn Pro Glu Val Gln Tyr Thr Ser Asn
   690 695 700
   Tyr Ala Lys Ser Ala Asn Val Asp Phe Thr Val Asp Asn Asn Gly Leu
   705 710 715 720
   Tyr Thr Glu Pro Arg Pro Ile Gly Thr Arg Tyr Leu Thr Arg Pro Leu
   725 730 735
<210> 4
   <211> 1872
   <212> DNA
   <213> AAV-1
<220>
   <221> CDS
   <222> (1)..(1869)
<400> 4
   atg ccg ggc ttc tac gag atc gtg atc aag gtg ccg agc gac ctg gac     48
   Met Pro Gly Phe Tyr Glu Ile Val Ile Lys Val Pro Ser Asp Leu Asp
   1               5                   10                  15
   gag cac ctg ccg ggc att tct gac tcg ttt gtg agc tgg gtg gcc gag     96
   Glu His Leu Pro Gly Ile Ser Asp Ser Phe Val Ser Trp Val Ala Glu
               20                  25                  30
   aag gaa tgg gag ctg ccc ccg gat tct gac atg gat ctg aat ctg att     144
   Lys Glu Trp Glu Leu Pro Pro Asp Ser Asp Met Asp Leu Asn Leu Ile
           35                  40                  45
   gag cag gca ccc ctg acc gtg gcc gag aag ctg cag cgc gac ttc ctg     192
   Glu Gln Ala Pro Leu Thr Val Ala Glu Lys Leu Gln Arg Asp Phe Leu
       50                  55                  60
   gtc caa tgg cgc cgc gtg agt aag gcc ccg gag gcc ctc ttc ttt gtt     240
   Val Gln Trp Arg Arg Val Ser Lys Ala Pro Glu Ala Leu Phe Phe Val
   65                  70                  75                  80
   cag ttc gag aag ggc gag tcc tac ttc cac ctc cat att ctg gtg gag     288
   Gln Phe Glu Lys Gly Glu Ser Tyr Phe His Leu His Ile Leu Val Glu
                   85                  90                  95
   acc acg ggg gtc aaa tcc atg gtg ctg ggc cgc ttc ctg agt cag att     336
   Thr Thr Gly Val Lys Ser Met Val Leu Gly Arg Phe Leu Ser Gln Ile
               100                 105                 110
   agg gac aag ctg gtg cag acc atc tac cgc ggg atc gag ccg acc ctg     384
   Arg Asp Lys Leu Val Gln Thr Ile Tyr Arg Gly Ile Glu Pro Thr Leu
           115                 120                 125
   ccc aac tgg ttc gcg gtg acc aag acg cgt aat ggc gcc gga ggg ggg     432
   Pro Asn Trp Phe Ala Val Thr Lys Thr Arg Asn Gly Ala Gly Gly Gly
       130                 135                 140
   aac aag gtg gtg gac gag tgc tac atc ccc aac tac ctc ctg ccc aag     480
   Asn Lys Val Val Asp Glu Cys Tyr Ile Pro Asn Tyr Leu Leu Pro Lys
   145                 150                 155                 160
   act cag ccc gag ctg cag tgg gcg tgg act aac atg gag gag tat ata     528
   Thr Gln Pro Glu Leu Gln Trp Ala Trp Thr Asn Met Glu Glu Tyr Ile
                   165                 170                 175
   agc gcc tgt ttg aac ctg gcc gag cgc aaa cgg ctc gtg gcg cag cac      576
   Ser Ala Cys Leu Asn Leu Ala Glu Arg Lys Arg Leu Val Ala Gln His
               180                 185                 190
   ctg acc cac gtc agc cag acc cag gag cag aac aag gag aat ctg aac      624
   Leu Thr His Val Ser Gln Thr Gln Glu Gln Asn Lys Glu Asn Leu Asn
           195                 200                 205
   ccc aat tct gac gcg cct gtc atc cgg tca aaa acc tcc gcg cgc tac      672
   Pro Asn Ser Asp Ala Pro Val Ile Arg Ser Lys Thr Ser Ala Arg Tyr
       210                 215                 220
   atg gag ctg gtc ggg tgg ctg gtg gac cgg ggc atc acc tcc gag aag      720
   Met Glu Leu Val Gly Trp Leu Val Asp Arg Gly Ile Thr Ser Glu Lys
   225                 230                 235                 240
   cag tgg atc cag gag gac cag gcc tcg tac atc tcc ttc aac gcc gct      768
   Gln Trp Ile Gln Glu Asp Gln Ala Ser Tyr Ile Ser Phe Asn Ala Ala
                   245                 250                 255
   tcc aac tcg cgg tcc cag atc aag gcc gct ctg gac aat gcc ggc aag      816
   Ser Asn Ser Arg Ser Gln Ile Lys Ala Ala Leu Asp Asn Ala Gly Lys
               260                 265                 270
   atc atg gcg ctg acc aaa tcc gcg ccc gac tac ctg gta ggc ccc gct      864
   Ile Met Ala Leu Thr Lys Ser Ala Pro Asp Tyr Leu Val Gly Pro Ala
           275                 280                 285
   ccg ccc gcg gac att aaa acc aac cgc atc tac cgc atc ctg gag ctg      912
   Pro Pro Ala Asp Ile Lys Thr Asn Arg Ile Tyr Arg Ile Leu Glu Leu
       290                 295                 300
   aac ggc tac gaa cct gcc tac gcc ggc tcc gtc ttt ctc ggc tgg gcc      960
   Asn Gly Tyr Glu Pro Ala Tyr Ala Gly Ser Val Phe Leu Gly Trp Ala
   305                 310                 315                 320
   cag aaa agg ttc ggg aag cgc aac acc atc tgg ctg ttt ggg ccg gcc      1008
   Gln Lys Arg Phe Gly Lys Arg Asn Thr Ile Trp Leu Phe Gly Pro Ala
                   325                 330                 335
   acc acg ggc aag acc aac atc gcg gaa gcc atc gcc cac gcc gtg ccc      1056
   Thr Thr Gly Lys Thr Asn Ile Ala Glu Ala Ile Ala His Ala Val Pro
               340                 345                 350
   ttc tac ggc tgc gtc aac tgg acc aat gag aac ttt ccc ttc aat gat      1104
   Phe Tyr Gly Cys Val Asn Trp Thr Asn Glu Asn Phe Pro Phe Asn Asp
           355                 360                 365
   tgc gtc gac aag atg gtg atc tgg tgg gag gag ggc aag atg acg gcc    1152
   Cys Val Asp Lys Met Val Ile Trp Trp Glu Glu Gly Lys Met Thr Ala
       370                 375                 380
   aag gtc gtg gag tcc gcc aag gcc att ctc ggc ggc agc aag gtg cgc    1200
   Lys Val Val Glu Ser Ala Lys Ala Ile Leu Gly Gly Ser Lys Val Arg
   385                 390                 395                 400
   gtg gac caa aag tgc aag tcg tcc gcc cag atc gac ccc acc ccc gtg    1248
   Val Asp Gln Lys Cys Lys Ser Ser Ala Gln Ile Asp Pro Thr Pro Val
                   405                 410                 415
   atc gtc acc tcc aac acc aac atg tgc gcc gtg att gac ggg aac agc    1296
   Ile Val Thr Ser Asn Thr Asn Met Cys Ala Val Ile Asp Gly Asn Ser
               420                 425                 430
   acc acc ttc gag cac cag cag ccg ttg cag gac cgg atg ttc aaa ttt    1344
   Thr Thr Phe Glu His Gln Gln Pro Leu Gln Asp Arg Met Phe Lys Phe
           435                 440                 445
   gaa ctc acc cgc cgt ctg gag cat gac ttt ggc aag gtg aca aag cag    1392
   Glu Leu Thr Arg Arg Leu Glu His Asp Phe Gly Lys Val Thr Lys Gln
       450                 455                 460
   gaa gtc aaa gag ttc ttc cgc tgg gcg cag gat cac gtg acc gag gtg    1440
   Glu Val Lys Glu Phe Phe Arg Trp Ala Gln Asp His Val Thr Glu Val
   465                 470                 475                 480
   gcg cat gag ttc tac gtc aga aag ggt gga gcc aac aaa aga ccc gcc    1488
   Ala His Glu Phe Tyr Val Arg Lys Gly Gly Ala Asn Lys Arg Pro Ala
                   485                 490                 495
   ccc gat gac gcg gat aaa agc gag ccc aag cgg gcc tgc ccc tca gtc    1536
   Pro Asp Asp Ala Asp Lys Ser Glu Pro Lys Arg Ala Cys Pro Ser Val
               500                 505                 510
   gcg gat cca tcg acg tca gac gcg gaa gga gct ccg gtg gac ttt gcc    1584
   Ala Asp Pro Ser Thr Ser Asp Ala Glu Gly Ala Pro Val Asp Phe Ala
           515                 520                 525
   gac agg tac caa aac aaa tgt tct cgt cac gcg ggc atg ctt cag atg    1632
   Asp Arg Tyr Gln Asn Lys Cys Ser Arg His Ala Gly Met Leu Gln Met
       530                 535                 540
   ctg ttt ccc tgc aag aca tgc gag aga atg aat cag aat ttc aac att    1680
   Leu Phe Pro Cys Lys Thr Cys Glu Arg Met Asn Gln Asn Phe Asn Ile
   545                 550                 555                 560
   tgc ttc acg cac ggg acg aga gac tgt tca gag tgc ttc ccc ggc gtg    1728
   Cys Phe Thr His Gly Thr Arg Asp Cys Ser Glu Cys Phe Pro Gly Val
                   565                 570                 575
   tca gaa tct caa ccg gtc gtc aga aag agg acg tat cgg aaa ctc tgt    1776
   Ser Glu Ser Gln Pro Val Val Arg Lys Arg Thr Tyr Arg Lys Leu Cys
               580                 585                 590
   gcc att cat cat ctg ctg ggg cgg gct ccc gag att gct tgc tcg gcc    1824
   Ala Ile His His Leu Leu Gly Arg Ala Pro Glu Ile Ala Cys Ser Ala
           595                 600                 605
   tgc gat ctg gtc aac gtg gac ctg gat gac tgt gtt tct gag caa taa    1872
   Cys Asp Leu Val Asn Val Asp Leu Asp Asp Cys Val Ser Glu Gln
       610                 615                 620
<210> 5
   <211> 623
   <212> PRT
   <213> AAV-1
<400> 5
   Met Pro Gly Phe Tyr Glu Ile Val Ile Lys Val Pro Ser Asp Leu Asp
   1               5                   10                  15
   Glu His Leu Pro Gly Ile Ser Asp Ser Phe Val Ser Trp Val Ala Glu
               20                  25                  30
   Lys Glu Trp Glu Leu Pro Pro Asp Ser Asp Met Asp Leu Asn Leu Ile
           35                  40                  45
   Glu Gln Ala Pro Leu Thr Val Ala Glu Lys Leu Gln Arg Asp Phe Leu
       50                  55                  60
   Val Gln Trp Arg Arg Val Ser Lys Ala Pro Glu Ala Leu Phe Phe Val
   65                  70                  75                  80
   Gln Phe Glu Lys Gly Glu Ser Tyr Phe His Leu His Ile Leu Val Glu
                   85                  90                  95
   Thr Thr Gly Val Lys Ser Met Val Leu Gly Arg Phe Leu Ser Gln Ile
               100                 105                 110
   Arg Asp Lys Leu Val Gln Thr Ile Tyr Arg Gly Ile Glu Pro Thr Leu
   115 120 125
   Pro Asn Trp Phe Ala Val Thr Lys Thr Arg Asn Gly Ala Gly Gly Gly
   130 135 140
   Asn Lys Val Val Asp Glu Cys Tyr Ile Pro Asn Tyr Leu Leu Pro Lys
   145 150 155 160
   Thr Gln Pro Glu Leu Gln Trp Ala Trp Thr Asn Met Glu Glu Tyr Ile
   165 170 175
   Ser Ala Cys Leu Asn Leu Ala Glu Arg Lys Arg Leu Val Ala Gln His
   180 185 190
   Leu Thr His Val Ser Gln Thr Gln Glu Gln Asn Lys Glu Asn Leu Asn
   195 200 205
   Pro Asn Ser Asp Ala Pro Val Ile Arg Ser Lys Thr Ser Ala Arg Tyr
   210 215 220
   Met Glu Leu Val Gly Trp Leu Val Asp Arg Gly Ile Thr Ser Glu Lys
   225 230 235 240
   Gln Trp Ile Gln Glu Asp Gln Ala Ser Tyr Ile Ser Phe Asn Ala Ala
   245 250 255
   Ser Asn Ser Arg Ser Gln Ile Lys Ala Ala Leu Asp Asn Ala Gly Lys
   260 265 270
   Ile Met Ala Leu Thr Lys Ser Ala Pro Asp Tyr Leu Val Gly Pro Ala
   275 280 285
   Pro Pro Ala Asp Ile Lys Thr Asn Arg Ile Tyr Arg Ile Leu Glu Leu
   290 295 300
   Asn Gly Tyr Glu Pro Ala Tyr Ala Gly Ser Val Phe Leu Gly Trp Ala
   305 310 315 320
   Gln Lys Arg Phe Gly Lys Arg Asn Thr Ile Trp Leu Phe Gly Pro Ala
   325 330 335
   Thr Thr Gly Lys Thr Asn Ile Ala Glu Ala Ile Ala His Ala Val Pro
   340 345 350
   Phe Tyr Gly Cys Val Asn Trp Thr Asn Glu Asn Phe Pro Phe Asn Asp
   355 360 365
   Cys Val Asp Lys Met Val Ile Trp Trp Glu Glu Gly Lys Met Thr Ala
   370 375 380
   Lys Val Val Glu Ser Ala Lys Ala Ile Leu Gly Gly Ser Lys Val Arg
   385 390 395 400
   Val Asp Gln Lys Cys Lys Ser Ser Ala Gln Ile Asp Pro Thr Pro Val
   405 410 415
   Ile Val Thr Ser Asn Thr Asn Met Cys Ala Val Ile Asp Gly Asn Ser
   420 425 430
   Thr Thr Phe Glu His Gln Gln Pro Leu Gln Asp Arg Met Phe Lys Phe
   435 440 445
   Glu Leu Thr Arg Arg Leu Glu His Asp Phe Gly Lys Val Thr Lys Gln
   450 455 460
   Glu Val Lys Glu Phe Phe Arg Trp Ala Gln Asp His Val Thr Glu Val
   465 470 475 480
   Ala His Glu Phe Tyr Val Arg Lys Gly Gly Ala Asn Lys Arg Pro Ala
   485 490 495
   Pro Asp Asp Ala Asp Lys Ser Glu Pro Lys Arg Ala Cys Pro Ser Val
   500 505 510
   Ala Asp Pro Ser Thr Ser Asp Ala Glu Gly Ala Pro Val Asp Phe Ala
   515 520 525
   Asp Arg Tyr Gln Asn Lys Cys Ser Arg His Ala Gly Met Leu Gln Met
   530 535 540
   Leu Phe Pro Cys Lys Thr Cys Glu Arg Met Asn Gln Asn Phe Asn Ile
   545 550 555 560
   Cys Phe Thr His Gly Thr Arg Asp Cys Ser Glu Cys Phe Pro Gly Val
   565 570 575
   Ser Glu Ser Gln Pro Val Val Arg Lys Arg Thr Tyr Arg Lys Leu Cys
   580 585 590
   Ala Ile His His Leu Leu Gly Arg Ala Pro Glu Ile Ala Cys Ser Ala
   595 600 605
   Cys Asp Leu Val Asn Val Asp Leu Asp Asp Cys Val Ser Glu Gln
   610 615 620
<210> 6
   <211> 1641
   <212> DNA
   <213> AAV-1
<220>
   <221> CDS
   <222> (1)..(1638)
<400> 6
   atg ccg ggc ttc tac gag atc gtg atc aag gtg ccg agc gac ctg gac     48
   Met Pro Gly Phe Tyr Glu Ile Val Ile Lys Val Pro Ser Asp Leu Asp
    1               5                  10                  15
   gag cac ctg ccg ggc att tct gac tcg ttt gtg agc tgg gtg gcc gag     96
   Glu His Leu Pro Gly Ile Ser Asp Ser Phe Val Ser Trp Val Ala Glu
               20                  25                  30
   aag gaa tgg gag ctg ccc ccg gat tct gac atg gat ctg aat ctg att    144
   Lys Glu Trp Glu Leu Pro Pro Asp Ser Asp Met Asp Leu Asn Leu Ile
           35                  40                  45
   gag cag gca ccc ctg acc gtg gcc gag aag ctg cag cgc gac ttc ctg    192
   Glu Gln Ala Pro Leu Thr Val Ala Glu Lys Leu Gln Arg Asp Phe Leu
       50                  55                  60
   gtc caa tgg cgc cgc gtg agt aag gcc ccg gag gcc ctc ttc ttt gtt    240
   Val Gln Trp Arg Arg Val Ser Lys Ala Pro Glu Ala Leu Phe Phe Val
   65                  70                  75                  80
   cag ttc gag aag ggc gag tcc tac ttc cac ctc cat att ctg gtg gag    288
   Gln Phe Glu Lys Gly Glu Ser Tyr Phe His Leu His Ile Leu Val Glu
                   85                  90                  95
   acc acg ggg gtc aaa tcc atg gtg ctg ggc cgc ttc ctg agt cag att    336
   Thr Thr Gly Val Lys Ser Met Val Leu Gly Arg Phe Leu Ser Gln Ile
               100                 105                 110
   agg gac aag ctg gtg cag acc atc tac cgc ggg atc gag ccg acc ctg    384
   Arg Asp Lys Leu Val Gln Thr Ile Tyr Arg Gly Ile Glu Pro Thr Leu
           115                 120                 125
   ccc aac tgg ttc gcg gtg acc aag acg cgt aat ggc gcc gga ggg ggg    432
   Pro Asn Trp Phe Ala Val Thr Lys Thr Arg Asn Gly Ala Gly Gly Gly
       130                 135                 140
   aac aag gtg gtg gac gag tgc tac atc ccc aac tac ctc ctg ccc aag    480
   Asn Lys Val Val Asp Glu Cys Tyr Ile Pro Asn Tyr Leu Leu Pro Lys
   145                 150                 155                 160
   act cag ccc gag ctg cag tgg gcg tgg act aac atg gag gag tat ata    528
   Thr Gln Pro Glu Leu Gln Trp Ala Trp Thr Asn Met Glu Glu Tyr Ile
                   165                 170                 175
   agc gcc tgt ttg aac ctg gcc gag cgc aaa cgg ctc gtg gcg cag cac    576
   Ser Ala Cys Leu Asn Leu Ala Glu Arg Lys Arg Leu Val Ala Gln His
               180                 185                 190
   ctg acc cac gtc agc cag acc cag gag cag aac aag gag aat ctg aac    624
   Leu Thr His Val Ser Gln Thr Gln Glu Gln Asn Lys Glu Asn Leu Asn
           195                 200                 205
   ccc aat tct gac gcg cct gtc atc cgg tca aaa acc tcc gcg cgc tac    672
   Pro Asn Ser Asp Ala Pro Val Ile Arg Ser Lys Thr Ser Ala Arg Tyr
       210                 215                 220
   atg gag ctg gtc ggg tgg ctg gtg gac cgg ggc atc acc tcc gag aag    720
   Met Glu Leu Val Gly Trp Leu Val Asp Arg Gly Ile Thr Ser Glu Lys
   225                 230                 235                 240
   cag tgg atc cag gag gac cag gcc tcg tac atc tcc ttc aac gcc gct    768
   Gln Trp Ile Gln Glu Asp Gln Ala Ser Tyr Ile Ser Phe Asn Ala Ala
                   245                 250                 255
   tcc aac tcg cgg tcc cag atc aag gcc gct ctg gac aat gcc ggc aag    816
   Ser Asn Ser Arg Ser Gln Ile Lys Ala Ala Leu Asp Asn Ala Gly Lys
               260                 265                 270
   atc atg gcg ctg acc aaa tcc gcg ccc gac tac ctg gta ggc ccc gct    864
   Ile Met Ala Leu Thr Lys Ser Ala Pro Asp Tyr Leu Val Gly Pro Ala
           275                 280                 285
   ccg ccc gcg gac att aaa acc aac cgc atc tac cgc atc ctg gag ctg    912
   Pro Pro Ala Asp Ile Lys Thr Asn Arg Ile Tyr Arg Ile Leu Glu Leu
       290                 295                 300
   aac ggc tac gaa cct gcc tac gcc ggc tcc gtc ttt ctc ggc tgg gcc    960
   Asn Gly Tyr Glu Pro Ala Tyr Ala Gly Ser Val Phe Leu Gly Trp Ala
   305                 310                 315                 320
   cag aaa agg ttc ggg aag cgc aac acc atc tgg ctg ttt ggg ccg gcc    1008
   Gln Lys Arg Phe Gly Lys Arg Asn Thr Ile Trp Leu Phe Gly Pro Ala
                   325                 330                 335
   acc acg ggc aag acc aac atc gcg gaa gcc atc gcc cac gcc gtg ccc    1056
   Thr Thr Gly Lys Thr Asn Ile Ala Glu Ala Ile Ala His Ala Val Pro
                   340                   345                   350
   ttc tac ggc tgc gtc aac tgg acc aat gag aac ttt ccc ttc aat gat     1104
   Phe Tyr Gly Cys Val Asn Trp Thr Asn Glu Asn Phe Pro Phe Asn Asp
               355                 360                 365
   tgc gtc gac aag atg gtg atc tgg tgg gag gag ggc aag atg acg gcc     1152
   Cys Val Asp Lys Met Val Ile Trp Trp Glu Glu Gly Lys Met Thr Ala
       370                 375                 380
   aag gtc gtg gag tcc gcc aag gcc att ctc ggc ggc agc aag gtg cgc     1200
   Lys Val Val Glu Ser Ala Lys Ala Ile Leu Gly Gly Ser Lys Val Arg
   385                 390                 395                 400
   gtg gac caa aag tgc aag tcg tcc gcc cag atc gac ccc acc ccc gtg     1248
   Val Asp Gln Lys Cys Lys Ser Ser Ala Gln Ile Asp Pro Thr Pro Val
                   405                 410                 415
   atc gtc acc tcc aac acc aac atg tgc gcc gtg att gac ggg aac agc     1296
   Ile Val Thr Ser Asn Thr Asn Met Cys Ala Val Ile Asp Gly Asn Ser
               420                 425                 430
   acc acc ttc gag cac cag cag ccg ttg cag gac cgg atg ttc aaa ttt     1344
   Thr Thr Phe Glu His Gln Gln Pro Leu Gln Asp Arg Met Phe Lys Phe
           435                 440                 445
   gaa ctc acc cgc cgt ctg gag cat gac ttt ggc aag gtg aca aag cag     1392
   Glu Leu Thr Arg Arg Leu Glu His Asp Phe Gly Lys Val Thr Lys Gln
       450                 455                 460
   gaa gtc aaa gag ttc ttc cgc tgg gcg cag gat cac gtg acc gag gtg     1440
   Glu Val Lys Glu Phe Phe Arg Trp Ala Gln Asp His Val Thr Glu Val
   465                 470                 475                 480
   gcg cat gag ttc tac gtc aga aag ggt gga gcc aac aaa aga ccc gcc     1488
   Ala His Glu Phe Tyr Val Arg Lys Gly Gly Ala Asn Lys Arg Pro Ala
                   485                 490                 495
   ccc gat gac gcg gat aaa agc gag ccc aag cgg gcc tgc ccc tca gtc     1536
   Pro Asp Asp Ala Asp Lys Ser Glu Pro Lys Arg Ala Cys Pro Ser Val
               500                 505                 510
   gcg gat cca tcg acg tca gac gcg gaa gga gct ccg gtg gac ttt gcc     1584
   Ala Asp Pro Ser Thr Ser Asp Ala Glu Gly Ala Pro Val Asp Phe Ala
           515                 520                 525
   gac agg tat ggc tgc cga tgg tta tct tcc aga ttg gct cga gga caa     1632
   Asp Arg Tyr Gly Cys Arg Trp Leu Ser Ser Arg Leu Ala Arg Gly Gln
   530 535 540
   cct ctc tga 1641
   Pro Leu
   545
<210> 7
   <211> 546
   <212> PRT
   <213> AAV-1
<400> 7
   Met Pro Gly Phe Tyr Glu Ile Val Ile Lys Val Pro Ser Asp Leu Asp
   1 5 10 15
   Glu His Leu Pro Gly Ile Ser Asp Ser Phe Val Ser Trp Val Ala Glu
   20 25 30
   Lys Glu Trp Glu Leu Pro Pro Asp Ser Asp Met Asp Leu Asn Leu Ile
   35 40 45
   Glu Gln Ala Pro Leu Thr Val Ala Glu Lys Leu Gln Arg Asp Phe Leu
   50 55 60
   Val Gln Trp Arg Arg Val Ser Lys Ala Pro Glu Ala Leu Phe Phe Val
   65 70 75 80
   Gln Phe Glu Lys Gly Glu Ser Tyr Phe His Leu His Ile Leu Val Glu
   85 90 95
   Thr Thr Gly Val Lys Ser Met Val Leu Gly Arg Phe Leu Ser Gln Ile
   100 105 110
   Arg Asp Lys Leu Val Gln Thr Ile Tyr Arg Gly Ile Glu Pro Thr Leu
   115 120 125
   Pro Asn Trp Phe Ala Val Thr Lys Thr Arg Asn Gly Ala Gly Gly Gly
   130 135 140
   Asn Lys Val Val Asp Glu Cys Tyr Ile Pro Asn Tyr Leu Leu Pro Lys
   145 150 155 160
   Thr Gln Pro Glu Leu Gln Trp Ala Trp Thr Asn Met Glu Glu Tyr Ile
   165 170 175
   Ser Ala Cys Leu Asn Leu Ala Glu Arg Lys Arg Leu Val Ala Gln His
   180 185 190
   Leu Thr His Val Ser Gln Thr Gln Glu Gln Asn Lys Glu Asn Leu Asn
           195                 200                 205
   Pro Asn Ser Asp Ala Pro Val Ile Arg Ser Lys Thr Ser Ala Arg Tyr
       210                 215                 220
   Met Glu Leu Val Gly Trp Leu Val Asp Arg Gly Ile Thr Ser Glu Lys
   225                 230                 235                 240
   Gln Trp Ile Gln Glu Asp Gln Ala Ser Tyr Ile Ser Phe Asn Ala Ala
                   245                 250                 255
   Ser Asn Ser Arg Ser Gln Ile Lys Ala Ala Leu Asp Asn Ala Gly Lys
               260                 265                 270
   Ile Met Ala Leu Thr Lys Ser Ala Pro Asp Tyr Leu Val Gly Pro Ala
           275                 280                 285
   Pro Pro Ala Asp Ile Lys Thr Asn Arg Ile Tyr Arg Ile Leu Glu Leu
       290                 295                 300
   Asn Gly Tyr Glu Pro Ala Tyr Ala Gly Ser Val Phe Leu Gly Trp Ala
   305                 310                 315                 320
   Gln Lys Arg Phe Gly Lys Arg Asn Thr Ile Trp Leu Phe Gly Pro Ala
                   325                 330                 335
   Thr Thr Gly Lys Thr Asn Ile Ala Glu Ala Ile Ala His Ala Val Pro
               340                 345                 350
   Phe Tyr Gly Cys Val Asn Trp Thr Asn Glu Asn Phe Pro Phe Asn Asp
           355                 360                 365
   Cys Val Asp Lys Met Val Ile Trp Trp Glu Glu Gly Lys Met Thr Ala
       370                 375                 380
   Lys Val Val Glu Ser Ala Lys Ala Ile Leu Gly Gly Ser Lys Val Arg
   385                 390                 395                 400
   Val Asp Gln Lys Cys Lys Ser Ser Ala Gln Ile Asp Pro Thr Pro Val
                   405                 410                 415
   Ile Val Thr Ser Asn Thr Asn Met Cys Ala Val Ile Asp Gly Asn Ser
               420                 425                 430
   Thr Thr Phe Glu His Gln Gln Pro Leu Gln Asp Arg Met Phe Lys Phe
           435                 440                 445
   Glu Leu Thr Arg Arg Leu Glu His Asp Phe Gly Lys Val Thr Lys Gln
       450                 455                 460
   Glu Val Lys Glu Phe Phe Arg Trp Ala Gln Asp His Val Thr Glu Val
   465                 470                 475                 480
   Ala His Glu Phe Tyr Val Arg Lys Gly Gly Ala Asn Lys Arg Pro Ala
                   485                 490                 495
   Pro Asp Asp Ala Asp Lys Ser Glu Pro Lys Arg Ala Cys Pro Ser Val
               500                 505                 510
   Ala Asp Pro Ser Thr Ser Asp Ala Glu Gly Ala Pro Val Asp Phe Ala
           515                 520                 525
   Asp Arg Tyr Gly Cys Arg Trp Leu Ser Ser Arg Leu Ala Arg Gly Gln
       530                 535                 540
   Pro Leu
   545
<210> 8
   <211> 1200
   <212> DNA
   <213> AAV-1
<220>
   <221> CDS
   <222> (1)..(1197)
<400> 8
   atg gag ctg gtc ggg tgg ctg gtg gac cgg ggc atc acc tcc gag aag     48
   Met Glu Leu Val Gly Trp Leu Val Asp Arg Gly Ile Thr Ser Glu Lys
     1               5                  10                  15
   cag tgg atc cag gag gac cag gcc tcg tac atc tcc ttc aac gcc gct     96
   Gln Trp Ile Gln Glu Asp Gln Ala Ser Tyr Ile Ser Phe Asn Ala Ala
                20                  25                  30
   tcc aac tcg cgg tcc cag atc aag gcc gct ctg gac aat gcc ggc aag     144
   Ser Asn Ser Arg Ser Gln Ile Lys Ala Ala Leu Asp Asn Ala Gly Lys
            35                  40                  45
   atc atg gcg ctg acc aaa tcc gcg ccc gac tac ctg gta ggc ccc gct     192
   Ile Met Ala Leu Thr Lys Ser Ala Pro Asp Tyr Leu Val Gly Pro Ala
        50                  55                  60
   ccg ccc gcg gac att aaa acc aac cgc atc tac cgc atc ctg gag ctg   240
   Pro Pro Ala Asp Ile Lys Thr Asn Arg Ile Tyr Arg Ile Leu Glu Leu
    65                 70                  75                  80
   aac ggc tac gaa cct gcc tac gcc ggc tcc gtc ttt ctc ggc tgg gcc   288
   Asn Gly Tyr Glu Pro Ala Tyr Ala Gly Ser Val Phe Leu Gly Trp Ala
                   85                  90                  95
   cag aaa agg ttc ggg aag cgc aac acc atc tgg ctg ttt ggg ccg gcc   336
   Gln Lys Arg Phe Gly Lys Arg Asn Thr Ile Trp Leu Phe Gly Pro Ala
               100                 105                 110
   acc acg ggc aag acc aac atc gcg gaa gcc atc gcc cac gcc gtg ccc   384
   Thr Thr Gly Lys Thr Asn Ile Ala Glu Ala Ile Ala His Ala Val Pro
           115                 120                 125
   ttc tac ggc tgc gtc aac tgg acc aat gag aac ttt ccc ttc aat gat   432
   Phe Tyr Gly Cys Val Asn Trp Thr Asn Glu Asn Phe Pro Phe Asn Asp
       130                 135                 140
   tgc gtc gac aag atg gtg atc tgg tgg gag gag ggc aag atg acg gcc   480
   Cys Val Asp Lys Met Val Ile Trp Trp Glu Glu Gly Lys Met Thr Ala
   145                 150                 155                 160
   aag gtc gtg gag tcc gcc aag gcc att ctc ggc ggc agc aag gtg cgc   528
   Lys Val Val Glu Ser Ala Lys Ala Ile Leu Gly Gly Ser Lys Val Arg
                   165                 170                 175
   gtg gac caa aag tgc aag tcg tcc gcc cag atc gac ccc acc ccc gtg   576
   Val Asp Gln Lys Cys Lys Ser Ser Ala Gln Ile Asp Pro Thr Pro Val
               180                 185                 190
   atc gtc acc tcc aac acc aac atg tgc gcc gtg att gac ggg aac agc   624
   Ile Val Thr Ser Asn Thr Asn Met Cys Ala Val Ile Asp Gly Asn Ser
           195                 200                 205
   acc acc ttc gag cac cag cag ccg ttg cag gac cgg atg ttc aaa ttt   672
   Thr Thr Phe Glu His Gln Gln Pro Leu Gln Asp Arg Met Phe Lys Phe
       210                 215                 220
   gaa ctc acc cgc cgt ctg gag cat gac ttt ggc aag gtg aca aag cag   720
   Glu Leu Thr Arg Arg Leu Glu His Asp Phe Gly Lys Val Thr Lys Gln
   225                 230                 235                 240
   gaa gtc aaa gag ttc ttc cgc tgg gcg cag gat cac gtg acc gag gtg   768
   Glu Val Lys Glu Phe Phe Arg Trp Ala Gln Asp His Val Thr Glu Val
                   245                 250                 255
   gcg cat gag ttc tac gtc aga aag ggt gga gcc aac aaa aga ccc gcc    816
   Ala His Glu Phe Tyr Val Arg Lys Gly Gly Ala Asn Lys Arg Pro Ala
               260                 265                 270
   ccc gat gac gcg gat aaa agc gag ccc aag cgg gcc tgc ccc tca gtc    864
   Pro Asp Asp Ala Asp Lys Ser Glu Pro Lys Arg Ala Cys Pro Ser Val
           275                 280                 285
   gcg gat cca tcg acg tca gac gcg gaa gga gct ccg gtg gac ttt gcc    912
   Ala Asp Pro Ser Thr Ser Asp Ala Glu Gly Ala Pro Val Asp Phe Ala
       290                 295                 300
   gac agg tac caa aac aaa tgt tct cgt cac gcg ggc atg ctt cag atg    960
   Asp Arg Tyr Gln Asn Lys Cys Ser Arg His Ala Gly Met Leu Gln Met
   305                 310                 315                 320
   ctg ttt ccc tgc aag aca tgc gag aga atg aat cag aat ttc aac att    1008
   Leu Phe Pro Cys Lys Thr Cys Glu Arg Met Asn Gln Asn Phe Asn Ile
                   325                 330                 335
   tgc ttc acg cac ggg acg aga gac tgt tca gag tgc ttc ccc ggc gtg    1056
   Cys Phe Thr His Gly Thr Arg Asp Cys Ser Glu Cys Phe Pro Gly Val
               340                 345                 350
   tca gaa tct caa ccg gtc gtc aga aag agg acg tat cgg aaa ctc tgt    1104
   Ser Glu Ser Gln Pro Val Val Arg Lys Arg Thr Tyr Arg Lys Leu Cys
           355                 360                 365
   gcc att cat cat ctg ctg ggg cgg gct ccc gag att gct tgc tcg gcc    1152
   Ala Ile His His Leu Leu Gly Arg Ala Pro Glu Ile Ala Cys Ser Ala
       370                 375                 380
   tgc gat ctg gtc aac gtg gac ctg gat gac tgt gtt tct gag caa taa    1200
   Cys Asp Leu Val Asn Val Asp Leu Asp Asp Cys Val Ser Glu Gln
   385                 390                 395
<210> 9
   <211> 399
   <212> PRT
   <213> AAV-1
<400> 9
   Met Glu Leu Val Gly Trp Leu Val Asp Arg Gly Ile Thr Ser Glu Lys
   1               5                   10                  15
   Gln Trp Ile Gln Glu Asp Gln Ala Ser Tyr Ile Ser Phe Asn Ala Ala
   20 25 30
   Ser Asn Ser Arg Ser Gln Ile Lys Ala Ala Leu Asp Asn Ala Gly Lys
   35 40 45
   Ile Met Ala Leu Thr Lys Ser Ala Pro Asp Tyr Leu Val Gly Pro Ala
   50 55 60
   Pro Pro Ala Asp Ile Lys Thr Asn Arg Ile Tyr Arg Ile Leu Glu Leu
   65 70 75 80
   Asn Gly Tyr Glu Pro Ala Tyr Ala Gly Ser Val Phe Leu Gly Trp Ala
   85 90 95
   Gln Lys Arg Phe Gly Lys Arg Asn Thr Ile Trp Leu Phe Gly Pro Ala
   100 105 110
   Thr Thr Gly Lys Thr Asn Ile Ala Glu Ala Ile Ala His Ala Val Pro
   115 120 125
   Phe Tyr Gly Cys Val Asn Trp Thr Asn Glu Asn Phe Pro Phe Asn Asp
   130 135 140
   Cys Val Asp Lys Met Val Ile Trp Trp Glu Glu Gly Lys Met Thr Ala
   145 150 155 160
   Lys Val Val Glu Ser Ala Lys Ala Ile Leu Gly Gly Ser Lys Val Arg
   165 170 175
   Val Asp Gln Lys Cys Lys Ser Ser Ala Gln Ile Asp Pro Thr Pro Val
   180 185 190
   Ile Val Thr Ser Asn Thr Asn Met Cys Ala Val Ile Asp Gly Asn Ser
   195 200 205
   Thr Thr Phe Glu His Gln Gln Pro Leu Gln Asp Arg Met Phe Lys Phe
   210 215 220
   Glu Leu Thr Arg Arg Leu Glu His Asp Phe Gly Lys Val Thr Lys Gln
   225 230 235 240
   Glu Val Lys Glu Phe Phe Arg Trp Ala Gln Asp His Val Thr Glu Val
   245 250 255
   Ala His Glu Phe Tyr Val Arg Lys Gly Gly Ala Asn Lys Arg Pro Ala
   260 265 270
   Pro Asp Asp Ala Asp Lys Ser Glu Pro Lys Arg Ala Cys Pro Ser Val
   275 280 285
   Ala Asp Pro Ser Thr Ser Asp Ala Glu Gly Ala Pro Val Asp Phe Ala
   290 295 300
   Asp Arg Tyr Gln Asn Lys Cys Ser Arg His Ala Gly Met Leu Gln Met
   305 310 315 320
   Leu Phe Pro Cys Lys Thr Cys Glu Arg Met Asn Gln Asn Phe Asn Ile
   325 330 335
   Cys Phe Thr His Gly Thr Arg Asp Cys Ser Glu Cys Phe Pro Gly Val
   340 345 350
   Ser Glu Ser Gln Pro Val Val Arg Lys Arg Thr Tyr Arg Lys Leu Cys
   355 360 365
   Ala Ile His His Leu Leu Gly Arg Ala Pro Glu Ile Ala Cys Ser Ala
   370 375 380
   Cys Asp Leu Val Asn Val Asp Leu Asp Asp Cys Val Ser Glu Gln
   385 390 395
<210> 10
   <211> 969
   <212> DNA
   <213> AAV-1
<220>
   <221> CDS
   <222> (1)..(966)
<220>
   <221> misc_feature
   <222> (943)..(944)
   <223> minor splice site
<400> 10
   atg gag ctg gtc ggg tgg ctg gtg gac cgg ggc atc acc tcc gag aag 48
   Met Glu Leu Val Gly Trp Leu Val Asp Arg Gly Ile Thr Ser Glu Lys
   1 5 10 15
   cag tgg atc cag gag gac cag gcc tcg tac atc tcc ttc aac gcc gct 96
   Gln Trp Ile Gln Glu Asp Gln Ala Ser Tyr Ile Ser Phe Asn Ala Ala
   20 25 30
   tcc aac tcg cgg tcc cag atc aag gcc gct ctg gac aat gcc ggc aag 144
   Ser Asn Ser Arg Ser Gln Ile Lys Ala Ala Leu Asp Asn Ala Gly Lys
           35                  40                  45
   atc atg gcg ctg acc aaa tcc gcg ccc gac tac ctg gta ggc ccc gct     192
   Ile Met Ala Leu Thr Lys Ser Ala Pro Asp Tyr Leu Val Gly Pro Ala
       50                  55                  60
   ccg ccc gcg gac att aaa acc aac cgc atc tac cgc atc ctg gag ctg     240
   Pro Pro Ala Asp Ile Lys Thr Asn Arg Ile Tyr Arg Ile Leu Glu Leu
   65                  70                  75                  80
   aac ggc tac gaa cct gcc tac gcc ggc tcc gtc ttt ctc ggc tgg gcc     288
   Asn Gly Tyr Glu Pro Ala Tyr Ala Gly Ser Val Phe Leu Gly Trp Ala
                   85                  90                  95
   cag aaa agg ttc ggg aag cgc aac acc atc tgg ctg ttt ggg ccg gcc     336
   Gln Lys Arg Phe Gly Lys Arg Asn Thr Ile Trp Leu Phe Gly Pro Ala
               100                 105                 110
   acc acg ggc aag acc aac atc gcg gaa gcc atc gcc cac gcc gtg ccc     384
   Thr Thr Gly Lys Thr Asn Ile Ala Glu Ala Ile Ala His Ala Val Pro
           115                 120                 125
   ttc tac ggc tgc gtc aac tgg acc aat gag aac ttt ccc ttc aat gat     432
   Phe Tyr Gly Cys Val Asn Trp Thr Asn Glu Asn Phe Pro Phe Asn Asp
       130                 135                 140
   tgc gtc gac aag atg gtg atc tgg tgg gag gag ggc aag atg acg gcc     480
   Cys Val Asp Lys Met Val Ile Trp Trp Glu Glu Gly Lys Met Thr Ala
   145                 150                 155                 160
   aag gtc gtg gag tcc gcc aag gcc att ctc ggc ggc agc aag gtg cgc     528
   Lys Val Val Glu Ser Ala Lys Ala Ile Leu Gly Gly Ser Lys Val Arg
                   165                 170                 175
   gtg gac caa aag tgc aag tcg tcc gcc cag atc gac ccc acc ccc gtg     576
   Val Asp Gln Lys Cys Lys Ser Ser Ala Gln Ile Asp Pro Thr Pro Val
               180                 185                 190
   atc gtc acc tcc aac acc aac atg tgc gcc gtg att gac ggg aac agc     624
   Ile Val Thr Ser Asn Thr Asn Met Cys Ala Val Ile Asp Gly Asn Ser
           195                 200                 205
   acc acc ttc gag cac cag cag ccg ttg cag gac cgg atg ttc aaa ttt     672
   Thr Thr Phe Glu His Gln Gln Pro Leu Gln Asp Arg Met Phe Lys Phe
       210                 215                 220
   gaa ctc acc cgc cgt ctg gag cat gac ttt ggc aag gtg aca aag cag     720
   Glu Leu Thr Arg Arg Leu Glu His Asp Phe Gly Lys Val Thr Lys Gln
   225                 230                 235                 240
   gaa gtc aaa gag ttc ttc cgc tgg gcg cag gat cac gtg acc gag gtg    768
   Glu Val Lys Glu Phe Phe Arg Trp Ala Gln Asp His Val Thr Glu Val
                   245                 250                 255
   gcg cat gag ttc tac gtc aga aag ggt gga gcc aac aaa aga ccc gcc    816
   Ala His Glu Phe Tyr Val Arg Lys Gly Gly Ala Asn Lys Arg Pro Ala
               260                 265                 270
   ccc gat gac gcg gat aaa agc gag ccc aag cgg gcc tgc ccc tca gtc    864
   Pro Asp Asp Ala Asp Lys Ser Glu Pro Lys Arg Ala Cys Pro Ser Val
           275                 280                 285
   gcg gat cca tcg acg tca gac gcg gaa gga gct ccg gtg gac ttt gcc    912
   Ala Asp Pro Ser Thr Ser Asp Ala Glu Gly Ala Pro Val Asp Phe Ala
       290                 295                 300
   gac agg tat ggc tgc cga tgg tta tct tcc aga ttg gct cga gga caa    960
   Asp Arg Tyr Gly Cys Arg Trp Leu Ser Ser Arg Leu Ala Arg Gly Gln
   305                 310                 315                 320
   cct ctc tga                                                        969
   Pro Leu
<210> 11
   <211> 322
   <212> PRT
   <213> AAV-1
<400> 11
   Met Glu Leu Val Gly Trp Leu Val Asp Arg Gly Ile Thr Ser Glu Lys
   1               5                   10                  15
   Gln Trp Ile Gln Glu Asp Gln Ala Ser Tyr Ile Ser Phe Asn Ala Ala
               20                  25                  30
   Ser Asn Ser Arg Ser Gln Ile Lys Ala Ala Leu Asp Asn Ala Gly Lys
           35                  40                  45
   Ile Met Ala Leu Thr Lys Ser Ala Pro Asp Tyr Leu Val Gly Pro Ala
       50                  55                  60
   Pro Pro Ala Asp Ile Lys Thr Asn Arg Ile Tyr Arg Ile Leu Glu Leu
   65                  70                  75                  80
   Asn Gly Tyr Glu Pro Ala Tyr Ala Gly Ser Val Phe Leu Gly Trp Ala
   85 90 95
   Gln Lys Arg Phe Gly Lys Arg Asn Thr Ile Trp Leu Phe Gly Pro Ala
   100 105 110
   Thr Thr Gly Lys Thr Asn Ile Ala Glu Ala Ile Ala His Ala Val Pro
   115 120 125
   Phe Tyr Gly Cys Val Asn Trp Thr Asn Glu Asn Phe Pro Phe Asn Asp
   130 135 140
   Cys Val Asp Lys Met Val Ile Trp Trp Glu Glu Gly Lys Met Thr Ala
   145 150 155 160
   Lys Val Val Glu Ser Ala Lys Ala Ile Leu Gly Gly Ser Lys Val Arg
   165 170 175
   Val Asp Gln Lys Cys Lys Ser Ser Ala Gln Ile Asp Pro Thr Pro Val
   180 185 190
   Ile Val Thr Ser Asn Thr Asn Met Cys Ala Val Ile Asp Gly Asn Ser
   195 200 205
   Thr Thr Phe Glu His Gln Gln Pro Leu Gln Asp Arg Met Phe Lys Phe
   210 215 220
   Glu Leu Thr Arg Arg Leu Glu His Asp Phe Gly Lys Val Thr Lys Gln
   225 230 235 240
   Glu Val Lys Glu Phe Phe Arg Trp Ala Gln Asp His Val Thr Glu Val
   245 250 255
   Ala His Glu Phe Tyr Val Arg Lys Gly Gly Ala Asn Lys Arg Pro Ala
   260 265 270
   Pro Asp Asp Ala Asp Lys Ser Glu Pro Lys Arg Ala Cys Pro Ser Val
   275 280 285
   Ala Asp Pro Ser Thr Ser Asp Ala Glu Gly Ala Pro Val Asp Phe Ala
   290 295 300
   Asp Arg Tyr Gly Cys Arg Trp Leu Ser Ser Arg Leu Ala Arg Gly Gln
   305 310 315 320
   Pro Leu
<210> 12
   <211> 2211
   <212> DNA
   <213> AAV-1
<220>
   <221> CDS
   <222> (1)..(2208)
<400> 12
   atg gct gcc gat ggt tat ctt cca gat tgg ctc gag gac aac ctc tct    48
   Met Ala Ala Asp Gly Tyr Leu Pro Asp Trp Leu Glu Asp Asn Leu Ser
     1               5                  10                  15
   gag ggc att cgc gag tgg tgg gac ttg aaa cct gga gcc ccg aag ccc    96
   Glu Gly Ile Arg Glu Trp Trp Asp Leu Lys Pro Gly Ala Pro Lys Pro
                20                  25                  30
   aaa gcc aac cag caa aag cag gac gac ggc cgg ggt ctg gtg ctt cct   144
   Lys Ala Asn Gln Gln Lys Gln Asp Asp Gly Arg Gly Leu Val Leu Pro
            35                  40                  45
   ggc tac aag tac ctc gga ccc ttc aac gga ctc gac aag ggg gag ccc   192
   Gly Tyr Lys Tyr Leu Gly Pro Phe Asn Gly Leu Asp Lys Gly Glu Pro
        50                  55                  60
   gtc aac gcg gcg gac gca gcg gcc ctc gag cac gac aag gcc tac gac   240
   Val Asn Ala Ala Asp Ala Ala Ala Leu Glu His Asp Lys Ala Tyr Asp
    65                  70                  75                  80
   cag cag ctc aaa gcg ggt gac aat ccg tac ctg cgg tat aac cac gcc   288
   Gln Gln Leu Lys Ala Gly Asp Asn Pro Tyr Leu Arg Tyr Asn His Ala
                    85                  90                  95
   gac gcc gag ttt cag gag cgt ctg caa gaa gat acg tct ttt ggg ggc   336
   Asp Ala Glu Phe Gln Glu Arg Leu Gln Glu Asp Thr Ser Phe Gly Gly
               100                 105                 110
   aac ctc ggg cga gca gtc ttc cag gcc aag aag cgg gtt ctc gaa cct   384
   Asn Leu Gly Arg Ala Val Phe Gln Ala Lys Lys Arg Val Leu Glu Pro
           115                 120                 125
   ctc ggt ctg gtt gag gaa ggc gct aag acg gct cct gga aag aaa cgt   432
   Leu Gly Leu Val Glu Glu Gly Ala Lys Thr Ala Pro Gly Lys Lys Arg
       130                 135                 140
   ccg gta gag cag tcg cca caa gag cca gac tcc tcc tcg ggc atc ggc   480
   Pro Val Glu Gln Ser Pro Gln Glu Pro Asp Ser Ser Ser Gly Ile Gly
   145                 150                 155                 160
   aag aca ggc cag cag ccc gct aaa aag aga ctc aat ttt ggt cag act    528
   Lys Thr Gly Gln Gln Pro Ala Lys Lys Arg Leu Asn Phe Gly Gln Thr
                   165                 170                 175
   ggc gac tca gag tca gtc ccc gat cca caa cct ctc gga gaa cct cca    576
   Gly Asp Ser Glu Ser Val Pro Asp Pro Gln Pro Leu Gly Glu Pro Pro
               180                 185                 190
   gca acc ccc gct gct gtg gga cct act aca atg gct tca ggc ggt ggc    624
   Ala Thr Pro Ala Ala Val Gly Pro Thr Thr Met Ala Ser Gly Gly Gly
           195                 200                 205
   gca cca atg gca gac aat aac gaa ggc gcc gac gga gtg ggt aat gcc    672
   Ala Pro Met Ala Asp Asn Asn Glu Gly Ala Asp Gly Val Gly Asn Ala
       210                 215                 220
   tca gga aat tgg cat tgc gat tcc aca tgg ctg ggc gac aga gtc atc    720
   Ser Gly Asn Trp His Cys Asp Ser Thr Trp Leu Gly Asp Arg Val Ile
   225                 230                 235                 240
   acc acc agc acc cgc acc tgg gcc ttg ccc acc tac aat aac cac ctc    768
   Thr Thr Ser Thr Arg Thr Trp Ala Leu Pro Thr Tyr Asn Asn His Leu
                   245                 250                 255
   tac aag caa atc tcc agt gct tca acg ggg gcc agc aac gac aac cac    816
   Tyr Lys Gln Ile Ser Ser Ala Ser Thr Gly Ala Ser Asn Asp Asn His
               260                 265                 270
   tac ttc ggc tac agc acc ccc tgg ggg tat ttt gat ttc aac aga ttc    864
   Tyr Phe Gly Tyr Ser Thr Pro Trp Gly Tyr Phe Asp Phe Asn Arg Phe
           275                 280                 285
   cac tgc cac ttt tca cca cgt gac tgg cag cga ctc atc aac aac aat    912
   His Cys His Phe Ser Pro Arg Asp Trp Gln Arg Leu Ile Asn Asn Asn
       290                 295                 300
   tgg gga ttc cgg ccc aag aga ctc aac ttc aaa ctc ttc aac atc caa    960
   Trp Gly Phe Arg Pro Lys Arg Leu Asn Phe Lys Leu Phe Asn Ile Gln
   305                 310                 315                 320
   gtc aag gag gtc acg acg aat gat ggc gtc aca acc atc gct aat aac   1008
   Val Lys Glu Val Thr Thr Asn Asp Gly Val Thr Thr Ile Ala Asn Asn
                   325                 330                 335
   ctt acc agc acg gtt caa gtc ttc tcg gac tcg gag tac cag ctt ccg   1056
   Leu Thr Ser Thr Val Gln Val Phe Ser Asp Ser Glu Tyr Gln Leu Pro
               340                 345                 350
   tac gtc ctc ggc tct gcg cac cag ggc tgc ctc cct ccg ttc ccg gcg    1104
   Tyr Val Leu Gly Ser Ala His Gln Gly Cys Leu Pro Pro Phe Pro Ala
           355                 360                 365
   gac gtg ttc atg att ccg caa tac ggc tac ctg acg ctc aac aat ggc    1152
   Asp Val Phe Met Ile Pro Gln Tyr Gly Tyr Leu Thr Leu Asn Asn Gly
       370                 375                 380
   agc caa gcc gtg gga cgt tca tcc ttt tac tgc ctg gaa tat ttc cct    1200
   Ser Gln Ala Val Gly Arg Ser Ser Phe Tyr Cys Leu Glu Tyr Phe Pro
   385                 390                 395                 400
   tct cag atg ctg aga acg ggc aac aac ttt acc ttc agc tac acc ttt    1248
   Ser Gln Met Leu Arg Thr Gly Asn Asn Phe Thr Phe Ser Tyr Thr Phe
                   405                 410                 415
   gag gaa gtg cct ttc cac agc agc tac gcg cac agc cag agc ctg gac    1296
   Glu Glu Val Pro Phe His Ser Ser Tyr Ala His Ser Gln Ser Leu Asp
               420                 425                 430
   cgg ctg atg aat cct ctc atc gac caa tac ctg tat tac ctg aac aga    1344
   Arg Leu Met Asn Pro Leu Ile Asp Gln Tyr Leu Tyr Tyr Leu Asn Arg
           435                 440                 445
   act caa aat cag tcc gga agt gcc caa aac aag gac ttg ctg ttt agc    1392
   Thr Gln Asn Gln Ser Gly Ser Ala Gln Asn Lys Asp Leu Leu Phe Ser
       450                 455                 460
   cgt ggg tct cca gct ggc atg tct gtt cag ccc aaa aac tgg cta cct    1440
   Arg Gly Ser Pro Ala Gly Met Ser Val Gln Pro Lys Asn Trp Leu Pro
   465                 470                 475                 480
   gga ccc tgt tat cgg cag cag cgc gtt tct aaa aca aaa aca gac aac    1488
   Gly Pro Cys Tyr Arg Gln Gln Arg Val Ser Lys Thr Lys Thr Asp Asn
                   485                 490                 495
   aac aac agc aat ttt acc tgg act ggt gct tca aaa tat aac ctc aat    1536
   Asn Asn Ser Asn Phe Thr Trp Thr Gly Ala Ser Lys Tyr Asn Leu Asn
               500                 505                 510
   ggg cgt gaa tcc atc atc aac cct ggc act gct atg gcc tca cac aaa    1584
   Gly Arg Glu Ser Ile Ile Asn Pro Gly Thr Ala Met Ala Ser His Lys
           515                 520                 525
   gac gac gaa gac aag ttc ttt ccc atg agc ggt gtc atg att ttt gga    1632
   Asp Asp Glu Asp Lys Phe Phe Pro Met Ser Gly Val Met Ile Phe Gly
       530                 535                 540
   aaa gag agc gcc gga gct tca aac act gca ttg gac aat gtc atg att     1680
   Lys Glu Ser Ala Gly Ala Ser Asn Thr Ala Leu Asp Asn Val Met Ile
   545                 550                 555                 560
   aca gac gaa gag gaa att aaa gcc act aac cct gtg gcc acc gaa aga     1728
   Thr Asp Glu Glu Glu Ile Lys Ala Thr Asn Pro Val Ala Thr Glu Arg
                   565                 570                 575
   ttt ggg acc gtg gca gtc aat ttc cag agc agc agc aca gac cct gcg     1776
   Phe Gly Thr Val Ala Val Asn Phe Gln Ser Ser Ser Thr Asp Pro Ala
               580                 585                 590
   acc gga gat gtg cat gct atg gga gca tta cct ggc atg gtg tgg caa     1824
   Thr Gly Asp Val His Ala Met Gly Ala Leu Pro Gly Met Val Trp Gln
           595                 600                 605
   gat aga gac gtg tac ctg cag ggt ccc att tgg gcc aaa att cct cac     1872
   Asp Arg Asp Val Tyr Leu Gln Gly Pro Ile Trp Ala Lys Ile Pro His
       610                 615                 620
   aca gat gga cac ttt cac ccg tct cct ctt atg ggc ggc ttt gga ctc     1920
   Thr Asp Gly His Phe His Pro Ser Pro Leu Met Gly Gly Phe Gly Leu
   625                 630                 635                 640
   aag aac ccg cct cct cag atc ctc atc aaa aac acg cct gtt cct gcg     1968
   Lys Asn Pro Pro Pro Gln Ile Leu Ile Lys Asn Thr Pro Val Pro Ala
                   645                 650                 655
   aat cct ccg gcg gag ttt tca gct aca aag ttt gct tca ttc atc acc     2016
   Asn Pro Pro Ala Glu Phe Ser Ala Thr Lys Phe Ala Ser Phe Ile Thr
               660                 665                 670
   caa tac tcc aca gga caa gtg agt gtg gaa att gaa tgg gag ctg cag     2064
   Gln Tyr Ser Thr Gly Gln Val Ser Val Glu Ile Glu Trp Glu Leu Gln
           675                 680                 685
   aaa gaa aac agc aag cgc tgg aat ccc gaa gtg cag tac aca tcc aat     2112
   Lys Glu Asn Ser Lys Arg Trp Asn Pro Glu Val Gln Tyr Thr Ser Asn
       690                 695                 700
   tat gca aaa tct gcc aac gtt gat ttt act gtg gac aac aat gga ctt     2160
   Tyr Ala Lys Ser Ala Asn Val Asp Phe Thr Val Asp Asn Asn Gly Leu
   705                 710                 715                 720
   tat act gag cct cgc ccc att ggc acc cgt tac ctt acc cgt ccc ctg     2208
   Tyr Thr Glu Pro Arg Pro Ile Gly Thr Arg Tyr Leu Thr Arg Pro Leu
   725 730 735
   taa 2211
<210> 13
   <211> 736
   <212> PRT
   <213> AAV-1
<400> 13
   Met Ala Ala Asp Gly Tyr Leu Pro Asp Trp Leu Glu Asp Asn Leu Ser
   1 5 10 15
   Glu Gly Ile Arg Glu Trp Trp Asp Leu Lys Pro Gly Ala Pro Lys Pro
   20 25 30
   Lys Ala Asn Gln Gln Lys Gln Asp Asp Gly Arg Gly Leu Val Leu Pro
   35 40 45
   Gly Tyr Lys Tyr Leu Gly Pro Phe Asn Gly Leu Asp Lys Gly Glu Pro
   50 55 60
   Val Asn Ala Ala Asp Ala Ala Ala Leu Glu His Asp Lys Ala Tyr Asp
   65 70 75 80
   Gln Gln Leu Lys Ala Gly Asp Asn Pro Tyr Leu Arg Tyr Asn His Ala
   85 90 95
   Asp Ala Glu Phe Gln Glu Arg Leu Gln Glu Asp Thr Ser Phe Gly Gly
   100 105 110
   Asn Leu Gly Arg Ala Val Phe Gln Ala Lys Lys Arg Val Leu Glu Pro
   115 120 125
   Leu Gly Leu Val Glu Glu Gly Ala Lys Thr Ala Pro Gly Lys Lys Arg
   130 135 140
   Pro Val Glu Gln Ser Pro Gln Glu Pro Asp Ser Ser Ser Gly Ile Gly
   145 150 155 160
   Lys Thr Gly Gln Gln Pro Ala Lys Lys Arg Leu Asn Phe Gly Gln Thr
   165 170 175
   Gly Asp Ser Glu Ser Val Pro Asp Pro Gln Pro Leu Gly Glu Pro Pro
   180 185 190
   Ala Thr Pro Ala Ala Val Gly Pro Thr Thr Met Ala Ser Gly Gly Gly
   195 200 205
   Ala Pro Met Ala Asp Asn Asn Glu Gly Ala Asp Gly Val Gly Asn Ala
   210 215 220
   Ser Gly Asn Trp His Cys Asp Ser Thr Trp Leu Gly Asp Arg Val Ile
   225 230 235 240
   Thr Thr Ser Thr Arg Thr Trp Ala Leu Pro Thr Tyr Asn Asn His Leu
   245 250 255
   Tyr Lys Gln Ile Ser Ser Ala Ser Thr Gly Ala Ser Asn Asp Asn His
   260 265 270
   Tyr Phe Gly Tyr Ser Thr Pro Trp Gly Tyr Phe Asp Phe Asn Arg Phe
   275 280 285
   His Cys His Phe Ser Pro Arg Asp Trp Gln Arg Leu Ile Asn Asn Asn
   290 295 300
   Trp Gly Phe Arg Pro Lys Arg Leu Asn Phe Lys Leu Phe Asn Ile Gln
   305 310 315 320
   Val Lys Glu Val Thr Thr Asn Asp Gly Val Thr Thr Ile Ala Asn Asn
   325 330 335
   Leu Thr Ser Thr Val Gln Val Phe Ser Asp Ser Glu Tyr Gln Leu Pro
   340 345 350
   Tyr Val Leu Gly Ser Ala His Gln Gly Cys Leu Pro Pro Phe Pro Ala
   355 360 365
   Asp Val Phe Met Ile Pro Gln Tyr Gly Tyr Leu Thr Leu Asn Asn Gly
   370 375 380
   Ser Gln Ala Val Gly Arg Ser Ser Phe Tyr Cys Leu Glu Tyr Phe Pro
   385 390 395 400
   Ser Gln Met Leu Arg Thr Gly Asn Asn Phe Thr Phe Ser Tyr Thr Phe
   405 410 415
   Glu Glu Val Pro Phe His Ser Ser Tyr Ala His Ser Gln Ser Leu Asp
   420 425 430
   Arg Leu Met Asn Pro Leu Ile Asp Gln Tyr Leu Tyr Tyr Leu Asn Arg
   435 440 445
   Thr Gln Asn Gln Ser Gly Ser Ala Gln Asn Lys Asp Leu Leu Phe Ser
   450 455 460
   Arg Gly Ser Pro Ala Gly Met Ser Val Gln Pro Lys Asn Trp Leu Pro
   465 470 475 480
   Gly Pro Cys Tyr Arg Gln Gln Arg Val Ser Lys Thr Lys Thr Asp Asn
   485 490 495
   Asn Asn Ser Asn Phe Thr Trp Thr Gly Ala Ser Lys Tyr Asn Leu Asn
   500 505 510
   Gly Arg Glu Ser Ile Ile Asn Pro Gly Thr Ala Met Ala Ser His Lys
   515 520 525
   Asp Asp Glu Asp Lys Phe Phe Pro Met Ser Gly Val Met Ile Phe Gly
   530 535 540
   Lys Glu Ser Ala Gly Ala Ser Asn Thr Ala Leu Asp Asn Val Met Ile
   545 550 555 560
   Thr Asp Glu Glu Glu Ile Lys Ala Thr Asn Pro Val Ala Thr Glu Arg
   565 570 575
   Phe Gly Thr Val Ala Val Asn Phe Gln Ser Ser Ser Thr Asp Pro Ala
   580 585 590
   Thr Gly Asp Val His Ala Met Gly Ala Leu Pro Gly Met Val Trp Gln
   595 600 605
   Asp Arg Asp Val Tyr Leu Gln Gly Pro Ile Trp Ala Lys Ile Pro His
   610 615 620
   Thr Asp Gly His Phe His Pro Ser Pro Leu Met Gly Gly Phe Gly Leu
   625 630 635 640
   Lys Asn Pro Pro Pro Gln Ile Leu Ile Lys Asn Thr Pro Val Pro Ala
   645 650 655
   Asn Pro Pro Ala Glu Phe Ser Ala Thr Lys Phe Ala Ser Phe Ile Thr
   660 665 670
   Gln Tyr Ser Thr Gly Gln Val Ser Val Glu Ile Glu Trp Glu Leu Gln
   675 680 685
   Lys Glu Asn Ser Lys Arg Trp Asn Pro Glu Val Gln Tyr Thr Ser Asn
   690 695 700
   Tyr Ala Lys Ser Ala Asn Val Asp Phe Thr Val Asp Asn Asn Gly Leu
   705                 710                 715                 720
   Tyr Thr Glu Pro Arg Pro Ile Gly Thr Arg Tyr Leu Thr Arg Pro Leu
                   725                 730                 735
<210> 14
   <211> 1800
   <212> DNA
   <213> AAV-1
<220>
   <221> CDS
   <222> (1)..(1797)
<400> 14
   acg gct cct gga aag aaa cgt ccg gta gag cag tcg cca caa gag cca      48
   Thr Ala Pro Gly Lys Lys Arg Pro Val Glu Gln Ser Pro Gln Glu Pro
     1               5                  10                  15
   gac tcc tcc tcg ggc atc ggc aag aca ggc cag cag ccc gct aaa aag      96
   Asp Ser Ser Ser Gly Ile Gly Lys Thr Gly Gln Gln Pro Ala Lys Lys
                20                  25                  30
   aga ctc aat ttt ggt cag act ggc gac tca gag tca gtc ccc gat cca     144
   Arg Leu Asn Phe Gly Gln Thr Gly Asp Ser Glu Ser Val Pro Asp Pro
            35                  40                  45
   caa cct ctc gga gaa cct cca gca acc ccc gct gct gtg gga cct act     192
   Gln Pro Leu Gly Glu Pro Pro Ala Thr Pro Ala Ala Val Gly Pro Thr
        50                  55                  60
   aca atg gct tca ggc ggt ggc gca cca atg gca gac aat aac gaa ggc     240
   Thr Met Ala Ser Gly Gly Gly Ala Pro Met Ala Asp Asn Asn Glu Gly
    65                  70                  75                  80
   gcc gac gga gtg ggt aat gcc tca gga aat tgg cat tgc gat tcc aca     288
   Ala Asp Gly Val Gly Asn Ala Ser Gly Asn Trp His Cys Asp Ser Thr
                    85                  90                  95
   tgg ctg ggc gac aga gtc atc acc acc agc acc cgc acc tgg gcc ttg     336
   Trp Leu Gly Asp Arg Val Ile Thr Thr Ser Thr Arg Thr Trp Ala Leu
               100                 105                 110
   ccc acc tac aat aac cac ctc tac aag caa atc tcc agt gct tca acg     384
   Pro Thr Tyr Asn Asn His Leu Tyr Lys Gln Ile Ser Ser Ala Ser Thr
           115                 120                 125
   ggg gcc agc aac gac aac cac tac ttc ggc tac agc acc ccc tgg ggg    432
   Gly Ala Ser Asn Asp Asn His Tyr Phe Gly Tyr Ser Thr Pro Trp Gly
       130                 135                 140
   tat ttt gat ttc aac aga ttc cac tgc cac ttt tca cca cgt gac tgg    480
   Tyr Phe Asp Phe Asn Arg Phe His Cys His Phe Ser Pro Arg Asp Trp
   145                 150                 155                 160
   cag cga ctc atc aac aac aat tgg gga ttc cgg ccc aag aga ctc aac    528
   Gln Arg Leu Ile Asn Asn Asn Trp Gly Phe Arg Pro Lys Arg Leu Asn
                   165                 170                 175
   ttc aaa ctc ttc aac atc caa gtc aag gag gtc acg acg aat gat ggc    576
   Phe Lys Leu Phe Asn Ile Gln Val Lys Glu Val Thr Thr Asn Asp Gly
               180                 185                 190
   gtc aca acc atc gct aat aac ctt acc agc acg gtt caa gtc ttc tcg    624
   Val Thr Thr Ile Ala Asn Asn Leu Thr Ser Thr Val Gln Val Phe Ser
           195                 200                 205
   gac tcg gag tac cag ctt ccg tac gtc ctc ggc tct gcg cac cag ggc    672
   Asp Ser Glu Tyr Gln Leu Pro Tyr Val Leu Gly Ser Ala His Gln Gly
       210                 215                 220
   tgc ctc cct ccg ttc ccg gcg gac gtg ttc atg att ccg caa tac ggc    720
   Cys Leu Pro Pro Phe Pro Ala Asp Val Phe Met Ile Pro Gln Tyr Gly
   225                 230                 235                 240
   tac ctg acg ctc aac aat ggc agc caa gcc gtg gga cgt tca tcc ttt    768
   Tyr Leu Thr Leu Asn Asn Gly Ser Gln Ala Val Gly Arg Ser Ser Phe
                   245                 250                 255
   tac tgc ctg gaa tat ttc cct tct cag atg ctg aga acg ggc aac aac    816
   Tyr Cys Leu Glu Tyr Phe Pro Ser Gln Met Leu Arg Thr Gly Asn Asn
               260                 265                 270
   ttt acc ttc agc tac acc ttt gag gaa gtg cct ttc cac agc agc tac    864
   Phe Thr Phe Ser Tyr Thr Phe Glu Glu Val Pro Phe His Ser Ser Tyr
           275                 280                 285
   gcg cac agc cag agc ctg gac cgg ctg atg aat cct ctc atc gac caa    912
   Ala His Ser Gln Ser Leu Asp Arg Leu Met Asn Pro Leu Ile Asp Gln
       290                 295                 300
   tac ctg tat tac ctg aac aga act caa aat cag tcc gga agt gcc caa    960
   Tyr Leu Tyr Tyr Leu Asn Arg Thr Gln Asn Gln Ser Gly Ser Ala Gln
   305                 310                 315                 320
   aac aag gac ttg ctg ttt agc cgt ggg tct cca gct ggc atg tct gtt    1008
   Asn Lys Asp Leu Leu Phe Ser Arg Gly Ser Pro Ala Gly Met Ser Val
                   325                 330                 335
   cag ccc aaa aac tgg cta cct gga ccc tgt tat cgg cag cag cgc gtt    1056
   Gln Pro Lys Asn Trp Leu Pro Gly Pro Cys Tyr Arg Gln Gln Arg Val
               340                 345                 350
   tct aaa aca aaa aca gac aac aac aac agc aat ttt acc tgg act ggt    1104
   Ser Lys Thr Lys Thr Asp Asn Asn Asn Ser Asn Phe Thr Trp Thr Gly
           355                 360                 365
   gct tca aaa tat aac ctc aat ggg cgt gaa tcc atc atc aac cct ggc    1152
   Ala Ser Lys Tyr Asn Leu Asn Gly Arg Glu Ser Ile Ile Asn Pro Gly
       370                 375                 380
   act gct atg gcc tca cac aaa gac gac gaa gac aag ttc ttt ccc atg    1200
   Thr Ala Met Ala Ser His Lys Asp Asp Glu Asp Lys Phe Phe Pro Met
   385                 390                 395                 400
   agc ggt gtc atg att ttt gga aaa gag agc gcc gga gct tca aac act    1248
   Ser Gly Val Met Ile Phe Gly Lys Glu Ser Ala Gly Ala Ser Asn Thr
                   405                 410                 415
   gca ttg gac aat gtc atg att aca gac gaa gag gaa att aaa gcc act    1296
   Ala Leu Asp Asn Val Met Ile Thr Asp Glu Glu Glu Ile Lys Ala Thr
               420                 425                 430
   aac cct gtg gcc acc gaa aga ttt ggg acc gtg gca gtc aat ttc cag    1344
   Asn Pro Val Ala Thr Glu Arg Phe Gly Thr Val Ala Val Asn Phe Gln
           435                 440                 445
   agc agc agc aca gac cct gcg acc gga gat gtg cat gct atg gga gca    1392
   Ser Ser Ser Thr Asp Pro Ala Thr Gly Asp Val His Ala Met Gly Ala
       450                 455                 460
   tta cct ggc atg gtg tgg caa gat aga gac gtg tac ctg cag ggt ccc    1440
   Leu Pro Gly Met Val Trp Gln Asp Arg Asp Val Tyr Leu Gln Gly Pro
   465                 470                 475                 480
   att tgg gcc aaa att cct cac aca gat gga cac ttt cac ccg tct cct    1488
   Ile Trp Ala Lys Ile Pro His Thr Asp Gly His Phe His Pro Ser Pro
                   485                 490                 495
   ctt atg ggc ggc ttt gga ctc aag aac ccg cct cct cag atc ctc atc    1536
   Leu Met Gly Gly Phe Gly Leu Lys Asn Pro Pro Pro Gln Ile Leu Ile
               500                 505                 510
   aaa aac acg cct gtt cct gcg aat cct ccg gcg gag ttt tca gct aca    1584
   Lys Asn Thr Pro Val Pro Ala Asn Pro Pro Ala Glu Phe Ser Ala Thr
           515                 520                 525
   aag ttt gct tca ttc atc acc caa tac tcc aca gga caa gtg agt gtg    1632
   Lys Phe Ala Ser Phe Ile Thr Gln Tyr Ser Thr Gly Gln Val Ser Val
       530                 535                 540
   gaa att gaa tgg gag ctg cag aaa gaa aac agc aag cgc tgg aat ccc    1680
   Glu Ile Glu Trp Glu Leu Gln Lys Glu Asn Ser Lys Arg Trp Asn Pro
   545                 550                 555                 560
   gaa gtg cag tac aca tcc aat tat gca aaa tct gcc aac gtt gat ttt    1728
   Glu Val Gln Tyr Thr Ser Asn Tyr Ala Lys Ser Ala Asn Val Asp Phe
                   565                 570                 575
   act gtg gac aac aat gga ctt tat act gag cct cgc ccc att ggc acc    1776
   Thr Val Asp Asn Asn Gly Leu Tyr Thr Glu Pro Arg Pro Ile Gly Thr
               580                 585                 590
   cgt tac ctt acc cgt ccc ctg taa                                    1800
   Arg Tyr Leu Thr Arg Pro Leu
           595
<210> 15
   <211> 599
   <212> PRT
   <213> AAV-1
<400> 15
   Thr Ala Pro Gly Lys Lys Arg Pro Val Glu Gln Ser Pro Gln Glu Pro
   1               5                   10                  15
   Asp Ser Ser Ser Gly Ile Gly Lys Thr Gly Gln Gln Pro Ala Lys Lys
               20                  25                  30
   Arg Leu Asn Phe Gly Gln Thr Gly Asp Ser Glu Ser Val Pro Asp Pro
           35                  40                  45
   Gln Pro Leu Gly Glu Pro Pro Ala Thr Pro Ala Ala Val Gly Pro Thr
       50                  55                  60
   Thr Met Ala Ser Gly Gly Gly Ala Pro Met Ala Asp Asn Asn Glu Gly
   65                  70                  75                  80
   Ala Asp Gly Val Gly Asn Ala Ser Gly Asn Trp His Cys Asp Ser Thr
   85 90 95
   Trp Leu Gly Asp Arg Val Ile Thr Thr Ser Thr Arg Thr Trp Ala Leu
   100 105 110
   Pro Thr Tyr Asn Asn His Leu Tyr Lys Gln Ile Ser Ser Ala Ser Thr
   115 120 125
   Gly Ala Ser Asn Asp Asn His Tyr Phe Gly Tyr Ser Thr Pro Trp Gly
   130 135 140
   Tyr Phe Asp Phe Asn Arg Phe His Cys His Phe Ser Pro Arg Asp Trp
   145 150 155 160
   Gln Arg Leu Ile Asn Asn Asn Trp Gly Phe Arg Pro Lys Arg Leu Asn
   165 170 175
   Phe Lys Leu Phe Asn Ile Gln Val Lys Glu Val Thr Thr Asn Asp Gly
   180 185 190
   Val Thr Thr Ile Ala Asn Asn Leu Thr Ser Thr Val Gln Val Phe Ser
   195 200 205
   Asp Ser Glu Tyr Gln Leu Pro Tyr Val Leu Gly Ser Ala His Gln Gly
   210 215 220
   Cys Leu Pro Pro Phe Pro Ala Asp Val Phe Met Ile Pro Gln Tyr Gly
   225 230 235 240
   Tyr Leu Thr Leu Asn Asn Gly Ser Gln Ala Val Gly Arg Ser Ser Phe
   245 250 255
   Tyr Cys Leu Glu Tyr Phe Pro Ser Gln Met Leu Arg Thr Gly Asn Asn
   260 265 270
   Phe Thr Phe Ser Tyr Thr Phe Glu Glu Val Pro Phe His Ser Ser Tyr
   275 280 285
   Ala His Ser Gln Ser Leu Asp Arg Leu Met Asn Pro Leu Ile Asp Gln
   290 295 300
   Tyr Leu Tyr Tyr Leu Asn Arg Thr Gln Asn Gln Ser Gly Ser Ala Gln
   305 310 315 320
   Asn Lys Asp Leu Leu Phe Ser Arg Gly Ser Pro Ala Gly Met Ser Val
   325 330 335
   Gln Pro Lys Asn Trp Leu Pro Gly Pro Cys Tyr Arg Gln Gln Arg Val
   340 345 350
   Ser Lys Thr Lys Thr Asp Asn Asn Asn Ser Asn Phe Thr Trp Thr Gly
   355 360 365
   Ala Ser Lys Tyr Asn Leu Asn Gly Arg Glu Ser Ile Ile Asn Pro Gly
   370 375 380
   Thr Ala Met Ala Ser His Lys Asp Asp Glu Asp Lys Phe Phe Pro Met
   385 390 395 400
   Ser Gly Val Met Ile Phe Gly Lys Glu Ser Ala Gly Ala Ser Asn Thr
   405 410 415
   Ala Leu Asp Asn Val Met Ile Thr Asp Glu Glu Glu Ile Lys Ala Thr
   420 425 430
   Asn Pro Val Ala Thr Glu Arg Phe Gly Thr Val Ala Val Asn Phe Gln
   435 440 445
   Ser Ser Ser Thr Asp Pro Ala Thr Gly Asp Val His Ala Met Gly Ala
   450 455 460
   Leu Pro Gly Met Val Trp Gln Asp Arg Asp Val Tyr Leu Gln Gly Pro
   465 470 475 480
   Ile Trp Ala Lys Ile Pro His Thr Asp Gly His Phe His Pro Ser Pro
   485 490 495
   Leu Met Gly Gly Phe Gly Leu Lys Asn Pro Pro Pro Gln Ile Leu Ile
   500 505 510
   Lys Asn Thr Pro Val Pro Ala Asn Pro Pro Ala Glu Phe Ser Ala Thr
   515 520 525
   Lys Phe Ala Ser Phe Ile Thr Gln Tyr Ser Thr Gly Gln Val Ser Val
   530 535 540
   Glu Ile Glu Trp Glu Leu Gln Lys Glu Asn Ser Lys Arg Trp Asn Pro
   545 550 555 560
   Glu Val Gln Tyr Thr Ser Asn Tyr Ala Lys Ser Ala Asn Val Asp Phe
   565 570 575
   Thr Val Asp Asn Asn Gly Leu Tyr Thr Glu Pro Arg Pro Ile Gly Thr
   580 585 590
   Arg Tyr Leu Thr Arg Pro Leu
   595
<210> 16
   <211> 1605
   <212> DNA
   <213> AAV-1
<220>
   <221> CDS
   <222> (1)..(1602)
<400> 16
   atg gct tca ggc ggt ggc gca cca atg gca gac aat aac gaa ggc gcc     48
   Met Ala Ser Gly Gly Gly Ala Pro Met Ala Asp Asn Asn Glu Gly Ala
   1               5                   10                  15
   gac gga gtg ggt aat gcc tca gga aat tgg cat tgc gat tcc aca tgg     96
   Asp Gly Val Gly Asn Ala Ser Gly Asn Trp His Cys Asp Ser Thr Trp
               20                  25                  30
   ctg ggc gac aga gtc atc acc acc agc acc cgc acc tgg gcc ttg ccc     144
   Leu Gly Asp Arg Val Ile Thr Thr Ser Thr Arg Thr Trp Ala Leu Pro
           35                  40                  45
   acc tac aat aac cac ctc tac aag caa atc tcc agt gct tca acg ggg     192
   Thr Tyr Asn Asn His Leu Tyr Lys Gln Ile Ser Ser Ala Ser Thr Gly
       50                  55                  60
   gcc agc aac gac aac cac tac ttc ggc tac agc acc ccc tgg ggg tat     240
   Ala Ser Asn Asp Asn His Tyr Phe Gly Tyr Ser Thr Pro Trp Gly Tyr
   65                  70                  75                  80
   ttt gat ttc aac aga ttc cac tgc cac ttt tca cca cgt gac tgg cag     288
   Phe Asp Phe Asn Arg Phe His Cys His Phe Ser Pro Arg Asp Trp Gln
                   85                  90                  95
   cga ctc atc aac aac aat tgg gga ttc cgg ccc aag aga ctc aac ttc     336
   Arg Leu Ile Asn Asn Asn Trp Gly Phe Arg Pro Lys Arg Leu Asn Phe
               100                 105                 110
   aaa ctc ttc aac atc caa gtc aag gag gtc acg acg aat gat ggc gtc     384
   Lys Leu Phe Asn Ile Gln Val Lys Glu Val Thr Thr Asn Asp Gly Val
           115                 120                 125
   aca acc atc gct aat aac ctt acc agc acg gtt caa gtc ttc tcg gac     432
   Thr Thr Ile Ala Asn Asn Leu Thr Ser Thr Val Gln Val Phe Ser Asp
       130                 135                 140
   tcg gag tac cag ctt ccg tac gtc ctc ggc tct gcg cac cag ggc tgc     480
   Ser Glu Tyr Gln Leu Pro Tyr Val Leu Gly Ser Ala His Gln Gly Cys
   145                 150                 155                 160
   ctc cct ccg ttc ccg gcg gac gtg ttc atg att ccg caa tac ggc tac     528
   Leu Pro Pro Phe Pro Ala Asp Val Phe Met Ile Pro Gln Tyr Gly Tyr
                   165                 170                 175
   ctg acg ctc aac aat ggc agc caa gcc gtg gga cgt tca tcc ttt tac     576
   Leu Thr Leu Asn Asn Gly Ser Gln Ala Val Gly Arg Ser Ser Phe Tyr
               180                 185                 190
   tgc ctg gaa tat ttc cct tct cag atg ctg aga acg ggc aac aac ttt     624
   Cys Leu Glu Tyr Phe Pro Ser Gln Met Leu Arg Thr Gly Asn Asn Phe
           195                 200                 205
   acc ttc agc tac acc ttt gag gaa gtg cct ttc cac agc agc tac gcg     672
   Thr Phe Ser Tyr Thr Phe Glu Glu Val Pro Phe His Ser Ser Tyr Ala
       210                 215                 220
   cac agc cag agc ctg gac cgg ctg atg aat cct ctc atc gac caa tac     720
   His Ser Gln Ser Leu Asp Arg Leu Met Asn Pro Leu Ile Asp Gln Tyr
   225                 230                 235                 240
   ctg tat tac ctg aac aga act caa aat cag tcc gga agt gcc caa aac     768
   Leu Tyr Tyr Leu Asn Arg Thr Gln Asn Gln Ser Gly Ser Ala Gln Asn
                   245                 250                 255
   aag gac ttg ctg ttt agc cgt ggg tct cca gct ggc atg tct gtt cag     816
   Lys Asp Leu Leu Phe Ser Arg Gly Ser Pro Ala Gly Met Ser Val Gln
               260                 265                 270
   ccc aaa aac tgg cta cct gga ccc tgt tat cgg cag cag cgc gtt tct     864
   Pro Lys Asn Trp Leu Pro Gly Pro Cys Tyr Arg Gln Gln Arg Val Ser
           275                 280                 285
   aaa aca aaa aca gac aac aac aac agc aat ttt acc tgg act ggt gct     912
   Lys Thr Lys Thr Asp Asn Asn Asn Ser Asn Phe Thr Trp Thr Gly Ala
       290                 295                 300
   tca aaa tat aac ctc aat ggg cgt gaa tcc atc atc aac cct ggc act     960
   Ser Lys Tyr Asn Leu Asn Gly Arg Glu Ser Ile Ile Asn Pro Gly Thr
   305                 310                 315                 320
   gct atg gcc tca cac aaa gac gac gaa gac aag ttc ttt ccc atg agc    1008
   Ala Met Ala Ser His Lys Asp Asp Glu Asp Lys Phe Phe Pro Met Ser
                   325                 330                 335
   ggt gtc atg att ttt gga aaa gag agc gcc gga gct tca aac act gca    1056
   Gly Val Met Ile Phe Gly Lys Glu Ser Ala Gly Ala Ser Asn Thr Ala
               340                 345                 350
   ttg gac aat gtc atg att aca gac gaa gag gaa att aaa gcc act aac    1104
   Leu Asp Asn Val Met Ile Thr Asp Glu Glu Glu Ile Lys Ala Thr Asn
           355                 360                 365
   cct gtg gcc acc gaa aga ttt ggg acc gtg gca gtc aat ttc cag agc    1152
   Pro Val Ala Thr Glu Arg Phe Gly Thr Val Ala Val Asn Phe Gln Ser
       370                 375                 380
   agc agc aca gac cct gcg acc gga gat gtg cat gct atg gga gca tta    1200
   Ser Ser Thr Asp Pro Ala Thr Gly Asp Val His Ala Met Gly Ala Leu
   385                 390                 395                 400
   cct ggc atg gtg tgg caa gat aga gac gtg tac ctg cag ggt ccc att    1248
   Pro Gly Met Val Trp Gln Asp Arg Asp Val Tyr Leu Gln Gly Pro Ile
                   405                 410                 415
   tgg gcc aaa att cct cac aca gat gga cac ttt cac ccg tct cct ctt    1296
   Trp Ala Lys Ile Pro His Thr Asp Gly His Phe His Pro Ser Pro Leu
               420                 425                 430
   atg ggc ggc ttt gga ctc aag aac ccg cct cct cag atc ctc atc aaa    1344
   Met Gly Gly Phe Gly Leu Lys Asn Pro Pro Pro Gln Ile Leu Ile Lys
           435                 440                 445
   aac acg cct gtt cct gcg aat cct ccg gcg gag ttt tca gct aca aag    1392
   Asn Thr Pro Val Pro Ala Asn Pro Pro Ala Glu Phe Ser Ala Thr Lys
       450                 455                 460
   ttt gct tca ttc atc acc caa tac tcc aca gga caa gtg agt gtg gaa    1440
   Phe Ala Ser Phe Ile Thr Gln Tyr Ser Thr Gly Gln Val Ser Val Glu
   465                 470                 475                 480
   att gaa tgg gag ctg cag aaa gaa aac agc aag cgc tgg aat ccc gaa    1488
   Ile Glu Trp Glu Leu Gln Lys Glu Asn Ser Lys Arg Trp Asn Pro Glu
                   485                 490                 495
   gtg cag tac aca tcc aat tat gca aaa tct gcc aac gtt gat ttt act    1536
   Val Gln Tyr Thr Ser Asn Tyr Ala Lys Ser Ala Asn Val Asp Phe Thr
               500                 505                 510
   gtg gac aac aat gga ctt tat act gag cct cgc ccc att ggc acc cgt    1584
   Val Asp Asn Asn Gly Leu Tyr Thr Glu Pro Arg Pro Ile Gly Thr Arg
           515                 520                 525
   tac ctt acc cgt ccc ctg taa 1605
   Tyr Leu Thr Arg Pro Leu
   530
<210> 17
   <211> 534
   <212> PRT
   <213> AAV-1
<400> 17
   Met Ala Ser Gly Gly Gly Ala Pro Met Ala Asp Asn Asn Glu Gly Ala
   1 5 10 15
   Asp Gly Val Gly Asn Ala Ser Gly Asn Trp His Cys Asp Ser Thr Trp
   20 25 30
   Leu Gly Asp Arg Val Ile Thr Thr Ser Thr Arg Thr Trp Ala Leu Pro
   35 40 45
   Thr Tyr Asn Asn His Leu Tyr Lys Gln Ile Ser Ser Ala Ser Thr Gly
   50 55 60
   Ala Ser Asn Asp Asn His Tyr Phe Gly Tyr Ser Thr Pro Trp Gly Tyr
   65 70 75 80
   Phe Asp Phe Asn Arg Phe His Cys His Phe Ser Pro Arg Asp Trp Gln
   85 90 95
   Arg Leu Ile Asn Asn Asn Trp Gly Phe Arg Pro Lys Arg Leu Asn Phe
   100 105 110
   Lys Leu Phe Asn Ile Gln Val Lys Glu Val Thr Thr Asn Asp Gly Val
   115 120 125
   Thr Thr Ile Ala Asn Asn Leu Thr Ser Thr Val Gln Val Phe Ser Asp
   130 135 140
   Ser Glu Tyr Gln Leu Pro Tyr Val Leu Gly Ser Ala His Gln Gly Cys
   145 150 155 160
   Leu Pro Pro Phe Pro Ala Asp Val Phe Met Ile Pro Gln Tyr Gly Tyr
   165 170 175
   Leu Thr Leu Asn Asn Gly Ser Gln Ala Val Gly Arg Ser Ser Phe Tyr
   180 185 190
   Cys Leu Glu Tyr Phe Pro Ser Gln Met Leu Arg Thr Gly Asn Asn Phe
   195 200 205
   Thr Phe Ser Tyr Thr Phe Glu Glu Val Pro Phe His Ser Ser Tyr Ala
   210 215 220
   His Ser Gln Ser Leu Asp Arg Leu Met Asn Pro Leu Ile Asp Gln Tyr
   225 230 235 240
   Leu Tyr Tyr Leu Asn Arg Thr Gln Asn Gln Ser Gly Ser Ala Gln Asn
   245 250 255
   Lys Asp Leu Leu Phe Ser Arg Gly Ser Pro Ala Gly Met Ser Val Gln
   260 265 270
   Pro Lys Asn Trp Leu Pro Gly Pro Cys Tyr Arg Gln Gln Arg Val Ser
   275 280 285
   Lys Thr Lys Thr Asp Asn Asn Asn Ser Asn Phe Thr Trp Thr Gly Ala
   290 295 300
   Ser Lys Tyr Asn Leu Asn Gly Arg Glu Ser Ile Ile Asn Pro Gly Thr
   305 310 315 320
   Ala Met Ala Ser His Lys Asp Asp Glu Asp Lys Phe Phe Pro Met Ser
   325 330 335
   Gly Val Met Ile Phe Gly Lys Glu Ser Ala Gly Ala Ser Asn Thr Ala
   340 345 350
   Leu Asp Asn Val Met Ile Thr Asp Glu Glu Glu Ile Lys Ala Thr Asn
   355 360 365
   Pro Val Ala Thr Glu Arg Phe Gly Thr Val Ala Val Asn Phe Gln Ser
   370 375 380
   Ser Ser Thr Asp Pro Ala Thr Gly Asp Val His Ala Met Gly Ala Leu
   385 390 395 400
   Pro Gly Met Val Trp Gln Asp Arg Asp Val Tyr Leu Gln Gly Pro Ile
   405 410 415
   Trp Ala Lys Ile Pro His Thr Asp Gly His Phe His Pro Ser Pro Leu
   420 425 430
   Met Gly Gly Phe Gly Leu Lys Asn Pro Pro Pro Gln Ile Leu Ile Lys
   435 440 445
   Asn Thr Pro Val Pro Ala Asn Pro Pro Ala Glu Phe Ser Ala Thr Lys
   450 455 460
   Phe Ala Ser Phe Ile Thr Gln Tyr Ser Thr Gly Gln Val Ser Val Glu
   465 470 475 480
   Ile Glu Trp Glu Leu Gln Lys Glu Asn Ser Lys Arg Trp Asn Pro Glu
   485 490 495
   Val Gln Tyr Thr Ser Asn Tyr Ala Lys Ser Ala Asn Val Asp Phe Thr
   500 505 510
   Val Asp Asn Asn Gly Leu Tyr Thr Glu Pro Arg Pro Ile Gly Thr Arg
   515 520 525
   Tyr Leu Thr Arg Pro Leu
   530
<210> 18
   <211> 4681
   <212> DNA
   <213> aav-2
<400> 18
   ttggccactc cctctctgcg cgctcgctcg ctcactgagg ccgggcgacc aaaggtcgcc 60
   cgacgcccgg gctttgcccg ggcggcctca gtgagcgagc gagcgcgcag agagggagtg 120
   gccaactcca tcactagggg ttcctggagg ggtggagtcg tgacgtgaat tacgtcatag 180
   ggttagggag gtcctgtatt agaggtcacg tgagtgtttt gcgacatttt gcgacaccat 240
   gtggtcacgc tgggtattta agcccgagtg agcacgcagg gtctccattt tgaagcggga 300
   ggtttgaacg cgcagccgcc atgccggggt tttacgagat tgtgattaag gtccccagcg 360
   accttgacgg gcatctgccc ggcatttctg acagctttgt gaactgggtg gccgagaagg 420
   aatgggagtt gccgccagat tctgacatgg atctgaatct gattgagcag gcacccctga 480
   ccgtggccga gaagctgcag cgcgactttc tgacggaatg gcgccgtgtg agtaaggccc 540
   cggaggccct tttctttgtg caatttgaga agggagagag ctacttccac atgcacgtgc 600
   tcgtggaaac caccggggtg aaatccatgg ttttgggacg tttcctgagt cagattcgcg 660
   aaaaactgat tcagagaatt taccgcggga tcgagccgac tttgccaaac tggttcgcgg 720
   tcacaaagac cagaaatggc gccggaggcg ggaacaaggt ggtggatgag tgctacatcc 780
   ccaattactt gctccccaaa acccagcctg agctccagtg ggcgtggact aatatggaac 840
   agtatttaag cgcctgtttg aatctcacgg agcgtaaacg gttggtggcg cagcatctga 900
   cgcacgtgtc gcagacgcag gagcagaaca aagagaatca gaatcccaat tctgatgcgc 960
   cggtgatcag atcaaaaact tcagccaggt acatggagct ggtcgggtgg ctcgtggaca 1020
   aggggattac ctcggagaag cagtggatcc aggaggacca ggcctcatac atctccttca 1080
   atgcggcctc caactcgcgg tcccaaatca aggctgcctt ggacaatgcg ggaaagatta 1140
   tgagcctgac taaaaccgcc cccgactacc tggtgggcca gcagcccgtg gaggacattt 1200
   ccagcaatcg gatttataaa attttggaac taaacgggta cgatccccaa tatgcggctt 1260
   ccgtctttct gggatgggcc acgaaaaagt tcggcaagag gaacaccatc tggctgtttg 1320
   ggcctgcaac taccgggaag accaacatcg cggaggccat agcccacact gtgcccttct 1380
   acgggtgcgt aaactggacc aatgagaact ttcccttcaa cgactgtgtc gacaagatgg 1440
   tgatctggtg ggaggagggg aagatgaccg ccaaggtcgt ggagtcggcc aaagccattc 1500
   tcggaggaag caaggtgcgc gtggaccaga aatgcaagtc ctcggcccag atagacccga 1560
   ctcccgtgat cgtcacctcc aacaccaaca tgtgcgccgt gattgacggg aactcaacga 1620
   ccttcgaaca ccagcagccg ttgcaagacc ggatgttcaa atttgaactc acccgccgtc 1680
   tggatcatga ctttgggaag gtcaccaagc aggaagtcaa agactttttc cggtgggcaa 1740
   aggatcacgt ggttgaggtg gagcatgaat tctacgtcaa aaagggtgga gccaagaaaa 1800
   gacccgcccc cagtgacgca gatataagtg agcccaaacg ggtgcgcgag tcagttgcgc 1860
   agccatcgac gtcagacgcg gaagcttcga tcaactacgc agacaggtac caaaacaaat 1920
   gttctcgtca cgtgggcatg aatctgatgc tgtttccctg cagacaatgc gagagaatga 1980
   atcagaattc aaatatctgc ttcactcacg gacagaaaga ctgtttagag tgctttcccg 2040
   tgtcagaatc tcaaccggtt tctgtcgtca aaaaggcgta tcagaaactg tgctacattc 2100
   atcatatcat gggaaaggtg ccagacgctt gcactgcctg cgatctggtc aatgtggatt 2160
   tggatgactg catctttgaa caataaatga tttaaatcag gtatggctgc cgatggttat 2220
   cttccagatt ggctcgagga cactctctct gaaggaataa gacagtggtg gaagctcaaa 2280
   cctggcccac caccaccaaa gcccgcagag cggcataagg acgacagcag gggtcttgtg 2340
   cttcctgggt acaagtacct cggacccttc aacggactcg acaagggaga gccggtcaac 2400
   gaggcagacg ccgcggccct cgagcacgac aaagcctacg accggcagct cgacagcgga 2460
   gacaacccgt acctcaagta caaccacgcc gacgcggagt ttcaggagcg ccttaaagaa 2520
   gatacgtctt ttgggggcaa cctcggacga gcagtcttcc aggcgaaaaa gagggttctt 2580
   gaacctctcg gcctggttga ggaacctgtt aagacggctc cgggaaaaaa gaggccggta 2640
   gagcactctc ctgtggagcc agactcctcc tcgggaaccg gaaagccggg ccagcagcct 2700
   gcaagaaaaa gattgaattt tggtcagact ggagacgcag actcagtacc tgacccccag 2760
   cctctcggac agccaccagc agccccctct ggtctgggaa ctaatacgat ggctacaggc 2820
   agtggcgcac caatggcaga caataacgag ggcgccgacg gagtgggtaa ttcctccgga 2880
   aattggcatt gcgattccac atggatgggc gacagagtca tcaccaccag cacccgaacc 2940
   tgggccctgc ccacctacaa caaccacctc tacaaacaaa tttccagcca atcaggagcc 3000
   tcgaacgaca atcactactt tggctacagc accccttggg ggtattttga cttcaacaga 3060
   ttccactgcc acttttcacc acgtgactgg caaagactca tcaacaacaa ctggggattc 3120
   cgacccaaga gactcaactt caacctcttt aacattcaag tcaaagaggt cacgcagaat 3180
   gacggtacga cgacgattgc caataacctt accagcacgg ttcaggtgtt tactgactcg 3240
   gagtaccagc tcccgtacgt cctcggctcg gcgcatcaag gatgcctccc gccgttccca 3300
   gcagacgtct tcatggtgcc acagtatgga tacctcaccc tgaacaacgg gagtcaggca 3360
   gtaggacgct cttcatttta ctgcctggag tactttcctt ctcagatgct gcgtaccgga 3420
   aacaacttta ccttcagcta cacttttgag gacgttcctt tccacagcag ctacgctcac 3480
   agccagagtc tggaccgtct catgaatcct ctcatcgacc agtacctgta ttacttgagc 3540
   agaacaaaca ctccaagtgg aaccaccacg cagtcaaggc ttcagttttc tcaggcccca 3600
   gccagtgaca ttcgggacca gtctaggaac tggcttcctg gaccctgtta ccgccagcag 3660
   cgagtatgaa agacatctgc ggataacaac aacagtgaat actcgtggac tggagctacc 3720
   aagtaccacc tcaatggcag agactctctg gtgaatccgg ggcccgccat ggcaagccac 3780
   aaggacgatg aagaaaagtt ttttcctcag agcggggttc tcatctttgg gaagcaaggc 3840
   tcagagaaaa caaatgtgaa cattgaaaag gtcatgatta cagacgaaga ggaaatccca 3900
   acaaccaatc ccgtggctac ggagcagtat ggttctgtat ctaccaacct ccagagaggc 3960
   aacagacaag cagctaccgc agatgtcaac acacaaggcg ttcttccagg catggtctgg 4020
   caggacagag atgtgtacct tcaggggccc atctgggcaa agattccaca cacggacgga 4080
   cattttcacc cctctcccct catgggtgga ttcggactta aacaccctcc tccacagatt 4140
   ctcatcaaga acaccccggt acctgcgaat ccttcgacca ccttcagtgc ggcaaagttt 4200
   gcttccttca tcacacagta ctccacggga cacggtcagc gtggagatcg agtgggagct 4260
   gcagaacgaa aacagcaaac gctggaatcc cgaaattcag tacacttcca actacaacaa 4320
   gtctgttaat cgtggacttt accgtggata ctaatggcgt gtattcagag cctcgcccca 4380
   ttggcaccag atacctgact cgtaatctgt aattgcttgt taatcaataa accgtttaat 4440
   tcgtttcagt tgaactttgg tctctgcgta tttctttctt atctagtttc catggctacg 4500
   tagataagta gcatggcggg ttaatcatta actacaagga acccctagtg atggagttgg 4560
   ccactccctc tctgcgcgct cgctcgctca ctgaggccgg gcgaccaaag gtcgcccgac 4620
   gcccgggctt tgcccgggcg gcctcagtga gcgagcgagc gcgcagagag ggagtgggca 4680
   a 4681
<210> 19
   <211> 4683
   <212> DNA
   <213> aav-6
<400> 19
   ttggccactc cctctctgcg cgctcgctcg ctcactgagg ccgggcgacc aaaggtcgcc 60
   cgacgcccgg gctttgcccg ggcggcctca gtgagcgagc gagcgcgcag agagggagtg 120
   gccaactcca tcactagggg ttcctggagg ggtggagtcg tgacgtgaat tacgtcatag 180
   ggttagggag gtcctgtatt agaggtcacg tgagtgtttt gcgacatttt gcgacaccat 240
   gtggtcacgc tgggtattta agcccgagtg agcacgcagg gtctccattt tgaagcggga 300
   ggtttgaacg cgcagcgcca tgccggggtt ttacgagatt gtgattaagg tccccagcga 360
   ccttgacgag catctgcccg gcatttctga cagctttgtg aactgggtgg ccgagaagga 420
   atgggagttg ccgccagatt ctgacatgga tctgaatctg attgagcagg cacccctgac 480
   cgtggccgag aagctgcagc gcgacttcct ggtccactgg cgccgcgtga gtaaggcccc 540
   ggaggccctc ttctttgttc agttcgagaa gggcgagtcc tacttccacc tccatattct 600
   ggtggagacc acgggggtca aatccatggt gctgggccgc ttcctgagtc agattagcga 660
   caagctggtg cagaccatct accgcgggat cgagccgacc ctgcccaact ggttcgcggt 720
   gaccaagacg cgtaatggcg ccggaggggg gaacaaggtg gtggacgagt gctacatccc 780
   caactacctc ctgcccaaga ctcagcccga gctgcagtgg gcgtggacta acatggagga 840
   gtatataagc gcgtgtttaa acctggccga gcgcaaacgg ctcgtggcgc acgacctgac 900
   ccacgtcagc cagacccagg agcagaacaa ggagaatctg aaccccaatt ctgacgcgcc 960
   tgtcatccgg tcaaaaacct ccgcacgcta catggagctg gtcgggtggc tggtggaccg 1020
   gggcatcacc tccgagaagc agtggatcca ggaggaccag gcctcgtaca tctccttcaa 1080
   cgccgcctcc aactcgcggt cccagatcaa ggccgctctg gacaatgccg gcaagatcat 1140
   ggcgctgacc aaatccgcgc ccgactacct ggtaggcccc gctccgcccg ccgacattaa 1200
   aaccaaccgc atttaccgca tcctggagct gaacggctac gaccctgcct acgccggctc 1260
   cgtctttctc ggctgggccc agaaaaggtt cggaaaacgc aacaccatct ggctgtttgg 1320
   gccggccacc acgggcaaga ccaacatcgc ggaagccatc gcccacgccg tgcccttcta 1380
   cggctgcgtc aactggacca atgagaactt tcccttcaac gattgcgtcg acaagatggt 1440
   gatctggtgg gaggagggca agatgacggc caaggtcgtg gagtccgcca aggccattct 1500
   cggcggcagc aaggtgcgcg tggaccaaaa gtgcaagtcg tccgcccaga tcgatcccac 1560
   ccccgtgatc gtcacctcca acaccaacat gtgcgccgtg attgacggga acagcaccac 1620
   cttcgagcac cagcagccgt tgcaggaccg gatgttcaaa tttgaactca cccgccgtct 1680
   ggagcatgac tttggcaagg tgacaaagca ggaagtcaaa gagttcttcc gctgggcgca 1740
   ggatcacgtg accgaggtgg cgcatgagtt ctacgtcaga aagggtggag ccaacaacag 1800
   acccgccccc gatgacgcgg ataaaagcga gcccaagcgg gcctgcccct cagtcgcgga 1860
   tccatcgacg tcagacgcgg aaggagctcc ggtggacttt gccgacaggt accaaaacaa 1920
   atgttctcgt cacgcgggca tgcttcagat gctgtttccc tgcaaaacat gcgagagaat 1980
   gaatcagaat ttcaacattt gcttcacgca cgggaccaga gactgttcag aatgtttccc 2040
   cggcgtgtca gaatctcaac cggtcgtcag aaagaggacg tatcggaaac tctgtgccat 2100
   tcatcatctg ctggggcggg ctcccgagat tgcttgctcg gcctgcgatc tggtcaacgt 2160
   ggatctggat gactgtgttt ctgagcaata aatgacttaa accaggtatg gctgccgatg 2220
   gttatcttcc agattggctc gaggacaacc tctctgaggg cattcggcag tggtgggact 2280
   tgaaacctgg agccccgaaa cccaaagcca accagcaaaa gcaggacgac ggccggggtc 2340
   tggtgcttcc tggctacaag tacctcggac ccttcaacgg actcgacaag ggggagcccg 2400
   tcaacgcggc ggatgcagcg gccctcgagc acgacaaggc ctacgaccag cagctcaaag 2460
   cgggtgacaa tccgtacctg cggtataacc acgccgacgc cgagtttcag gagcgtctgc 2520
   aagaagatac gtcttttggg ggcaacctcg ggcgagcagt cttccaggcc aagaagaggg 2580
   ttctcgaacc ttttggtctg gttgaggaag gtgctaagac ggctcctgga aagaaacgtc 2640
   cggtagagca gtcgccacaa gagccagact cctcctcggg cattggcaag acaggccagc 2700
   agcccgctaa aaagagactc aattttggtc agactggcga ctcagagtca gtccccgacc 2760
   cacaacctct cggagaacct ccagcaaccc ccgctgctgt gggacctact acaatggctt 2820
   caggcggtgg cgcaccaatg gcagacaata acgaaggcgc cgacggagtg ggtaatgcct 2880
   caggaaattg gcattgcgat tccacatggc tgggcgacag agtcatcacc accagcaccc 2940
   gaacatgggc cttgcccacc tataacaacc acctctacaa gcaaatctcc agtgcttcaa 3000
   cgggggccag caacgacaac cactacttcg gctacagcac cccctggggg tattttgatt 3060
   tcaacagatt ccactgccat ttctcaccac gtgactggca gcgactcatc aacaacaatt 3120
   ggggattccg gcccaagaga ctcaacttca agctcttcaa catccaagtc aaggaggtca 3180
   cgacgaatga tggcgtcacg accatcgcta ataaccttac cagcacggtt caagtcttgt 3240
   cggactcgga gtaccagttc ccgtacgtcc tcggctctgc gcaccagggc tgcctccctc 3300
   cgttcccggc ggacgtgttc atgattccgc agtacggcta cctaacgctc aacaatggca 3360
   gccaggcagt gggacgctca tccttttact gcctggaata tttcccatcg cagatgctga 3420
   gaacgggcaa taactttacc ttcagctaca ccttcgagga cgtgcctttc cacagcagct 3480
   acgcgcacag ccagagcctg gaccggctga tgaatcctct catcgaccag tacctgtatt 3540
   acctgaacag aactcacaat cagtccggaa gtgcccaaaa caaggacttg ctgtttagcc 3600
   gtgggtctcc agctggcatg tctgttcagc ccaaaaactg gctacctgga ccctgttacc 3660
   ggcagcagcg cgtttctaaa acaaaaacag acaacaacaa cagcaacttt acctggactg 3720
   gtgcttcaaa atataacctt aatgggcgtg aatctataat caaccctggc actgctatgg 3780
   cctcacacaa agacgacaaa gacaagttct ttcccatgag cggtgtcatg atttttggaa 3840
   aggagagcgc cggagcttca aacactgcat tggacaatgt catgatcaca gacgaagagg 3900
   aaatcaaagc cactaacccc gtggccaccg aaagatttgg gactgtggca gtcaatctcc 3960
   agagcagcag cacagaccct gcgaccggag atgtgcatgt tatgggagcc ttacctggaa 4020
   tggtgtggca agacagagac gtatacctgc agggtcctat ttgggccaaa attcctcaca 4080
   cggatggaca ctttcacccg tctcctctca tgggcggctt tggacttaag cacccgcctc 4140
   ctcagatcct catcaaaaac acgcctgttc ctgcgaatcc tccggcagag ttttcggcta 4200
   caaagtttgc ttcattcatc acccagtatt ccacaggaca agtgagcgtg gagattgaat 4260
   gggagctgca gaaagaaaac agcaaacgct ggaatcccga agtgcagtat acatctaact 4320
   atgcaaaatc tgccaacgtt gatttcactg tggacaacaa tggactttat actgagcctc 4380
   gccccattgg cacccgttac ctcacccgtc ccctgtaatt gtgtgttaat caataaaccg 4440
   gttaattcgt gtcagttgaa ctttggtctc atgtccttat tatcttatct ggtcaccata 4500
   gcaaccggtt acacattaac tgcttagttg cgcttcgcga atacccctag tgatggagtt 4560
   gcccactccc tctatgcgcg ctcgctcgct cggtggggcc ggcagagcag agctctgccg 4620
   tctgcggacc tttggtccgc aggccccacc gagcgagcga gcgcgcatag agggagtggc 4680
   caa 4683
<210> 20
   <211> 16
   <212> DNA
   <213> rep binding motif
<400> 20
   gctcgctcgc tcgctg 16